# FASCICULE DE BREVET EUROPEEN

(11) **EP 1 828 125 B1**
(45) Date de publication et mention de la délivrance du brevet: **09.04.2008**
(21) Numéro de dépôt: 05822892.5
(22) Date de dépôt: 29.11.2005
(51) Int. Cl.: C07D 209/44, C07D 405/12

(54) **DERIVES D'ARYLPIPERAZINE ET LEUR UTILISATION COMME LIGANDS SELECTIFS DU RECEPTEUR D3 DE LA DOPAMINE**
ARYLPIPERAZINDERIVATE UND DEREN VERWENDUNG ALS FÜR DEN DOPAMIN-D3-REZEPTOR SELEKTIVE LIGANDEN
ARYLPIPERAZINE DERIVATIVES AND THEIR USE AS LIGANGS SELECTIVE OF THE DOPAMINE D3 RECEPTOR

(30) Priorité: 01.12.2004 FR 0412763
(43) Date de publication de la demande: 05.09.2007
(73) Titulaire: BIOPROJET, 75003 Paris (FR)
(72) Inventeur: CAPET, Marc, F-35520 MELESSE (FR); DANVY, Denis, F-76190 YVETOT (FR); DARTOIS, Catherine, F-35760 SAINT GREGOIRE (FR); LEVOIN, Nicolas, F-35310 MORDELLES (FR); MORVAN, Marcel, F-35740 PACE (FR); BERREBI-BERTRAND, Isabelle, F-35740 PACE (FR); CALMELS, Thierry, F-35520 MELESSE (FR); ROBERT, Philippe, F-35740 PACE (FR); SCHWARTZ, Jean-Charles, F-75014 PARIS (FR); LECOMTE, Jeanne-Marie, F-75003 PARIS (FR)
(74) Mandataire: Colombet, Alain André
(86) Numéro de dépôt international: PCT/FR2005/002964
(87) Numéro de publication internationale: WO 2006/058993

(56) Documents cités:
- WO-A-01/49679
- WO-A-03/068752
- WO-A-20/04089905
- US-A- 5 523 299
- HACKLING A E ET AL: "DOPAMINE D3 RECEPTOR LIGANDS WITH ANTAGONIST PROPERTIES" CHEMBIOCHEM - A EUROPEAN JOURNAL OF CHEMICAL BIOLOGY, WILEY VCH, WEINHEIM, DE, vol. 3, no. 10, 4 octobre 2002 (2002-10-04), pages 946-961, XP001154341 ISSN: 1439-4227

## Description

La présente invention concerne de nouveaux dérivés d'arylpipérazine, leur procédé de préparation et leur utilisation en tant qu'agents thérapeutiques.

Plus précisément, les composés selon la présente invention présentent la propriété d'être des ligands sélectifs du récepteur D3 de la dopamine.

De nombreux dérivés d'arylpipérazine sont déjà connus comme antagonistes de la dopamine au niveau de ses récepteurs de type D2 ou D4, certains d'entre eux jouissant de propriétés neuroleptiques, ou encore comme antagonistes de la sérotonine ou de la noradrénaline.

Le brevet WO 01/49 679 décrit des dérivés d'arylpipérazine présentant des propriétés antagonistes de la dopamine sur ses récepteurs D3 et D4 ; néanmoins ces composés présentent d'une part un phényl sur la position 4 de la pipérazine, ce groupe phényl étant nécessairement substitué par un halogène, et d'autre part, sur la position 1 de la pipérazine, une chaîne alkylène portant éventuellement, un carbonyle puis un bicycle formé d'un hétérocycle azoté à cinq ou six chaînons fusionné avec un benzène, tel que l'indoline ou l'isoquinoléine. Néanmoins, seuls des composés présentant une chaîne alkylène de 2 à 4 atomes de carbone sont décrits.

Par ailleurs, aucun composé de type isoindoline n'est décrit.

Il a maintenant été découvert, et ce de façon totalement inattendue, que les composés selon l'invention, constituant une nouvelle série de dérivés d'arylpipérazine présentaient une forte affinité pour le récepteur D3 de la dopamine. Contrairement aux composés décrits dans WO 01/49769, les dérivés selon l'invention présentent un cycle indoline, une chaine alkylène comprenant 5 ou 6 atomes de carbone et un groupe phényle non substitué par un atome d'halogène. Ils sont, en outre des ligands sélectifs de D3.

Ces composés sont utiles comme médicaments en neuropsychiatrie, notamment dans le traitement des états psychotiques ou dépressifs, dans le traitement de désordres moteurs tels que les dyskinésies ou tremblements essentiels. En outre, ils sont utiles dans le traitement des états de dépendance à la nicotine, la cocaïne, l'alcool, les morphiniques ainsi que pour faciliter la désaccoutumance chez les sujets pharmacodépendants.

La présente invention concerne donc les composés de formule (I) : 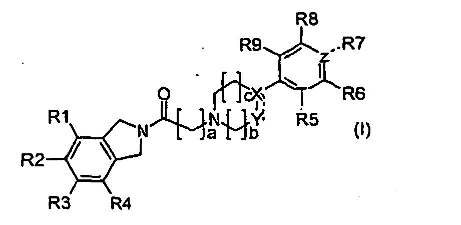 dans laquelle :
■ a = 5 ou 6 ;
■ b et c identiques ou différents représentent 1 ou 2 ;
■ X ---- Y représente >N-CH₂-, >C=CH-, >CH-CH₂- ;
■ Z et T identiques ou différents représentent indépendamment un atome d'azote ou de carbone ;
■ ----- est absent ou représente une simple liaison, étant entendu que lorsque Z et/ou T représente un atome d'azote, - est absent ;
■ R1, R2, R3 et R4 représentent chacun indépendamment un atome d'hydrogène ou d'halogène ou un groupement hydroxy, alkyle, monofluoroalkyle, polyfluoroalkyle, alkoxy, polyfluoroalkoxy, alkylsulfanyle, polyfluoroalkylsulfanyle, cyano, -OS(O)ₘ-Alkyle, -NRR', -COOR, -COR, -CONRR', -C(OH)RR', -alkylC(OH)RR', -alkylCOOR, -alkylCOR, -alkylCONRR', -alkylNRR', -OalkylNRR', -Oalkyl(NRR')COOR ou hétérocycle saturé ou insaturé ou bien deux des R1, R2, R3 et R4 adjacents sont reliés entre eux pour former un cycle hydrocarboné ou un hétérocycle saturé ou insaturé, fusionné au noyau phényle auquel ils sont rattachés ;
■ R5, R6, R7, R8 et R9 représentent chacun indépendamment un atome d'hydrogène ou un groupement hydroxy, alkyle, monofluoroalkyle, polyfluoroalkyle, alkoxy, polyfluoroalkoxy, alkylsulfanyle, polyfluoroalkylsulfanyle, cyano, -NRR', -COOR, -COR, -CONRR' ou bien deux des R5, R6, R7, R8 et R9 adjacents sont reliés entre eux pour former un cycle hydrocarboné ou un hétérocycle saturé ou insaturé, fusionné au noyau phényle auquel ils sont rattachés ;
où R, R' identiques ou différents représentent indépendamment un atome d'hydrogène, un groupe alkyle éventuellement substitué par un ou plusieurs groupes choisis parmi OH, NRR', OR, COOR ou R et R' sont reliés entre eux pour former un polyméthylène ou un oxapolyméthylène ;
m= 0, 1 ou 2 ;
étant entendu que lorsque T=Z=N, R5 et R7 sont absents, R6 et R8 sont choisis indépendamment parmi un atome d'hydrogène ou un groupement alkyle, monofluoroalkyle, polyfluoroalkyle, alkoxy, polyfluoroalkoxy, alkylsulfanyle, polyfluoroalkylsulfanyle, cyano, -COOR, -COR, -CONRR' et R9 est choisi parmi un atome d'hydrogène ou un groupement hydroxy, alkyle, monofluoroalkyle, polyfluoroalkyle, alkoxy, polyfluoroalkoxy, alkylsulfanyle, polyfluoroalkylsulfanyle, -NRR', -COOR, -COR, -CONRR'
ou bien R8 et R9 sont reliés entre eux pour former un cycle hydrocarboné ou un hétérocycle saturé ou insaturé, fusionné au noyau phényle auquel ils sont rattachés ;
ainsi que leurs stéréoisomères ou leur mélanges, leurs formes tautomères, leurs hydrates, solvates, leurs sels et esters pharmaceutiquement acceptables,

De préférence, a = 5.

De préférence, b et c représentent 1.

De préférence, X ---- Y représente >N-CH₂-.

De préférence, Z=T=C ou Z=T=N.

De préférence, R1, R2, R3, R4 représentent indépendamment un atome d'hydrogène ou d'halogène ou un groupe alkoxy, -OS(O)ₘ-Alkyle, cyano, -COOR, -COR, -CONRR', -C(OH)RR', -OalkylNRR'

De préférence, lorsque T=Z=C, R5, R6, R7, R8, R9 représentent indépendamment un atome d'hydrogène ou un groupement alkoxy, alkyle, cyano, polyfluoroalkoxy, tel qu'un groupe perfluoroalkoxy, tel que trifluorométhoxy, ou un groupement polyfluoroalkyle, tel qu'un groupe perfluoroalkyle, tel que trifluorométhyle, ou bien deux des R5, R6, R7, R8 et R9 adjacents sont reliés entre eux pour former un cycle hydrocarboné ou un hétérocycle saturé ou insaturé, fusionné au noyau phényle auquel ils sont rattachés, tel que un cycle benzocycloheptène ou benzofurane.

De préférence, lorsque T=Z=N, R5 et R7 sont absents, R6 et R8 sont choisis indépendamment parmi un atome d'hydrogène ou un groupement alkyle, monofluoroalkyle, polyfluoroalkyle, alkoxy, polyfluoroalkoxy, alkylsulfanyle, polyfluoroalkylsulfanyle, cyano, -COOR, -COR, -CONRR' et R9 est choisi parmi un atome d'hydrogène ou un groupement hydroxy, alkyle, monofluoroalkyle, polyfluoroalkyle, alkoxy, polyfluoroalkoxy, alkylsulfanyle, polyfluoroalkylsulfanyle, -NRR', -COOR, -COR, -CONRR' ou bien R8 et R9 sont reliés entre eux pour former un cycle hydrocarboné ou un hétérocycle saturé ou insaturé, fusionné au noyau phényle auquel ils sont rattachés ;

Plus préférentiellement, les composés de formule (1) sont choisis parmi les composés de formule (I') : 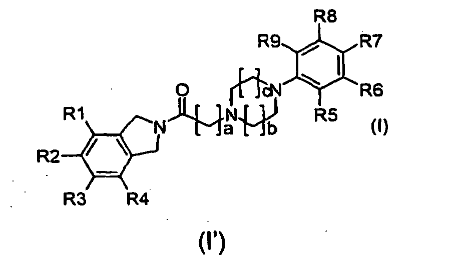 dans laquelle a, b et c, R1, R2, R3 et R4, R5, R6, R7, R8 et R9 sont tels que définis en formule (I),
ainsi que leurs stéréoisomères ou leurs mélanges, leurs formes tautomères, leurs hydrates, solvates, leurs sels et esters pharmaceutiquement acceptables.

Les composés de formule (I) peuvent être notamment choisis parmi :
- 1-(5-méthoxyisoindolin-2-yl)-6-[4-(3-trifluorométhylphényl)pipérazin-1-yl]hexan-1-one
- 1-(5-méthanesutfonyloxyisoindolin-2-yl)-6-[4-(3-trifluorométhylphényl)-pipérazin-1-yl]hexan-1-one
- chlorhydrate de 1-(5-cyanoisoindolin-2-yl)-6-[4-(3-trifluorométhylphényl)-pipérazin-1-yl]-hexan-1-one
- 1-isoindolin-2-yl-6-[4-(3-trifiuorométhoxyphényl)pipérazin-1-yl]hexan-1-one
- 1-isoindolin-2-yl-6-[4-(6,7,8,9-tétrahydro-5*H*-benzocyloheptén-2-yl)pipérazin-1-yl]hexan-1-one
- 1-(4-fluoroisoindolin-2-yl)-6-[4-(3-trifluorométhylphényl)pipérazin-1-yl]hexan-1-one
- 1-(5-fluoroisoindolin-2-yl)-6-[4-(3-trifluorométhylphényl)pipérazin-1-yl]hexan-1-one
- 1-(5-isopropoxyisoindolin-2-yl)-6-[4-(3-trifluorométhylphényl)pipérazin-1-yl]hexan-1-one
- 1-(4-chloroisoindolin-2-yl)-6-[4-(3-trifluorométhylphényl)pipérazin-1-yl]hexan-1-one
- 1-(6,7-dihydro-5*H*-[1,3]dioxolo[4,5-*f*]isoindol-6-yl)-6-[4-(3-trifluorométhylphényl)pipérazin-1-yl]hexan-1-one
- 1-(5-hydroxyméthylisoindolin-2-yl)-6-[4-(3-trifluorométhylphényl)-pipérazin-1-yl]hexan-1-one
- chlorhydrate de la 1-(5-méthoxycarbonylisoindolin-2-yl)-6-[4-(3-trifluorométhylphényl)pipérazin-1-yl]hexan-1-one
- 1-(5-carboxyisoindolin-2-yl)-6-[4-(3-trifluorométhylphényl)pipérazin-1-yl]hexan-1-one
- 1-[5-(2-hydroxyéthylaminocarbonyl)isoindolin-2-yl]-6-[4-(3-trifluorométhylphényl)pipérazin-1-yl]hexan-1-one
- 1-(5-morpholinocarbonylisoindolin-2-yl)-6-[4-(3-trifluorométhylphényl)-pipérazin-1-yl]hexan-1-one
- 1-(5-(pyrrolidin-1-ylcarbonylisoindolin-2-yl)-6-[4-(3-trifluorométhylphényl)-pipérazin-1-yl]hexan-1-one
- 1-(5-pipéridin-1-ylcarbonylisoindolin-2-yl)-6-[4-(3-trifluorométhylphényl)-pipérazin-1-yl]hexan-1-one
- 1-[5-(2-diméthylamino-éthylaminocarbonyl)isoindolin-2-yl]-6-[4-(3-trifluorométhylphényl)pipérazin-1-yl]hexan-1-one
- 1-[5-(4,5-dihydrooxazol-2yl)isoindolin-2-yl]-6-[4-(3-trifluorométhylphényl)-pipérazin-1-yl]hexan-1-one
- chlorhydrate de la 1-(5-morpholinoylméthylisoindolin-2-yl)-6-[4-(3-trifluorométhylphényl)pipérazin-1-yl]hexan-1-one
- 1-[5-(pyrrolidin-1-ylméthyl)isoindolin-2-yl]-6-[4-(3-trifluorométhylphényl)-pipérazin-1-yl]hexan-1-one
- 1-[5-(diméthylaminométhyl)isoindolin-2-yl]-6-[4-(3-trifluorométhylphényl)-pipérazin-1-yl]hexan-1-one
- 1-[5-(2-pipéridinoéthoxy)isoindolin-2-yl]-6-[4-(3-trifluorométhylphényl)-pipérazin-1-yl]hexan-1-one
- 1-[5-(3-pipéridinapropoxy)isoindolin-2-yl]-6-[4-(3-trifluorométhylphényl)-pipérazin-1-yl]hexan-1-one
- 1-[5-(3-pyrrolidinopropoxy)isoindolin-2-yl]-6-[4-(3-trifluorométhylphényl)-pipérazin-1-yl]hexan-1-one
- 1-(5-acétylisoindolin-2-yl)-6-[4-(3-trifluorométhyiphényl)pipérazin-1-yl]hexan-1-one
- 1-[5-(1-hydroxy-1-méthylethyl)isoindolin-2-yl]-6-[4-(3-trifluorométhylphényl)pipérazin-1-yl]hexan-1-one
- chlorhydrate de la 1-[5-(1-hydroxyéthyl)isoindolin-2-yl]-6-[4-(3-trifluorométhylphényl)pipérazin-1-yl]hexan-1-one
- 1-(5-aminocarbanylisoindolin-2-yl)-6-[4-(3-trifluorométhylphényl)-pipérazin-1-yl]hexan-1-one
- chlorhydrate de la 1-isoindolin-2-yl-6-[4-(3-trifluorométhylphényl)-pipérazin-1-yl]hexan-1-one
- 1-isoindolin-2-yl-6-[4-(2-méthoxyphényl)pipérazin-1-yl]hexan-1-one
- 1-isoindolin-2-yl-6-[4-(6,7,8,9-tétrahydro-5*H*-benzocyloheptén-1-yl)pipérazin-1-yl]hexan-1-one
- 1-isoindolin-2-yl-6-[4-(2,3-diméthylphényt)pipérazin-1-yl]hexan-1-one
- 1-isoindolin-2-yl-6-[4-benzofuran-7-ylpipérazin-1-yl]hexan-1-one
- 1-isoindolin-2-yl-6-[4-(2-cyano-3-trifluorométhylphényl)pipérazin-1-yl]hexan-1-one
- 1-isoindolin-2-yl-6-(4-indan-5ylpipérazin-1-yl)hexan-1-one
- 1-isoindolin-2-yl-6-(4-indan-4ylpipérazin-1-yl)hexan-1-one
- 1-isoindolin-2-yl-6-[4-(5,6,7,8-tétrahydronaphtalén-1-yl)piperazin-1-yl]hexan-1-one
- 1-isoindolin-2-yl-6-[4-(3-méthanesuffonyloxyphériyl)pipérazin-1-yl]hexan-1-one
- 1-isoindolin-2-yl-6-[4-(3,4-dihydro-2*H*-benzo[*b*][1,4]dioxépin-6-yl)pipérazin-1-yl]hexan-1-one
- 1-isoindolin-2-yl-6-[4-(3-trifluorométhyl-5-cyanophényl)pipérazin-1-yl]hexan-1-one
- 1-isoindolin-2-yl-6-[4-(5,6,7 ,8-tétrahydronaphtalén-2-yl)-piperazin-1-yl]hexan-1-one
- 1-isoindolin-2-yl-6-[4-(3-isopropoxyphényl)pipérazin-1-yl]hexan-1-one
- 1-isoindolin-2-yl-6-[4-(2-cyano-3-méthylphényl)pipérazin-1-yl]hexan-1-one
- 1-isoindolin-2-yl-6-[4-(2-cyano-3-isopropoxy)pipérazin-1-yl]hexan-1-one
- 1-(5,6-diméthoxyisoindolin-2-yl)-6-[4-(3-trifluorométhylphényl)pipérazin-1-yl]hexan-1-one
- 1-isoindolin-2-yl-6-[4-(2-cyanophényl)pipérazin-1-yl]hexan-1-one
- 1-isoindolin-2-yl-6-[4-(2-méthyl-phényl)pipérazin-1-yl]hexan-1-one
- 1-isoindolin-2-yl-6-(4-phényl-3,6-dihydro-2*H*-pyridin-1-yl)hexan-1-one
- 1-isoindolin-2-yl-6-[4-(3-trifluorométhylphényl)-3,6-dihydro-2*H*-pyridïn-1-yl]hexan-1-one
- 1-isoindolin-2-yl-6-[4-(3-trifluorométhylphényl)pipéridin-1-yl]hexan-1-one
- 1-isoindolin-2-yl-6-[4-(2-cyano-3-méthylphényl)-3,6-dihydro-2*H*-pyridin-1-yl]hexan-1-one
- 1-isoindolin-2-yl-6-[4-(2-cyano-3-méthylphényl)pipéridin-1-yl]hexan-1-one,
ainsi que leurs stéréoisomères ou leurs mélanges, leurs formes tautomères , leurs hydrates, solvates, leurs sels, formes libres et esters pharmaceutiquement acceptables.

Plus préférentiellement, les composés de formule (1) peuvent être choisis parmi :
- 1-(5-méthoxyisoindolin-2-yl)-6-[4-(3-trifluorométhylphényl)pipérazin-1-yl]hexan-1-one
- chlorhydrate de 1-(5-cyanoisoindolin-2-yl)-6-[4-(3-trifluorométhylphényl)-pipérazin-1-yl]-hexan-1-one
- 1-(5-fluoroisoindolin-2-yl)-6-[4-(3-trifluorométhyl-phényl)pipérazin-1-yl]hexan-1-one
- 1-(6,7-dihydro-5*H*-[1,3]dioxolo[4,5-f]isoindol-6-yl)-6-[4-(3-trifluorométhylphényl)pipérazin-1-yl]hexan-1-one
- chlorhydrate de la 1-(5-méthoxycarbonylisoindolin-2-yl)-6-[4-(3-trifluorométhylphényl)pipérazin-1-yl]hexan-1-one
- 1-[5-(3-pipéridinopropoxy)isoindolin-2-yl]-6-[4-(3-trifluorométhylphényl)pipérazin-1-yl]hexan-1-one
- 1-isoindolin-2-yl-6-[4-(5,6,7,8-tétrahydro-naphtalén-1-yl)piperazin-1-yl]hexan-1-one
- 1-isoindolin-2-yl-6-[4-(3-trifluorométhylphényl)-3,6-dihydro-2H-pyridin-1-yl]hexan-1-one
- 1-isoindolin-2-yl-6-[4-(3-trifluorométhyl-phényl)pipéridin-1-yl]hexan-1-one
ainsi que leurs stéréoisomères ou leurs mélanges, leurs formes tautomères, leurs hydrates, solvates, leurs sels, formes libres et esters pharmaceutiquement acceptables.

Selon la présente invention, les radicaux Alkyle représentent des radicaux hydrocarbonés saturés, en chaîne droite ou ramifiée, de 1 à 20 atomes de carbone, de préférence de 1 à 5 atomes de carbone.

On peut notamment citer, lorsqu'ils sont linéaires, les radicaux méthyle, éthyle, propyle, butyle, pentyle, hexyle, octyle, nonyle, décyle, dodécyle, hexadécyle, et octadécyle.

On peut notamment citer, lorsqu'ils sont ramifiés ou substitués par un ou plusieurs radicaux alkyle, les radicaux isopropyle, tert-butyl, 2-éthylhexyle, 2-méthylbutyle, 2-méthylpentyle, 1-méthylpentyle et 3-méthylheptyle.

Les radicaux Alkoxy selon la présente invention sont des radicaux de formule -O-Alkyle, l'alkyle étant tel que défini précédemment.

Parmi les atomes d'Halogène, on cite plus particulièrement les atomes de fluor, de chlore, de brome et d'iode, de préférence le fluor.

Les radicaux Alkényle représentent des radicaux hydrocarbonés, en chaîne droite ou linéaire, et comprennent une ou plusieurs insaturations éthyléniques. Parmi les radicaux Alkényle, on peut notamment citer les radicaux allyle ou vinyle.

Les radicaux Alkynyle représentent des radicaux hydrocarbonés, en chaîne droite ou linéaire, et comprennent une ou plusieurs insaturations acétyléniques. Parmi les radicaux Alkynyle, on peut notamment citer l'acétylène.

Le radical Cycloalkyle est un radical hydrocarboné mono-, bi- ou tricyclique saturé ou partiellement insaturé, non aromatique, de 3 à 10 atomes de carbone, tel que notamment le cyclopropyle, cyclopentyle, cyclohexyle ou adamantyle, ainsi que les cycles correspondants contenant une ou plusieurs insaturations.

Aryle désigne un système aromatique hydrocarboné, mono ou bicyclique de 6 à 10 atomes de carbone.

Parmi les radicaux Aryle, on peut notamment citer le radical phényle ou naphtyle, plus particulièrement substitué par un moins un atome d'halogène.

Parmi les radicaux -AlkyleAryle, on peut notamment citer le radical benzyle ou phénétyle.

Les radicaux Hétéroaryles désignent les systèmes aromatiques comprenant un ou plusieurs hétéroatomes choisis parmi l'azote, l'oxygène ou le soufre, mono ou bicyclique, de 5 à 10 atomes de carbone. Parmi les radicaux Hétéroaryles, on pourra citer le pyrazinyle, le thiényle, l'oxazolyle, le furazanyle, le pyrrolyle, le 1,2,4-thiadiazolyle, le naphthyridinyle, le pyridazinyle, le quinoxalinyle, le phtalazinyle, l'imidazo[1,2-a]pyridine, l'imidazo[2,1-b]thiazolyle, le cinnolinyle, le triazinyle, le benzofurazanyle, l'azaindolyle, le benzimidazolyle, le benzothiényle, le thiénopyridyle, le thiénopyrimidinyle, le pyrrolopyridyle, l'imidazopyridyle, le benzoazaindole, le 1,2,4-triazinyle, le benzothiazolyle, le furanyle, l'imidazolyle, l'indolyle, le triazolyle, le tétrazolyle, l'indolizinyle, l'isoxazolyle, l'isoquinolinyle, l'isothiazolyle, l'oxadiazolyle, le pyrazinyle, le pyridazinyle, le pyrazolyle, le pyridyle, le pyrimidinyle, le purinyle, le quinazolinyle, le quinolinyle, l'isoquinolyle, le 1,3,4-thiadiazolyle, le thiazolyle, le triazinyle, l'isothiazolyle, le carbazolyle, ainsi que les groupes correspondants issus de leur fusion ou de la fusion avec le noyau phényle.

L'expression « sels pharmaceutiquement acceptables » fait référence aux sels d'addition acide relativement non toxiques, inorganiques et organiques, et les sels d'addition de base, des composés de la présente invention. Ces sels peuvent être préparés *in situ* pendant l'isolement final et la purification des composés. En particulier, les sels d'addition acide peuvent être préparés en faisant réagir séparément le composé purifié sous sa forme épurée avec un acide organique ou inorganique et en isolant le sel ainsi formé. Parmi les exemples de sels d'addition acide on trouve les sels bromhydraté, chlorhydrate, sulfate, bisulfate, phosphate, nitrate, acétate, oxalate, valerate, oléate, palmitate, stéarate, laurate, borate, benzoate, lactate, phosphate, tosylate, citrate, maléate, fumarate, succinate, tartrate, naphthylate, mésylate, glucoheptanate, lactobionate, sulfamates, malonates, salicylates, propionates, méthylènebis-beta-hydroxynaphtoates, acide gentisique, iséthionates, di-p-toluoyltartrates, méthanesulfonates, éthanesulfonates, benzènesulfonates, p-toluènesulfonates, cyclohexyl sulfamates et quinateslaurylsulfonate, et analogues. (Voir par exemple S.M. Berge et al. « Pharmaceutical Salts » J. Pharm. Sci, 66 : p.1-19 (1977) qui est incorporé ici en référence). Les sels d'addition acide peuvent également être préparés en faisant réagir séparément le composé purifié sous sa forme acide avec une base organique ou inorganique et en isolant le sel ainsi formé. Les sels d'addition acide comprennent les sets aminés et métalliques. Les sels métalliques adaptés comprennent les sels de sodium, potassium, calcium, baryum, zinc, magnésium et aluminium. Les sels de sodium et de potassium sont préférés. Les sels d'addition inorganiques de base adaptés sont préparés à partir de bases métalliques qui comprennent hydrure de sodium, hydroxyde de sodium, hydroxyde de potassium, hydroxyde de calcium, hydroxyde d'aluminium, hydroxyde de lithium, hydroxyde de magnésium, hydroxyde de zinc. Les sels d'addition aminés de base adaptés sont préparés à partir d'amines qui ont une alcalinité suffisante pour former un sel stable, et de préférence comprennent les amines qui sont souvent utilisées en chimie médicinale en raison de leur faible toxicité et de leur acceptabilité pour l'usage médical: ammoniac, éthylènediamine, N-méthyl-glucamine, lysine, arginine, ornithine, choline, N,N'-dibenzyléthylenediamine, chloroprocaïne, diéthanolamine, procaïne, N-benzylphénéthylamine, diéthylamine, pipérazine, tris(hydroxyméthyl)-aminométhane, hydroxyde de tétraméthylammonium, triéthylamine, dibenzylamine, éphénamine, dehydroabiétylamine, N-éthyl-pipéridine, benzylamine, tétraméthylammonium, tétraéthylammonium, méthylamine, diméthylamine, triméthylamine, éthylamine, acides aminés basiques, par exemple lysine et arginine, dicyclohexylamine, et analogues.

L'invention se rapporte également aux stéréoisomères ou leurs mélanges, les formes tautomères, les hydrates, solvates, sels et esters pharmaceutiquement acceptables des composés de formule (I).

Les composés de l'invention de formule (I) définis tels que précédemment, possédant une fonction suffisamment acide ou une fonction suffisamment basique ou les deux, peuvent inclure les sels correspondants d'acide organique ou minéral ou de base organique ou minérale pharmaceutiquement acceptables.

Les composés de formule générale (I) peuvent être préparés par application ou adaptation de toute méthode connue en soi de et/ou à la portée de l'homme du métier, notamment celles décrites par Larock dans Comprehensive Organic Transformations, VCH Pub., 1989, ou par application ou adaptation des procédés décrits dans les exemples qui suivent.

Selon un autre objet, la présente invention concerne donc également le procédé de préparation des composés de formule (I) précédemment décrits comprenant l'étape de couplage des composés de formule (II) et (III) correspondants selon le schéma suivant : 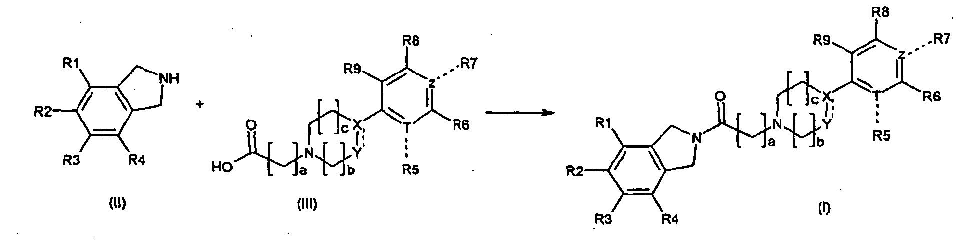 où, dans la formule (II) et (lll), R1, R2, R3, R4, R5, R6, R7, R8, R9, X, Y, Z, T, ..., ..., a, b et c ont la même signification que dans la formule (1).

Généralement, cette condensation est effectuée à l'aide d'agents de couplage peptidique tels qu'un carbodiimide (dicyclohexylcarbodiimide, diisopropylcarbodiimide, 3-éthyl-1-(3-diméthylaminopropyl)carbodümide par exemple) en présence d'un catalyseur (1-hydroxybenzotriazole, 4-diméthylaminopyridine) dans un solvant inerte tel que l'acétate d'éthyle, l'acétonitrile, le dichlorométhane ou un mélange de ces solvants à un température comprise entre -10°C et la température d'ébullition du milieu réactionnel. Cette condensation peut également être effectuée en activant d'abord l'acide sous forme de chlorure d'acide à l'aide d'un agent de chloruration (chlorure d'oxalyle, chlorure de thionyle, phosgène, diphosgène, triphosgène, oxychlorure de phosphore par exemple), sous forme d'anhydride symétrique ou d'anhydride mixte avec un chloroformate (chloroformate d'isobutyle, d'éthyle ou d'isopropyle par exemple), sous forme d'ester de phénol (paranitrophénol, parachlorophénol ou pentafluorophénol par exemple) ou sous forme d'ester d'hydroxyhétérocycle (N-hydroxysuccinimide ou 1-hydroxybenzotriazole par exemple). L'acide ainsi activé est ensuite opposé au composé de formule (II) éventuellement en présence d'une base organique ou minérale (triéthylamine, 4-diméthylaminopyridine, carbonate de potassium, carbonate de sodium, hydrogénocarbonate de potassium, hydrogénocarbonate de sodium) dans un solvant inerte tel que l'acétate d'éthyle; l'acétonitrile, le dichlorométhane ou un mélange de ces solvants à un température comprise entre -10°C et la température d'ébullition du milieu réactionnel.

Les dérivés de formule (II) dans laquelle R1, R2, R3 et R4 ont la même signification que dans la formule (I) sont soit disponibles commercialement, soit préparés à partir de méthodes décrites dans la littérature (EP 0343560, Syn. Commun. (1995), 3255, Bioorg. Med. Chem. Lett. (2001), 11, 685-688).

Les acides de formule (III) dans laquelle R5, R6, R7, R8, R9, a, b et c ont la même signification que dans la formule (I) peuvent être préparés à partir des esters (IV) dans laquelle R5, R6, R7, R8, R9, X, Y, Z, T, ..., ..., a, b et c ont la même signification que dans la formule (I) et R10 représente un alkyle ou un benzyle éventuellement substitué.

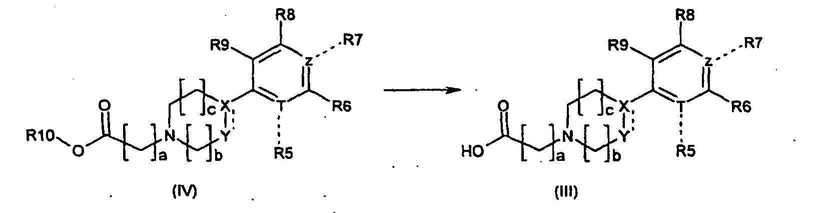

Cette réaction peut être réalisée en présence d'une solution aqueuse de base inorganique (soude, potasse, lithine) éventuellement en présence d'eau oxygénée et d'un cosolvant organique tel qu'un alcool (méthanol, éthanol par exemple) ou un éther (dioxane, tétrahydrofurane) ou un mélange de ces solvants. Cette réaction s'effectue à une température comprise entre -20°C et la température d'ébullition du milieu réactionnel. Lorsque R10 représente un benzyle éventuellement substitué, l'acide peut être obtenu par hydrogénolyse au moyen de dihydrogène ou par transfert (acide formique éventuellement en présence dé triéthylamine, formate d'ammonium, cyclohexène, cyclohexadiène par exemple) en présence d'un métal de transition (palladium par exemple) dans un solvant organique tel qu'un alcool (méthanol, éthanol, isopropanol), un ester (acétate d'éthyle, acétate d'isopropyle) ou un solvant halogéné (chloroforme par exemple) à une température comprise entre 10°C et 150°C et à une pression comprise entre 1 et 100 bar. Lorsque R10 représente un benzyle substitué par un ou plusieurs alkoxy, l'acide peut également être obtenu par oxydation à l'aide de nitrate de cérium ou de dichlorodicyanobenzoquinone.

Les esters de formule (IV) dans laquelle R5, R6, R7, R8, R9, X, Y, Z, T, ..., ..., a, b et c ont la même signification que dans la formule (I) et R10 représente un alkyle ou un benzyle éventuellement substitué peuvent être préparés par condensation des esters de formule (V) dans laquelle a = 5 ou 6, R10 représente un alkyle ou un benzyle éventuellement substitué et Hal représente un atome d'halogène, de préférence le brome avec des amines de formule (VI) dans laquelle R5, R6, R7, R8, R9, X, Y, Z, T, ..., ..., b et c ont la même signification que dans la formule (I).

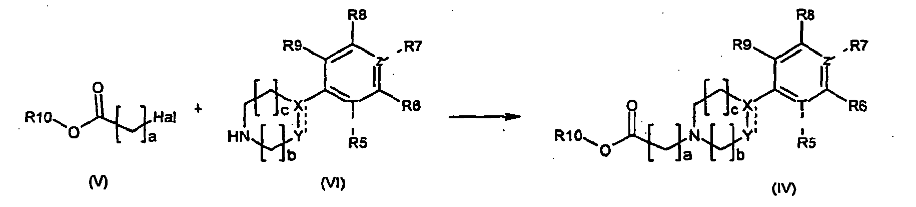

Cette réaction peut être réalisée en présence d'une base inorganique (carbonate de potassium, carbonate de césium) et en présence ou non d'une quantité catalytique d'iodure de potassium dans un solvant tel que l'acétonitrile, le N,N-diméthylformamide, l'acétonitrile à une température comprise entre 15°C et la température d'ébullition du milieu réactionnel.

Les amines cycliques de formule (VI) dans laquelle R5, R6, R7, R8, R9, Z, T, ..., ..., b et c ont la même signification que dans la formule (I) et X=N, Y=C peuvent être préparés par dialkylation d'anilines de formule (VII) dans laquelle R5, R6, R7, R8, R9, Z, T, ... ont la même signification que dans la formule (I) et d'une amine de formule (VIII) dans laquelle b et c ont la même signification que dans la formule (I) et Hal représente un atome d'halogène, de préférence le chlore :

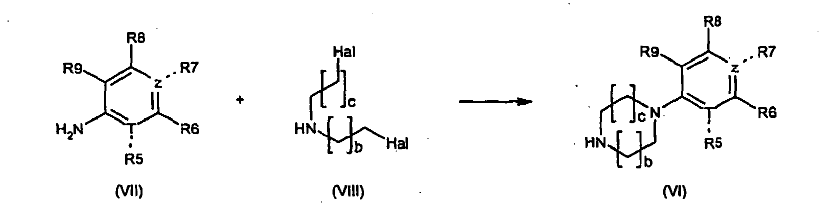

Cette réaction peut être réalisée par chauffage dans un solvant aromatique (toluène, chlorobenzène par exemple) ou dans un alcool (éthanol, butanol par exemple).

Les amines cycliques de formule (VI) dans laquelle R5, R6, R7, R8, R9, X, Y, Z, T, ..., ..., b et c ont la même signification que dans la formule (1) peuvent également être préparés par substitution d'halogénoaromatiques, de préférence fluoroaromatiques de formule (VII) dans laquelle Hal représente un atome d'halogène, de préférence le fluor, Z, T, R5, R6, R7, R8 et R9, ..., ont la même signification que dans la formule (I) lorsque ce dernier porte un groupement électroattracteur avec une diamine de formule (IX) dans laquelle b et c ont la même signification que dans la formule (I)

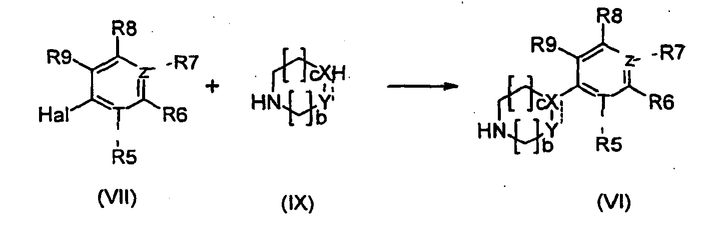

Généralement, cette réaction est réalisée par chauffage dans un solvant organique.

Les amines cycliques de formule (VI) peuvent également être préparées par substitution d'halogénoaromatique, l'halogène étant de préférence un iode, un brome ou un chlore ou d'un pseudohalogénoaromatique, le pseudohalogène étant un arylsulfonate ou un alkylsulfonate (paratoluènesulfonate, mésylate, triflate par exemple) de formule (VII).

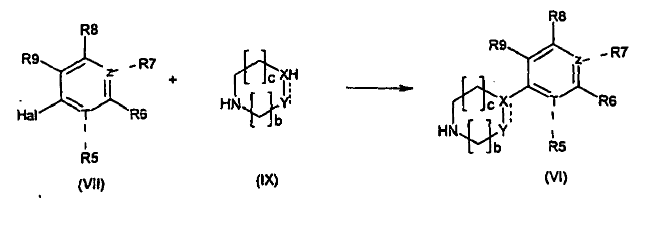

Cette réaction peut éventuellement être catalysée par des métaux de transition.

Les dérivés de formule (I) peuvent être également obtenus à partir de dérivés d'amine cyclique de formule (VI) dans laquelle R5, R6, R7, R8, R9, X, Y, Z, T, ..., ..., b et c ont la même signification que dans la formule (I) et de dérivés halogénés de formule (X) dans laquelle R1, R2, R3, R4 et a ont la même signification que dans la formule (I) et Hal représente un halogène, l'halogène étant choisi de préférence un atome de chlore ou de brome :

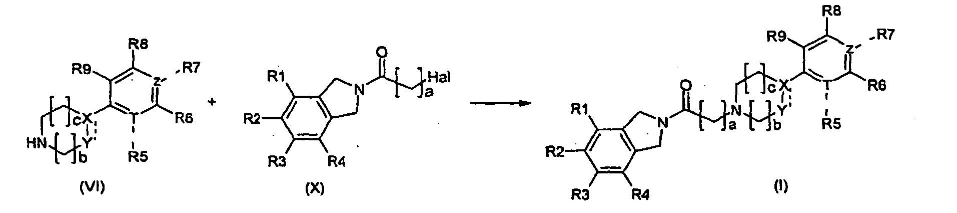

La substitution est effectuée en présence d'une base inorganique telle qu'un carbonate ou un hydrogénocarbonate (carbonate de potassium, carbonate de césium par exemple), dans un solvant organique tel que l'acétonitrile, le N,N-diméthyformamide ou le diméthysulfoxyde à une température comprise entre 0°C et la température d'ébullition du milieu réactionnel.

Les dérivés de formule (X) dans laquelle R1, R2, R3, R4 et a ont la même signification que dans la formule (I) et Hal représente un halogène, l'halogène étant choisi de préférence un atome de chlore ou de brome peuvent être préparés à partir de dérivés de l'isoindoline de formule (XI) dans laquelle R1, R2, R3 et R4 ont la même signification que dans la formule (I) et d'un acide de formule (XI) dans laquelle l'halogène est un atome de chlore ou de brome et a = 5 ou 6 :

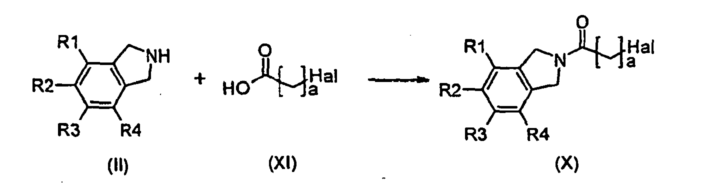

Généralement, cette condensation est effectuée à l'aide d'agents de couplage peptidique tels qu'un carbodiimide (dicyclohexylcarbodiimide, diisopropylcarbodiimide, 3-éthyl-1-(3-diméthylaminopropyl)carbodiimide par exemple) en présence d'un catalyseur (1-hydroxybenzotriazole, 4-diméthylaminopyridine) dans un solvant inerte tel que l'acétate d'éthyle, l'acétonitrile, le dichlorométhane ou un mélange de ces solvants à un température comprise entre -10°C et la température d'ébullition du milieu réactionnel. Cette condensation peut également être effectuée en activant d'abord l'acide sous forme de chlorure d'acide à l'aide d'un agent de chloruration (chlorure d'oxalyle, chlorure de thionyle, phosgène, diphosgène, triphosgène, oxychlorure de phosphore par exemple), sous forme d'anhydride symétrique ou d'anhydride mixte avec un chloroformate (chloroformate d'isobutyle, d'éthyle ou d'isopropyle par exemple), sous forme d'ester de phénol (paranitrophénol, parachlorophénol ou pentafluorophénol par exemple) ou sous forme d'ester d'hydroxyhétérocycle (N-hydroxysuccinimide ou 1-hydroxybenzotriazole par exemple). L'acide ainsi activé est ensuite opposé au composé de formule (II) éventuellement en présence d'une base organique ou minérale (triéthylamine, 4-diméthylaminopyridine, carbonate de potassium, carbonate de sodium, hydrogénocarbonate de potassium, hydrogénocarbonate de sodium) dans un solvant inerte tel que l'acétate d'éthyle, l'acétonitrile, le dichlorométhane ou un mélange de ces solvants à une température comprise entre -10°C et la température d'ébullition du milieu réactionnel.

Les intermédiaires réactionnels de formule V, VII, VIII, IX, XI sont disponibles commercialement ou peuvent être préparés par l'homme du métier par application ou adaptation de méthodes connues en soi.

Le procédé selon l'invention peut également comprendre l'étape ultérieure d'isolation des produits de formule (I) obtenus.

Dans les réactions décrites ici, il peut être nécessaire de protéger les groupes fonctionnels réactifs, par exemples les groupes hydroxy, amino, imino, thio, carboxy, lorsqu'ils sont souhaités dans le produit final, pour éviter leur participation indésirable dans les réactions. Les groupes de protection traditionnels peuvent être utilisés conformément à la pratique standard; pour des exemples voir T.W. Greene et P.G.M. Wuts dans Protective Groups in Organic Chemistry, John Wiley and Sons, 1991 ; J.F.W. McOmie in Protective Groups in Organic Chemistry, Plenum Press, 1973.

Le composé ainsi préparé peut être récupéré à partir du mélange de la réaction par les moyens traditionnels. Par exemple, les composés peuvent être récupérés en distillant le solvant du mélange de la réaction ou si nécessaire après distillation du solvant du mélange de la solution, en versant le reste dans de l'eau suivi par une extraction avec un solvant organique immiscible dans l'eau, et en distillant le solvant de l'extrait. En outre, le produit peut, si on le souhaite, être encore purifié par diverses techniques, telles que la recristallisation, la reprécipitation ou les diverses techniques de chromatographie, notamment la chromatographie sur colonne ou la chromatographie en couche mince préparative.

Il sera apprécié que les composés utiles selon la présente invention peuvent contenir des centres asymétriques. Ces centres asymétriques peuvent être indépendamment en configuration R ou S. II apparaîtra à l'homme du métier que certains composés utiles selon l'invention peuvent également présenter une isomérie géométrique. On doit comprendre que la présente invention comprend des isomères géométriques individuels et des stéréoisomères et des mélanges de ceux-ci, incluant des mélanges racémiques, de composés de formule (I) ci-dessus. Ce type d'isomères peuvent être séparés de leurs mélanges, par l'application ou l'adaptation de procédés connus, par exemple des techniques de chromatographie ou des techniques de recristallisation, ou ils sont préparés séparément à partir des isomères appropriés de leurs intermédiaires.

Aux fins de ce texte, il est entendu que les formes tautomériques sont comprises dans la citation d'un groupe donné, par exemple thio/mercapto ou oxo/hydroxy.

Les sels d'additions acides sont formés avec les composés utiles selon l'invention dans lesquels une fonction de base tels qu'un groupe amino, alkylamino ou dialkylamino est présente. Les sels d'addition acide pharmaceutiquement acceptables, c'est-à-dire non toxiques, sont préférés. Les sels sélectionnés sont choisis de façon optimale pour être compatibles avec les véhicules pharmaceutiques habituels et adaptés pour l'administration orale ou parentérale. Les sels d'addition acide des composés utiles selon cette invention peuvent être préparés par réaction de la base libre avec l'acide approprié, par l'application ou l'adaptation de procédés connus. Par exemple, les sels d'addition acide des composés utiles selon cette invention peuvent être préparés soit en dissolvant la base libre dans de l'eau ou dans une solution aqueuse alcoolisée ou des solvants adaptés contenant l'acide approprié et en isolant le sel en évaporant la solution, ou en faisant réagir la base libre et l'acide dans un solvant organique, auquel cas le sel se sépare directement ou peut être obtenu par concentration de la solution. Parmi les acides adaptés pour l'usage dans la préparation de ces sels on trouve acide chlorhydrique, acide bromhydrique, acide phosphorique, acide sulfurique, divers acides carboxyliques et sulfoniques organiques, tels que acide acétique, acide citrique, acide propionique, acide succinique, acide benzoïque, acide tartrique, acide fumarique, acide mandélique, acide ascorbique, acide malique, acide méthanesulfonique, acide toluène-sulfonique, acides gras, adipate, alginate, ascorbate, aspartate, benzènesulfonate, benzoate, propionate de cyclopentane, digluconate, dodécylsulfate, bisulfate, butyrate, lactate, laurate, sulfate de lauryle, malate, hydroiodide, 2-hydroxyéthanesulfonate, glycérophosphate, picrate, pivalate, pamoate, pectinate, persulfate, 3-phénylpropionate, thiocyanate, 2-naphtalène-sulfonate, undécanoate, nicotinate, hémisulfate, heptonate, hexanoate, camphorate, camphresulfonate et autres.

Les sels d'addition acide des composés utiles selon cette invention peuvent être régénérés à partir des sels par l'application ou l'adaptation de procédés connus. Par exemple, les composés parents utiles selon l'invention peuvent être régénérés à partir de leurs sels d'addition acide par traitement avec un alkali, par exemple une solution de bicarbonate de sodium aqueuse ou une solution d'ammoniac aqueuse.

Les composés utiles selon cette invention peuvent être régénérés à partir de leurs sels d'addition de base par l'application ou l'adaptation de procédés connus. Par exemple, les composés parents utiles selon l'invention peuvent être régénérés à partir de leurs sels d'addition de base par le traitement avec un acide, par exemple un acide chlorhydrique.

Les sels d'addition de base peuvent être formés lorsque le composé utile selon l'invention contient un groupe carboxyle, ou un bioisostère suffisamment acide. Les bases qui peuvent être utilisées pour préparer les sels d'addition de base comprennent de préférence celle qui produisent, lorsqu'elles sont associées à un acide libre, des sels pharmaceutiquement acceptables, c'est-à-dire des sels dont les cations ne sont pas toxiques pour le patient dans les doses pharmaceutiques des sels, de sorte que les effets inhibiteurs bénéfiques inhérents à la base libre ne soient pas annulés par les effets secondaires imputables aux cations. Les sels pharmaceutiquement acceptables, comprenant ceux dérivés des sels de métal alcalino-terreux, dans la portée de l'invention comprennent ceux dérivés des bases suivantes : hydrure de sodium, hydroxyde de sodium, hydroxyde de potassium, hydroxyde de calcium, hydroxyde d'aluminium, hydroxyde de lithium, hydroxyde de magnésium, hydroxyde de zinc, ammoniac, éthylènediamine, N-méthyl-glucamine, lysine, arginine, ornithine, choline, N,N'-dibenzyléthylènediamine, chloroprocaïne, diéthanolamine, procaïne, N-benzylphénéthylamine, diéthylamine, pipérazine, tris(hydroxyméthyl)aminométhane, hydroxyde de tétraméthylammonium et analogues.

Les composés utiles selon la présente invention peuvent être facilement préparés, ou formés pendant le processus de l'invention, sous forme de solvates (par exemple hydrates). Les hydrates des composés utiles selon la présente invention peuvent être facilement préparés par la recristallisation d'un mélange de solvant aqueux/organique, en utilisant des solvants organiques tels que dioxan, tétrahydrofuranne ou méthanol.

Les produits de bases ou les réactifs utilisés sont disponibles commercialement et/ou peuvent être préparés par l'application ou l'adaptation de procédés connus, par exemple des procédés tels que décrits dans les Exemples de Référence ou leurs équivalents chimiques évidents.

Selon la présente invention, les composés de formule (I) présentent une activité de ligand sélectif du récepteur D3.

La présente invention a également pour objet les compositions pharmaceutiques comprenant un composé selon l'invention avec un véhicule ou excipient pharmaceutiquement acceptable.

De préférence, ladite composition contient une quantité efficace du composé selon l'invention.

Selon un autre objet, la présente invention concerne également l'utilisation de composés de formule générale (I) pour la préparation de compositions pharmaceutiques destinées à agir comme ligand du récepteur D3 de la dopamine. De préférence, ledit ligand est un antagoniste de D3 ; encore plus préférentiellement un antagoniste sélectif de D3.

Selon un autre objet, la présente invention concerne également l'utilisation de composés de formule générale (1) pour la préparation de compositions pharmaceutiques destinées à prévenir et/ou traiter une affection neuropsychiatrique ou toute affection thérapeutique faisant intervenir le récepteur D3 de la dopamine. Lesdites affections sont choisies de préférence parmi la pharmacodépendance, les troubles de la sphère sexuelle, les troubles moteurs, la maladie de Parkinson, la psychose ou état psychotique, la dépression ou pharmacodépendance.

Selon l'invention, on entend par pharmacodépendance tout état lié à la désaccoutumance, l'abstinence et/ou à la désintoxication d'un sujet dépendant de tout agent, notamment les agents actifs thérapeutiques ; tels que les morphiniques, et/ou drogues telles que la cocaïne, l'héroïne, ou encore l'alcool et/ou la nicotine.

Selon l'invention, on entend notamment par troubles de la sphère sexuelle, l'impuissance, notamment l'impuissance masculine.

Selon l'invention, ladite prévention et/ou traitement de la maladie de Parkinson est de préférence un traitement complémentaire de la maladie de Parkinson.

Selon l'invention, on entend notamment par troubles moteurs les dyskinésies essentielles ou iatrogènes, et/ou les tremblements essentiels ou iatrogènes.

Selon un autre objet, la présente invention concerne également les méthodes de traitement thérapeutiques citées ci-dessus comprenant l'administration d'un composé selon l'invention à un patient qui en a besoin.

De préférence, ladite composition est administrée à un patient qui en a besoin.

Les compositions pharmaceutiques selon l'invention peuvent être présentées sous des formes destinées à l'administration par voie parentérale, orale, rectale, permuqueuse ou percutanée.

Elles seront donc présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, pour l'usage permuqueux.

Les excipients qui conviennent pour de telles administrations sont les dérivés de la cellulose ou de la cellulose microcristalline, les carbonates alcalino-terreux, le phosphate de magnésium, les amidons, les amidons modifiés, le lactose pour les formes solides.

Pour l'usage rectal, le beurre de cacao ou les stéarates de polyéthylèneglycol sont les excipients préférés.

Pour l'usage parentéral, l'eau, les solutés aqueux, le sérum physiologique, les solutés isotoniques sont les véhicules les plus commodément utilisés.

La posologie peut varier dans les limites importantes (0,5 mg à 1000 mg) en fonction de l'indication thérapeutique et de la voie d'administration, ainsi que de l'âge et du poids du sujet.

Les exemples suivants illustrent l'invention, sans toutefois la limiter. Les produits de départs utilisés sont des produits connus ou préparés selon des modes opératoires connus.

Les pourcentages sont exprimés en poids, sauf mention contraire.

### EXEMPLES

### Description des exemples :

Les points de fusion sont mesurés sur un appareil Büchi B-545.

Les spectres RMN du proton sont enregistrés un appareil Bruker AC 250 à 250,13 MHz. Les déplacements chimiques sont exprimés en ppm. Pour la multiplicité des signaux, les abréviations suivantes sont utilisées : s = singulet, d = doublet, t = triplet, q = quadruplet, m = multiplet et ms = massif. Les constantes de couplage sont exprimées en hertz (Hz).

Les chromatographies couche mince sont réalisées sur plaque de silice.

### Exemple 1 : préparation de la 1-(5-méthoxyisoindolin-2-yl)-6-[4-(3-trifluorométhy(phényl)pipérazin-1-yl]hexan-1-one

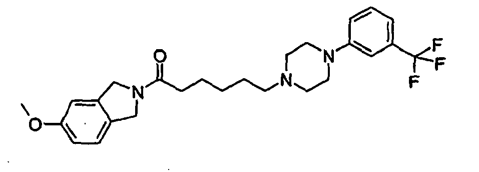

### Etape a : préparation du 6-[4-(3-trifluorométhylphényl)pipérazin-1-yl]hexanoate d'éthyle

Un mélange de 23 g (0,1 mol) de 1-(3-trifluorométhylphényl)pipérazine, de 150 mL d'acétonitrile, d'une quantité catalytique d'iodure de potassium, de 13,8 g (0,1 mol) de carbonate de potassium et de 22,3 g (0,1 mol) de 6-bromohexanoate d'éthyle est porté au reflux pendant 24 heures.
Le milieu réactionnel est concentré sous vide et est repris avec 100 mL d'oxyde de diéthyle. La phase organique est lavée avec 50 mL d'eau, séchée sur sulfate de magnésium, filtrée et concentrée sous vide. On obtient ainsi 37,2 g d'une huile visqueuse utilisée telle quelle dans les synthèses ultérieures.
Rf : 0,55 (acétate d'éthyle)
RMN ¹H (CDCl₃) : 7,35 (t, 1H, J = 7,5 Hz) ; 7,2 à 7,0 (ms, 3H) ; 4,15 (q, 2H, J = 7,5 Hz) ; 3,3 à 3,15 (ms, 4H) ; 2,65 à 2,5 (ms, 4H) ; 2,4 (t, 2H, J = 7,5 Hz) ; 3,2 (t, 2H, J = 7,5 Hz) ; 1,8 à 1,5 (ms, 4H) ; 1,5 à 1,3 (ms, 2H) ; 1,25 (t, 3H, J = 7,5 Hz)

### Etape b : préparation du chlorhydrate de l'acide 6-[4-(3-trifluorométhylphényl)pipérazin-1-yl]hexanoïque

Une solution de 16,6 g (50 mmol) de 6-[4-(3-trifluorométhylphényl)pipérazin-1-yl]hexanoate d'éthyle dans 200 mL d'éthanol est agitée et refroidie à environ 5°C. Après addition de 50 mL (50 mmol) d'une solution aqueuse normale de soude, le milieu réactionnel est agité pendant une nuit à température ambiante.
L'éthanol est concentré sous vidé. La phase aqueuse résiduelle est lavée avec 30 mL d'oxyde de diéthyle, refroidie et acidifiée avec 100 mL d'une solution aqueuse 0,5 N d'acide chlorhydrique. Le précipité formé est filtré, lavé avec de l'oxyde de diéthyle et séchée sous vide à 50°C sur pentaoxyde de phosphore. On obtient ainsi 9,5 g d'un solide blanc.
Point de fusion : 250°C
RMN ¹H (DMSO d₆) : 10,9 (s large, 1 H) ; 7,65 à 7,4 (ms, 1 H) ; 7,3 à 7,15 (ms, 2H) ; 7,15 à 7,05 (ms, 1H) ; 4,1 à 3,7 (ms, 2H) ; 3,65 à 3,3 (ms, 2H) ; 3,3 à 3,1 (ms, 2H) ; 3,1 à 2,8, (ms, 4H) ; 2,2 (t, 2H , J = 7,5 Hz) ; 1,85 à 1,6 (ms, 2H) ; 1,6 à 1,4 (ms, 2H) ; 1,4 à 1,2 (ms, 2H)

### Etape c : préparation de la 1-(5-méthoxyisoindolin-2-yl)-6-[4-(3-trifluorométhylphényl)pipérazin-1-yl]hexan-1-one

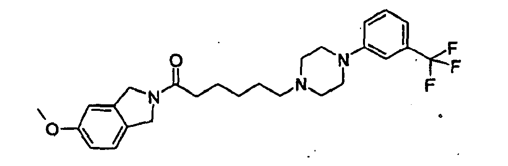

Dans un ballon, on introduit successivement 0,344 g (1 mmol) d'acide 6-[4-(3-trifluorométhylphényl)pipérazin-1-yl]hexanoïque, 0,149 g (1,1 mmol) d'hydroxybenzotriazole, 0,287 g (1 mmol) de chlorhydrate de 3-éthyl-1-(3-diméthylaminopropyl)carbodümide et 8 mL de dichlorométhane stabilisé sur amylène. Cette solution est agitée pendant 10 minutes à température ambiante puis on ajoute 0,15 g (1 mmol) de 5-méthoxyisoindoline et 0,31 mL (1,5 mmol) de triéthylamine.
Après agitation à température ambiante pendant une nuit, le milieu réactionnel est dilué avec de l'acétate d'éthyle et lavé deux fois à l'eau. La phase organique est séchée sur sulfate de magnésium, filtrée et concentré sous vide. Le résidu huileux obtenu est chromatographié sur une cartouche contenant 5 g de silice, l'élution étant effectuée avec un mélange dichlorométhane/méthanol 98/2 puis 95/5. Après concentration des fractions d'élution, le résidu est agité avec de l'oxyde de düsopropyle. Le précipité est alors filtré et séché sous vide. On obtient ainsi 0,16 g de 1-(5-méthoxyisoindolin-2-yl)-6-[4-(3-trifluorométhylphényl)-pipérazin-1-yl]hexan-1-one sous forme de solide.
Point de fusion : 92°C
RMN ¹H (CDCl₃) : 7,3 (t, 1H, J = 7,5 Hz) ; 7,25 à 7,0 (ms, 4H) ; 6,9 à 6,8 (ms, 2H) ; 4,8 (s, 2H) ; 4,75 (s, 2H) ; 3,8 (s, 3H) ; 3,3 à 3,15 (ms, 4H) ; 2,8 à 2,55 (ms, 4H) ; 2,55 à 2,2 (ms, 2H) ; 2,4 (t, 2H, J = 7,5 Hz) ; 1,9 à 1,35 (ms, 6H)

La 5-méthoxyisoindoline peut être préparée à partir du 3,4-diméthylanisole selon la technique décrite dans EP 0343560

### Exemple 2 : préparation de la 1-(5-méthanesulfonyloxyisoindolin-2-yl)-6-[4-(3-trifluorométhylphényl)pipérazin-1-yl]hexan-1-one

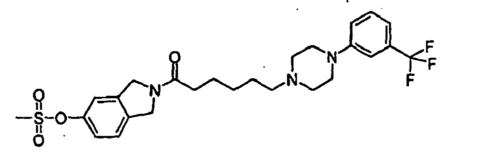

En opérant comme à l'exemple 1, étape c, mais à partir de 5-méthanesulfonyloxyisoindoline, on obtient la 1-(5-méthanesulfonyloxyisoindolin-2-yl)-6-[4-(3-trifluorométhylphényl)pipérazin-1-yl]-hexan-1-one sous forme de solide.
Point de fusion : 109°C
RMN ¹H (CDCl₃) : 7,3 à 7,20 (ms, 3H) ; 7,2 à 7,0 (ms, 4H) ; 4,8 (s large, 4H) ; 3,4 à 3,2 (ms, 4H) ; 3,15 (s, 3H) ; 2,75 à 2,55 (ms, 4H) ; 2,55 (t large, 2H, J = 7,5 Hz) ; 2,4 (t, 2H, J = 7,5Hz) ; 1,9 à 1,5 (ms, 4H) ; 1,5 à 1,3 (ms, 2H)

La 5-méthanesulfonyloxyisoindoline peut être préparée selon le procédé décrit dans Bioorg. Med. Chem. Lett. (2001), 11, 685-688.

### Exemple 3 : préparation du chlorhydrate de 1-(5-cyanoisoindolin-2-yl)-6-[4-(3-trifluorométhylphényl)pipérazin-1-yl]-hexan-1-one .

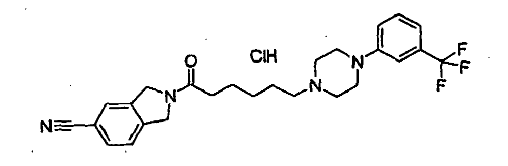

En opérant comme à l'exemple 1, étape c, mais à partir de 5-cyanoisoindoline on obtient le produit sous forme de base. Le résidu huileux est dissous dans de l'oxyde de diéthyle. Une addition d'une solution saturée de chlorure d'hydrogène dans l'oxyde de diéthyle est effectuée. Le précipité est filtré et séché sous vide. On obtient ainsi le chlorhydrate de 1-(5-cyanoisoindolin-2-yl)-6-[4-(3-trifluorométhylphényl)pipérazin-1-yl]-hexan-1-one sous forme d'un solide.
Point de fusion : 155-160°C
RMN ¹H (DMSO d₆) : 10,9 (t large, 1 H) ; 7,8 (s, 1H) ; 7,75 (d, 1H, J = 7,5 Hz) ; 7,6 à 7,3 (ms, 2H), 7,25 à 7,15 (ms, 2H) ; 7,1 (d, 1H, J = 7,5Hz) ; 5,2 (s, 4H) ; 4,85 (d, 2H, J = 9,5 Hz) ; 4,65 (d, 2H, J = 9,5 Hz) ; 3,9 (d, 2H, 10 Hz) ; 3,5 (d, 2H, J = 10 Hz) ; 3,35 à 2,95 (ms, 2H) ; 2,3 (t, 2H, J = 7,5 Hz) ; 1,9 à 1,7 (ms, 2H) ; 1,7 à 1,5 (ms, 2H) ; 1,45 à 2,2 (ms, 2H)

La 5-cyanoisoindoline peut être préparée selon le procédé décrit dans Bioorg. Med. Chem. Lett. (2001), 11, 685-688.

### Exemple 4 : préparation de la 1-isoindolin-2-yl-6-[4-(3-trifluorométhoxyphényl)-pipérazin-1-yl]hexan-1-one

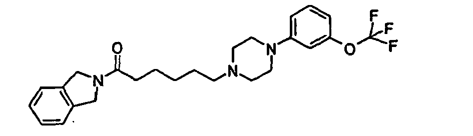

### Etape a : préparation du 6-[4-(3-trifluorométhoxyphényl)pipérazin-1-yl]hexanoate d'éthyle

En opérant comme à l'exemple 1, étape a, mais à partir de 2,46 g de 3-trifluorométhoxyphényl)pipérazine et 2,23 g de 6-bromohexanoate d'éthyle, on obtient 3,5 g de 6-[4-(3-trifluorométhoxyphényl)pipérazin-1-yl]hexanoate d'éthyle sous forme de solide utilisé tel quel dans les étapes ultérieures.

### Etape b : préparation de l'acide 6-[4-(3-trifluorométhoxyphényl)pipérazin-1-yl]hexanoïque

En opérant comme à l'exemple 1, étape b, mais à partir de 3,5 g de 6-[4-(3-trifluorométhoxyphényl)pipérazin-1-yl]hexanoate d'éthyle, on obtient 2,9 g d'acide 6-[4-(3-trifluorométhoxyphényl)pipérazin-1-yl]hexanoïque sous forme de solide blanc utilisé tel quel dans les étapes ultérieures.

### Etape c : préparation de la 1-isoindolin-2-yl-6-[4-(3-trifluorométhoxyphényl)-pipérazin-1-yl]hexan-1-one

En opérant comme à l'exemple 1, étape c, mais à partir de 0,142 g d'isoindoline et 0,36 g d'acide 6-[4-(3-trifluorométhoxyphényl)pipérazin-1-yl]hexanoïque, on obtient 0,125 g de 1-isoindolin-2-yl-6-[4-(3-trifluorométhoxyphényl)pipérazin-1-yl]hexan-1-one sous forme de solide blanc.
Point de fusion : 89°C
RMN ¹H (CDCl₃) : 7,4 à 7,15 (ms, 5H) ; 6,85 (d, 1H, J = 7 Hz) ; 6,8 (ms, 2H) ; 4,8 (s, 4H) ; 3,3 à 3,15 (ms, 4H) ; 2,7 à 2,5 (ms, 4H) ; 2,5 à 2,3 (ms, 4H) ; 1,9 à 1,7 (ms, 2H) ; 1,7 à 1,5 (ms, 2H) ; 1,5 à 1,35 (ms, 2H)

### Exemple 5: préparation de la 1-isoindolin-2-yl-6-[4-(6,7,8,9-tétrahydro-5H-benzocyloheptèn-2-yl)pipérazin-1-yl]hexan-1-one

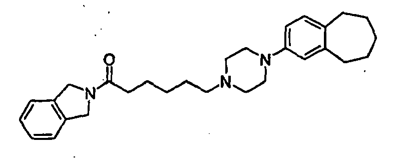

### Etape a : préparation de la 6,7,8,9-tétrahydro-5H benzocycloheptèn-2-ylamine

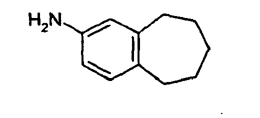

Une solution de 1,4 g (7,3 mmol) de 2-nitro-6,7,8,9-tétrahydro-5*H*-benzocyloheptène dans 15 mL de méthanol contenant 0,3 g de palladium sur charbon à 5% est hydrogénée pendant 16 heures à température ambiante.
Après filtration sur un lit de célite et concentration du solvant, on obtient 1,25 g de 6,7,8,9-tétrahydro-5*H*-benzocycloheptèn-2-ylamine.
Rf : 0,77 dichlorométhane/méthanol/ammoniaque 90/10/1
RMN ¹H (CDCl₃) : 6,9 (d, 1H, J = 10 Hz) ; 6,6 à 6,35 (ms, 2H) ; 3,7 à 3,25 (s large, 2H) ; 2,8 à 2,6 (ms, 4H) ; 1,9 à 1,75 (ms, 2H) ; 1,75 à 1,5 (ms, 4H)

Le 2-nitro-6,7,8,9-tétrahydro-5*H*-benzocyloheptène peut être obtenu selon le procédé décrit dans J. Am. Chem. Soc. (1969), 91, 3558-3566.

### Etape b : préparation de la 1-(6,7,8,9-tétrahydro-5H-benzocycloheptèn-2-yl)-pipérazine

Une solution de 1,25 g (7,7 mmol) d'aniline et de 1,4 g (7,7 mmol) de chlorhydrate de bis(2-chloroéthyl)amine dans 8 mL de chlorobenzène est portée à reflux pendant 2 jours.
La solution est diluée avec de l'oxyde de diéthyle et de l'eau. La phase organique est séparée. La phase aqueuse est lavée encore une fois avec le l'oxyde de diéthyle puis basifiée avec une solution aqueuse de soude 10N et extraite 2 fois avec de l'oxyde de diéthyle. Les phases éthérées sont réunies, lavées avec de l'eau, séchées sur sulfate de magnésium, filtrées et concentrées. On obtient ainsi 1,42 g de 1-(6,7,8,9-tétrahydro-5H-benzocycloheptèn-2-yl)-pipérazine.
Rf : 0,43 dichlorométhane/méthanol/ammoniaque 90/1011
RMN ¹H (CDCl₃) : 7,1 (d, 1H, J = 10 Hz) ; 6,7 (d, 1H, J = 2,5Hz) ; 6,65 (d, 1H, J = 10 Hz) ; 3,2 à 2,9 (ms, 8H) ; 2,85 (ms, 4H) ; 1;9 à 1,75 (ms, 2H) ; 1,75 à 1,5 (ms, 5H)

### Etape c : préparation du 6-[4-(6,7,8,9-tétrahydro-5H-benzocyloheptèn-2-yl)pipérazin-1-yl]hexanoate d'éthyle

En opérant comme à l'exemple 1, étape a, mais à partir de 0,8 g de 6-[4-(6,7,8,9-tétrahydro-5*H*-benzocyloheptèn-2-yl)pipérazin-1-yl]hexanoate d'éthyle et de 0,85 g de 6-bromohexanoate d'éthyle, on obtient 1,15 g de 6-[4-(6,7,8,9-tétrahydro-5*H*-benzocyloheptèn-2-yl)pipérazin-1-yl]hexanoate d'éthyle utilisé tel quel dans les synthèses ultérieures.

### Etape d : préparation de l'acide 6-[4-(6,7,8,9-tétrahydro-5H-benzocyloheptèn-2-yl)pipérazin-1-yl]hexanoïque

En opérant comme à l'exemple 1, étape b, mais à partir de 1,1 g de 6-[4-(6,7,8,9-tétrahydro-5*H*-benzocyloheptèn-2-yl)pipérazin-1-yl]hexanoate d'éthyle, on obtient 0,9 g d'acide 6-[4-(6,7,8,9-tétrahydro-5*H*-benzocyloheptèn-2-yl)pipérazin-1-yl]hexanoïque utilisé tel quel dans les synthèses ultérieures.

### Etape e : 1-isoindolin-2-yl-6-[4-(6,7,8,9-tétrahydro-5H-benzocyloheptèn-2-yl)pipérazin-1-yl]hexan-1- one

En opérant comme à l'exemple 1, étape c, mais à partir de 0,34 g d'acide 6-[4-(6,7,8,9-tétrahydro-5*H*-benzocyloheptèn-2-yl)pipérazin-1-yl]hexanoïque et 0,12 g d'isoindoline, on obtient 0,3 g de 1-isoindolin-2-yl-6-[4-(6,7,8,9-tétrahydro-5*H-*benzocyloheptèn-2-yl)pipérazin-1-yl]hexan-1-one sous forme de solide.
Point de fusion : 127°C
RMN ¹H (CDCl₃) 7,4 à 7,2 (ms, 4H) ; 7,0 (s, 1 H, J = 7,5 Hz) ; 6,7 (s, 1H) ; 6,7 (d, 1 H, J = 7,5 Hz) ; 4,8 (s, 4H) ; 3,25 à 3,1 (ms, 4H) ; 2,8 à 2,65 (ms, 4H) ; 2,65 à 2,5 (ms, 4H) ; 2,5 à 2,3 (ms, 4H) ; 1,9 à 1,7 (ms, 4H) ; 1,7 à 1,5 (ms, 6H) ; 1,5 à 1,35 (ms, 2H)

### Exemple 6 : préparation de la 1-(4-fluoroisoindolin-2-yl)-6-[4-(3-trifluorométhylphényl)pipérazin-1-yl]hexan-1-one

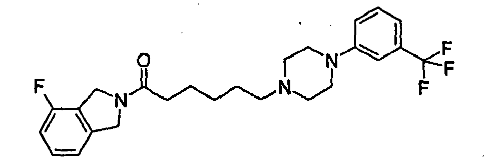

En opérant comme à l'exemple 1, étape c, mais à partir de 0,14 g de 4-fluoroisoindoline (préparation décrite dans EP0343 560 A2) et de 0,34 g d'acide 6-[4-(3-trifluorométhoxyphényl)pipérazin-1-yl]hexanoïque, on obtient 0,11 g de 1-(4-fluorosoindolin-2-yl)-6-[4-(3-trifluorométhylphényl)pipérazin-1-yl]-hexan-1-one sous forme de solide blanc.
RMN ¹H (CDCl₃) : 7,4 à 7,25 (ms, 2H) ; 7,2 à 6,9 (ms, 5H) ; 4,8 (s, 4H) ; 3,25 (m, 4H) ; 2,6 (m, 4H) ; 2,6 à 2,3 (ms, 4H) ; 1,8 (m, 2H) ; 1,7 à 1.2 (ms, 4H)

### Exemple 7 : préparation de la 1-(5-fluoroisoindolin-2-yl)-6-[4-(3-trifluorométhylphényl)pipérazin-1-yl]hexan-1-one

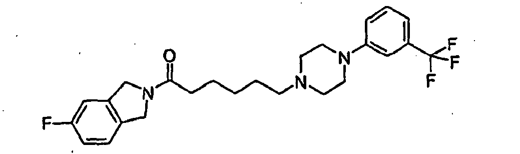

En opérant comme à l'exemple 1, étape c, mais à partir de 0,14 g de 5-fluoroisoindoline (préparation décrite dans EP0343 560 A2) et de 0,34 g d'acide 6-[4-(3-trifluorométhoxyphényl)pipérazin-1-yl]hexanoïque, on obtient 0,11 g de 1-(5-fluoroisoindolin-2-yl)-6-[4-(3-trifiuorométhylphényl)pipérazin-1-yl]hexan-1-one sous forme de solide beige.
RMN ¹H (CDCl₃) : 7,4 (m, 1H) ; 7,25 (m, 1H) ; 7,15 à 6,9 (ms, 5H) ; 4,8 (s, 4H) ; 3,2 (m, 4H) ; 2,65 (m, 4H) ; 2,5 (m, 2H) ; 2,4 (t, 2H, J = 7Hz) ; 1,8 (m, 2H) ; 1,75 à 1,35 (ms, 4H)

### Exemple 8 : préparation de la 1-(5-isopropoxyisoindolin-2-yl)-6-[4-(3-trifluorométhylphényl)pipérazin-1-yl]hexan-1-one

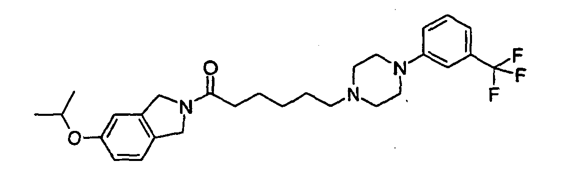

### Etape a : préparation de la 1-(5-hydroxyisoindolin-2-yl)-6-[4-(3-trifluorométhylphényl)pipérazin-1-yl]hexan-1-one

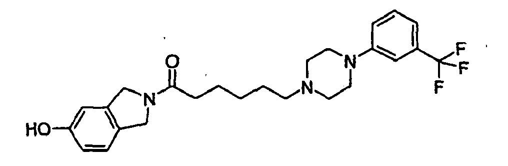

En opérant comme à l'exemple 1, étape c, mais à partir de 0,47 g de 5-hydroxyisoindoline (préparation décrite dans EP0343 560 A2) et de 0,76 g d'acide 6-[4-(3-trifluorométhoxyphényl)pipérazin-1-yl]hexanoïque, on obtient 0,17g de 1-(5-hydroxyisoindolin-2-yl)-6-[4-(3-trifluorométhylphényl)pipérazin-1-yl]hexan-1-one sous forme d'huile.

### Etape b : préparation de la 1-(5-isopropoxyisoindolin-2-yl)-6-[4-(3-trifluorométhylphényl)pipérazin-1-yl]hexan-1-one

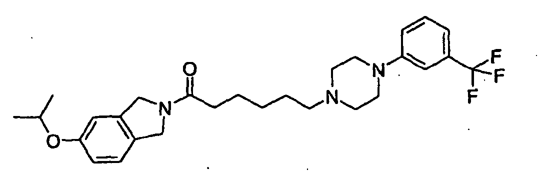

Un mélange de 0,17 g (0,37 mmol) de 1-(5-hydroxyisoindolin-2-yl)-6-[4-(3-trifluororriéthylphényl)pipérazin-1-yl]hexan-1-one, de 0,18 g (0,55 mmol) de carbonate de césium, de 75 mg de 2-iodopropane et de 5 mL d'acétonitrile est porté au reflux pendant 16 heures.
Après concentration du solvant, le résidu est repris avec de l'acétate d'éthyle et lavé avec de l'eau. La phase organique est séchée sur sulfate de magnésium, filtrée et concentrée. Le produit est purifié par chromatographie sur gel de silice (éluant dichlorométhane/méthanol 95/5). On obtient ainsi 38 mg de 1-(5-isopropoxyisoindolin-2-yl)-6-[4-(3-trifluorométhylphényl)pipérazin-1-yl]hexan-1-one sous forme de solide beige.
RMN ¹H (CDCl₃) : 7,4 (m, 1H) ; 7,2 à 7,0 (ms, 4H) ; 6,9 à 6,7 (ms, 2H) ; 4,75 (s, 2H) ; 4,7 (s, 2H) ; 4,55 (m, 1H) ; 3,2 (m, 4H) ; 2,6 (m, 4H) ; 2,55 à 2,2 (ms, 4H) ; 1,8 (m, 2H) ; 1,75 à 1,35 (ms, 4H) ; 1,35 (d, 6H, j = 7Hz)

### Exemple 9 : préparation de la 1-(4-chloroisoindolin-2-yl)-6-[4-(3-tritluorométhylphényl)pipérazin-1-yl]héxan-1-one

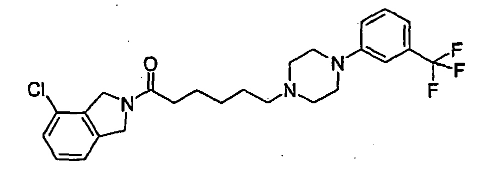

En opérant comme à l'exemple 1, étape c, mais à partir de 0,16 g de 4-chloroisoindoline (préparation décrite dans EP0343 560 A2) et de 0,34 g d'acide 6-[4-(3-trifluorométhoxyphényl)pipérazin-1-yl]hexanoïque, on obtient 0,30 g 1-(4-chloroisoindolin-2-yl)-6-[4-(3-trifluorométhylphényl)pipérazin-1-yl]hexan-1-one sous forme de solide blanc.
RMN ¹H (CDCl₃) : 7,4 (m, 1H) ; 7,3 à 7,0 (ms, 6H) ; 4,9 (s, 2H) ; 4,8 (s, 2H) ; 3,3 (m, 4H) ; 2,6 (m, 4H) ; 2,55 à 2,3 (ms, 4H) ; 1,8 (m, 2H) ; 1,7 à 1,35 (ms, 4H)

### Exemple 10 : préparation de la 1-(6,7-dihydro-5H-[1,3]dioxolo[4,5-f]isoindol-6-yl)-6-[4-(3-trifluorométhylphényl)pipérazin-1-yl]hexan-1-one

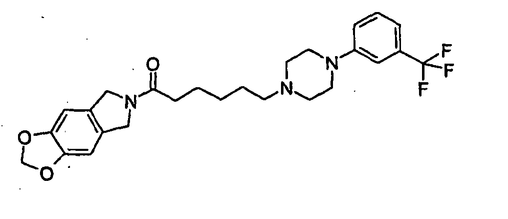

En opérant comme à l'exemple 1, étape c, mais à partir de 0,16 g de 6,7-dihydro-5*H*-[1,3]dioxolo[4,5-*f*]isoindoline et de 0,34 g d'acide 6-[4-(3-trifluorométhoxyphényl)pipérazin-1-yl]hexanoïque, on obtient 0,22 g de 1-(6,7-dihydro-5*H-*[1,3]dioxolo[4,5-*f*]isoindol-6-yl)-6-[4-(3-trifluorométhylphényl)pipérazin-1-yl]hexan-1-one sous forme de solide brun.
RMN ¹H (CDCl₃) : 7,35 (m, 1H) ; 7,2) 7,0 (ms, 3H) ; 6,75 (s, 1H) ; 6,7 (s, 1H) ; 6,0 (s, 2H) ; 4,7 (s, 4H) ; 3,25 (m, 4H) ; 2,65 (m, 4H) ; 2,5 à 2,3 (ms, 4H) ; 1,8 (m, 2H) ; 1,7 à 1,5 (ms, 2H) ; 1,5 à 1,3 (ms, 2H)

### Exemple 11 : préparation de la 1-(5-hydroxyméthylisoindolin-2-yl)-6-[4-(3-trifluorométhylphényl)pipérazin-1-yl]hexan-1-one

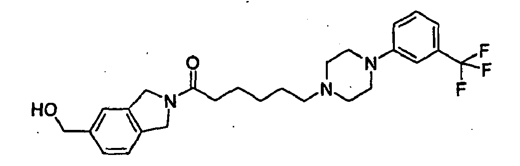

En opérant comme à l'exemple 1, étape c, mais à partir de 0,05 g de 5-hydroxyméthylisoindoline (préparation décrite dans EP0343 560 A2) et de 0,14 g d'acide 6-[4-(3-trifluorométhoxyphényl)pipérazin-1-yl]heXanoïque, on obtient 0,07 g de 1-(5-hydroxyméthylisoindolin-2-yl)-6-[4-(3-trifluorométhylphényl)-pipérazin-1-yl]hexan-1-one sous forme de solide blanc.
RMN ¹H (CDCl₃) : 7,4 à 7,2 (ms, 4H) ; 7,15 à 7,0 (ms, 3H) ;4,S (s, 4H) ; 4,7 (d, 2H, J = 5Hz) ; 3,2 (m, 4H) ; 2,7 (m, 4H) ; 2,55 à 2,3 (ms, 4H) ; 1,8 (m, 2H) ; 1,7 à 1,5 (ms, 3H) ; 1,45 (m, 2H)

### Exemple 12 : préparation du chlorhydrate de la 1-(5-méthoxycarbonylisoindolin-2-yl)-6-[4-(3-trifluorométhylphényl)pipérazin-1-yl]hexan-1-one

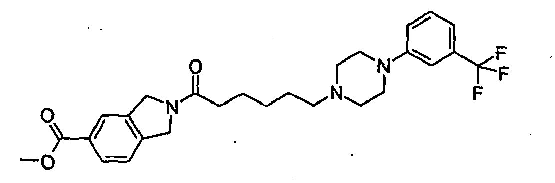

En opérant comme à l'exemple 1, étape c, mais à partir de 0,60 g de 5-méthoxycarbonylisoindoline (préparation décrite dans EP0343 560 A2) et de 0,90g d'acide 6-[4-(3-trifluorométhoxyphényl)pipérazin-1-yl]hexanoïque, on obtient 0,74 g de chlorhydrate de 1-(5-méthoxycarbonylisoindolin-2-yl)-6-[4-(3-trifluorométhylphényl)pipérazin-1-yl]hexan-1-one sous forme de solide brun.
RMN ¹H (CDCl₃): 8,0 (m, 2H) ; 7,45 à 7,25 (ms, 2H) ; 7,15 à 7,0 (ms, 3H); 4,8 (s, 4H) ; 3,9 (s, 3H) ; 3,2 (m, 4H) ; 2,65 (m, 4H) ; 2,55 à 2,3 (ms, 4H) ; 1,8 (m, 2H) ; 1,6 (m, 2H) ; 1,45 (m, 2H)

### Exemple 13 : préparation de la 1-(5-carboxyisoindolin-2-yl)-6-[4-(3-trifluorométhylphényl)pipérazin-1-yl]hexan-1-one

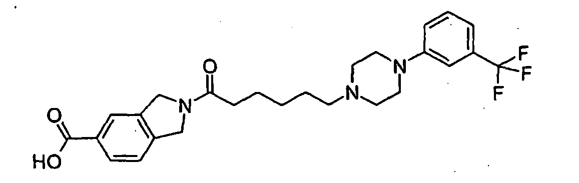

Un mélange de 0,61 g de chlorhydrate de 1-(5-méthoxycarbonylisoindolin-2-yl)-6-[4-(3-trifluorométhylphényl)pipérazin-1-yl]hexan-1-one, de 10 mL de méthanol et de 1,5 mL d'une solution aqueuse de soude normale est porté au reflux pendant sept heures.
Après concentration du méthanol, le résidu est repris avec de l'eau puis acidifié avec une solution aqueuse d'acide chlorhydrique normale. Le précipité est filtré, lavé avec de l'oxyde de diisopropyle et séché sous vide sur pentaoxyde de phosphore. On obtient ainsi 0,48 g (81%) de 1-(5-carboxyisoindolin-2-yl)-6-[4-(3-trifluorométhylphényl)pipérazin-1-yl]hexan-1-one.
RMN ¹H (DMSO D₆) : 7,9 (m, 2H) ; 7,5 à 7,3 (ms, 2H) ; 7,2 (ms, 1H) ; 7,1 (s, 1H) ; 7,0 (m, 1 H) ; 4,85 (s, 2H) ; 4,65 (s, 2H) ; 3,2 (m, 4H) ; 2,55 (m, 4H) ; 2,45 à 2,2 (ms, 4H) ; 1,7 à 1,4 (ms, 4H) ; 1,3 (m, 2H)

### Exemple 14 : préparation de la 1-[5-(2-hydroxyéthylaminocarbonyl)isoindolin-2-yl]-6-[4-(3-trifluorométhylphényl)pipérazin-1-yl]hexan-1-one

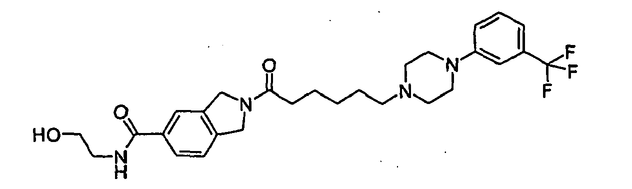

En opérant comme à l'exemple 1, étape c, mais à partir de 0,11 g de 1-(5-carboxyisoindolin-2-yl)-6-[4-(3-trifluorométhylphényl)pipérazin-1-yl]hexan-1-one et de 14 mg d'éthanolamine, on obtient 6 mg de 1-[5-(2-hydroxyéthylaminocarbonyl)isoindolin-2-yl]-6-[4-(3-trifluorométhylphényl)pipérazin-1-yl]hexan-1-one sous forme de solide blanc.
RMN ¹H (CDCl₃) : 7,8 à 7,6 (ms, 2H) ; 7,4 (m, 2H) ; 7,15 à 7,0 (ms, 3H) ; 6,6 (d large, 1H) ; 4,8 (s, 4H) ; 3,85 (m, 2H) ; 3,75 (m, 2H) ; 3,2 (m, 4H) ; 2,6 (m, 4H) ; 2,5 à 2,3 (ms, 4H) ; 1,8 (m, 2H) ; 1,7 à 1,35 (ms, 4H)

### Exemple 15 : préparation de la 1-(5-morpholinocarbonylisoindolin-2-yl)-6-[4-(3-trifluorométhylphényl)pipérazin-1-yl]hexan-1-one

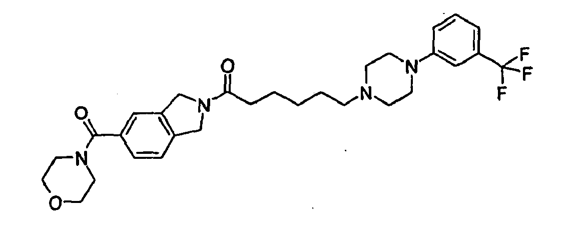

En opérant comme à l'exemple 1, étape c, mais à partir de 0,10 g de 1-(5-carboxyisoindotin-2-yl)-6-[4-(3-trifluorométhylphényl)pipérazin-1-yl]hexan-1-one et de 20 mg de morpholine, on obtient 51 mg de 1-(5-morpholinocarbonylisoindolin-2-yl)-6-[4-(3-trifluorométhylphényl)pipérazin-1-yl]hexan-1-one sous forme de solide jaune.
RMN ¹H (CDCl₃) : 7,45 à 7,3 (ms, 4H) ; 7,2 à 7,0 (ms, 3H) ; 4,8 (s, 4H) ; 3,9 à 3,5 (ms, 8H) ; 3,3 (m, 4H) ; 2,65 (m, 4H) ; 2,55 à 2,3 (ms, 4H) ; 1,8 (m, 2H) ; 1,7 à 1,4 (ms, 4H)

### Exemple 16 : préparation de la 1-(5-(pyrrolidin-1-ylcarbonylisoindolin-2-yl)-6-[4-(3-trifluorométhylphényl)pipérazin-1-yl]hexan-1-one

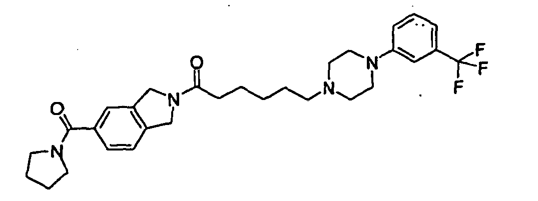

En opérant comme à l'exemple 1, étape c, mais à partir de 0,10 g de 1-(5-carboxyisoindolin-2-yl)-6-[4-(3-trifluorométhylphényl)pipérazin-1-yl]hexan-1-one et de 14 mg de pyrrolidine, on obtient 38 mg de 1-(5-(pyrrolidin-1-ylcarbonylisoindolin-2-yl)-6-[4-(3-trifluorométhylphényl)pipérazin-1-yl]hexan-1-one sous forme de solide jaune.
Point de fusion : 109°C
RMN ¹H (CDCl₃) : 7,5 à 7,4 (ms, 2H) ; 7,4 à 7,2 (ms, 2H) ; 7,2 à 7,0 (ms, 3H) ; 4,8 (s, 4H) ; 3,65 (m, 2H) ; 3,45 (m, 2H) ; 3,35 (m, 4H) ; 2,7 (m, 4H) ; 2,6 (m , 2H) ; 2,4 (t, 2H, J = 7Hz) ; 2,1 à 1,4 (ms, 10H)

### Exemple 17 : préparation de la 1-(5-pipéridin-1-ylcarbonylisoindolin-2-yl)-6-[4-(3-trifluorométhylphényl)pipérazin-1-yl]hexan-1-one

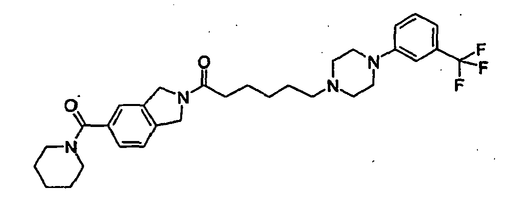

En opérant comme à l'exemple 1, étape c, mais à partir de 0,10 g de 1-(5-carboxyisoindolin-2-yl)-6-[4-(3-trifluorométhylphényl)pipérazin-1-yl]hexan-1-one et de 17 mg de pipéridine, on obtient 42 mg de 1-(5-pipéridin-1-ytcarbonytisoindolin-2-yl)-6-[4-(3-trifluorométhylphényl)pipérazin-1-yl]hexan-1-one sous forme de solide jaune.
Point de fusion : 91°C
RMN ¹H (CDCl₃) : 7,5 à 7,25 (ms, 4H) ; 7,2 à 7,0 (ms, 3H) ; 4,8 (s, 4H) ; 3,7 (m, 2H) ; 3,5 à 3,2 (ms, 6H) ; 2,9 à 2,5 (ms, 6H) ; 2,4 (t, 2H, J = 7Hz) ; 1,9 à 1,4 (ms, 12H)

### Exemple 18 : préparation de la 1-[5-(2-diméthylamino-éthylamino-carbonyl)-isoindolin-2-yl]-6-[4-(3-trifluorométhylphényl)pipérazin-1-yl]hexan-1-one

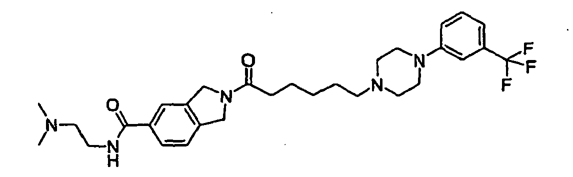

En opérant comme à l'exemple 1, étape c, mais à partir de 0,10 g de 1-(5-carboxyisoindolin-2-yl)-6-[4-(3-trifluorométhylphényl)pipérazin-1-yl]hexan-1-one et de 12 mg de 2-diméthylaminoéthylamine, on obtient 49 mg de 1-[5-(2-diméthylaminoéthylaminocarbonyl)isoindolin-2-yl]-6-[4-(3-trifluorométhylphényl)-pipérazin-1-yl]hexan-1-one sous forme de solide blanc.
Point de fusion : 120°C
RMN ¹H (CDCl₃) : 7,9 à 7,75 (ms, 2H) ; 7,35 (m, 2H) ; 7,15 à 7,0 (ms, 3H) ; 4,85 (s, 4H) ; 3,65 (m, 2H) ; 3,3 (m, 4H) ; 2,8 (m, 2H) ; 2,65 (m, 4H) ; 2,55 à 2,3 (ms, 10H) ; 1,8 (m, 2H) ; 1,6 (m, 2H) ; 1,45 (m, 2H)

### Exemple 19 : préparation de la 1-[5-(4,5-dihydrooxazol-2yl)isoindolin-2-yl]-6-[4-(3-trifluorométhylphényl)pipérazin-1-yl]hexan-1-one

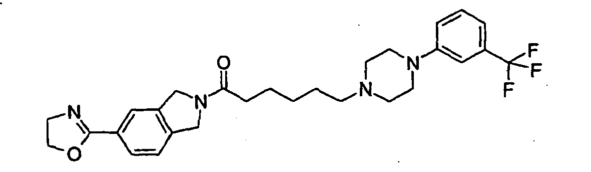

### Etape a : préparation de la 1-[5-(2-chloroéthylaminocarbonyl)isoindoün-2-yl]-6-[4-(3-trifluorométhylphényl)pipérazin-1-yl]hexan-1-one

En opérant comme à l'exemple 1, étape c, mais à partir de 0,20 g de 1-(5-carboxyisoindolin-2-yl)-6-[4-(3-trifluorométhylphényl)pipérazin-1-yl]hexan-1-one et de 52 mg de chlorhydrate de 2-chloroéthylamine, on obtient 0,19 g de 1-[5-(2-chloroéthylaminocarbonyl)isoindolin-2-yl]-6-[4-(3-trifluorométhylphényl)pipérazin-1-yl]hexan-1-one sous forme de solide.
RMN ¹H (CDCl₃) : 7,9 (m, 1H) ; 7,7 (m, 1 H) ; 7,5 à 7,2 (ms, 2H) ; 7,2 à 7,0 (ms, 3H) ; 6,6 (m, 1H) ; 4,8 (s, 4H) ; 4,45 (t, 2H, J = 7Hz) ; 4,1 (m, 2H) ; 3,4 (m, 4H) ; 3,0 (m, 4H) ; 2,8 (m, 2H) ; 2,4 (m, 2H) ; 1,9 à 1,75 (ms, 4H) ; 1,45 (m, 2H)

### Etape b : préparation de la 1-[5-(4,5-dihydrooxazol-2yl)isoindolin-2-yl]-6-[4-(3-trifluorométhylphényl)pipérazin-1-yl]hexan-1-one

Une solution de 0,18 g de 1-[5-(2-chloroéthylaminocarbonyl)isoindolin-2-yl]-6-[4-(3-trifluorométhylphényl)pipérazin-1-yl]hexan-1-one et de 0,11 g de 1,8-diazabicyclo[5.4.0]undec-7-ene dans 5 mL de dichlorométhane est chauffée au reflux pendant une nuit.
Après concentration du solvant, le résidu est dilué avec de l'eau. Le solide est filtré puis est purifié par chromatographie sur silice (éluant dichlorométhane/méthanol 95/5). On obtient ainsi 45 mg de 1-[5-(4,5-dihydro-oxazol-2yl)isoindolin-2-yl]-6-[4-(3-trifluorométhylphényl)pipérazin-1-yl]hexan-1-one sous forme de solide blanc.
Point de fusion : 129°C
RMN ¹H (CDCl₃) : 7,9 (m, 2H) ; 7,45 à 7,2 (ms, 3H) ; 7,2 à 7,0 (ms, 4H) ; 4,8 (s, 4H) ; 4,5 (t, 2H, J = 7Hz) ; 4,1 (t, 2H, J = 7Hz) ; 3,45 (m, 4H) ; 3,0 à 2,5 (ms, 6H) ; 2,4 (t, 2H, J = 7Hz) ; 1,8 (m, 2H) ; 1,7 à 1,4 (ms, 4H)

### Exemple 20 : préparation du chlorhydrate de la 1-(5-morpholinoylméthylisoindolin-2-yl)-6-[4-(3-trifluorométhylphényl)pipérazin-1-yl]hexan-1-one

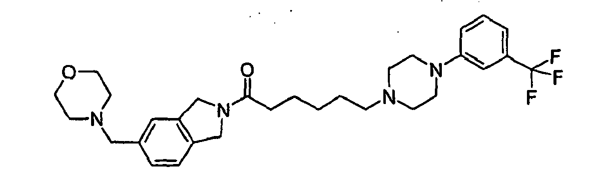

### Etape a : préparation de la 1-(5-méthanesulfonyloxyméthylisoindolin-2-yl)-6-[4-(3-trifluorométhylphényl)pipérazin-1-yl]hexan-1-one

Une solution de 0,17 g (0,36 mmol) de 1-(5-hydroxyméthylisoindolin-2-yl)-6-[4-(3-trifluorométhylphényl)pipérazin-1-yl]hexan-1-one et de 40 mg (0,40 mmol) de triéthylamine dans 2,5 mL de dichlorométhane est refroidie à -15°C. Une addition de 45 mg (0,40 mmol) de chlorure de méthanesulfonyle est alors effectuée.
Le mélange est agité pendant quatre heures à température ambiante puis est de nouveau refroidi à -15°C. Un ajout de 23 mg de chlorure de méthanesulfonyle est effectué. L'agitation est maintenue pendant une heure à température ambiante.
Le milieu est dilué avec du dichlorométhane et lavé avec de l'eau. La phase organique est séchée sur sulfate de magnésium, filtrée et concentrée. On obtient ainsi 0,18 g de 1-(5-méthanesulfonyloxyméthylisoindolin-2-yl)-6-[4-(3-trifluorométhylphényl)pipérazin-1-yl]hexan-1-one qui est utilisée sous forme brute dans l'étape suivante.

### Etape b: préparation de la 1-(5-morpholinométhylisoindolin-2-yl)-6-[4-(3-trifluorométhylphényl)pipérazin-1-yl]hexan-1-one

Un mélange de 0,18 g (0,32 mmol) de 1-(5-méthanesulfonyloxyméthylisoindolin-2-yl)-6-[4-(3-trifluorométhylphényl)pipérazin-1-yl]hexan-1-one, de 28 mg (0,32 mmol) de morpholine, de 49 mg (0,35 mmol) de carbonate de potassium et de 5 mL d'acétonitrile est porté au reflux pendant une nuit.
Après concentration du solvant, le résidu est repris avec de l'acétate d'éthyle puis est lavé avec de l'eau. La phase organique est séchée sur sulfate de magnésium, filtrée et concentrée.
Le produit est purifié par chromatographie sur gel de silice (éluant dichlorométhane/méthanol 95/5) puis salifié par une solution saturée d'acide chlorhydrique dans l'oxyde de diéthyle. On obtient ainsi 47 mg de chlorhydrate de 1-(5-morpholinométhylisoindolin-2-yl)-6-[4-(3-trifluorométhylphényl)pipérazin-1-yl]hexan-1-one sous forme de solide beige hygroscopique.
RMN ¹H (DMSO D₆) : 9,9 (s, large, 1H); 7,4 (m, 1 H) ; 7,4 à 7,0 (ms, 6H) ; 4,8 (s, 2H) ; 4,6 (s, 2H) ; 4,0 (m, 2H) ; 3,7 à 3,3 (ms, 8H) ; 3,25 à 3,0 (ms, 8H); 2,45 à 2,2 (ms, 4H) ; 1,8 à 1,45 (ms, 4H) ; 1,35 (m, 2H)

### Exemple 21 : préparation de la 1-[5-(pyrrolidin-1-ylméthyl)isoindolin-2-yl]-6-[4-(3-trifluorométhylphényl)pipérazin-1-yl]hexan-1-one

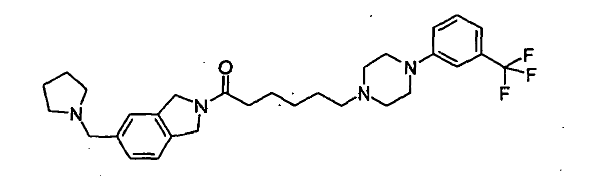

En opérant comme à l'exemple 20, étape b, mais à partir de 0,18 g de 1-(5-méthanesulfonyloxyméthylisoindolin-2-yl)-6-[4-(3-trifluorométhylphényl)pipérazin-1-yl]hexan-1-one et de 27 mg de pyrrolidine, on obtient 37 mg de 1-[5-(pyrrolidin-1-ylméthyl)isoindolin-2-yl]-6-[4-(3-trifïuorométhylphényi)pipérazin-1-yl]hexan-1-one sous forme de solide jaune.
RMN ¹H (CDCl₃) : 7,4 à 7,15 (ms, 4H) ; 7,15 à 7,0 (ms, 3H) ; 4,8 (s, 4H) ; 3,75 (m, 2H) ; 3.25 (m, 4H) ; 2,8 à 2,5 (ms, 8H) ; 2,5 à 2,3 (ms, 4H) ; 1,9 (m, 4H) ; 1,8 (m, 2H) ; 1,6 (m, 2H) ; 1,45 (m, 2H)

### Exemple 22 : préparation de la 1-[5-(diméthylaminométhyl)isoindolin-2-yl]-6-[4-(3-trifluorométhylphényl)pipérazin-1-yl]hexan-1-one

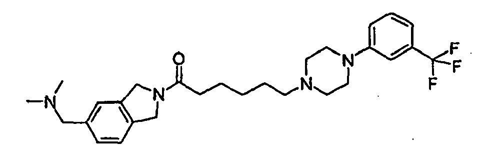

En opérant comme à l'exemple 20, étape b, mais à partir de 0,29 g de 1-(5-méthanesulfonyloxyméthylisoindolin-2-yl)-6-[4-(3-trifluorométhylphényl)pipérazin-1-yl]hexan-1-one et de 0,8 mL d'une solution de diméthylamine dans le tétrahydrofurane 2M, on obtient 55 mg de 1-[5-(diméthylaminométhyl)isoindolin-2-yl]-6-[4-(3-trifluorométhylphényl)pipérazin-1-yl]hexan-1-one sous forme de solide beige.
Point de fusion : 77°C
RMN ¹H (CDCl₃) : 7,45 à 7,2 (ms, 4H) ; 7,2 à 7,0 (ms, 3H) ; 4,8 (s, 4H) ; 3,55 (m, 2H) ; 3,25 (m, 4H) ; 2,65 (m, 4H) ; 2,55 à 2,2 (ms, 10H) ; 1,8 (m, 2H) ; 1,6 (m, 2H) ; 1,45 (m, 2H)

### Exemple 23 : préparation de la 1-[5-(2-pipéridinoéthoxy)isoindolin-2-yl]-6-[4-(3-trifluorométhylphényl)pipérazin-1-yl]hexan-1-one

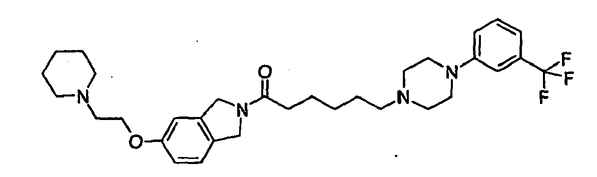

Un mélange de 0,1 g (0,22 mmol) de 1-(5-hydroxyisoindolin-2-yl)-6-[4-(3-trifluorométhylphényl)pipérazin-1-yl]hexan-1-one (exemple 8, étape a), de 40 mg (0,22 mmol) de chlorhydrate de 1-(2-chloroéthyl)pipéridine, de 64 mg (0,46 mmol) de carbonate de potassium et de 8 mL d'acétonitrile est porté au reflux pendant une nuit.
Après concentration du solvant, le résidu est repris avec de l'acétate d'éthyle puis est lavé avec de l'eau. La phase organique est séchée sur sulfate de magnésium, filtrée et concentrée. Le résidu est cristallisé dans du pentane, filtré et séché sous vide. On obtient ainsi 64 mg de 1-[5-(2-pipéridinoéthoxy)isoindolin-2-yl)-6-[4-(3-trifluorométhylphényl)pipérazin-1-yl]hexan-1-one sous forme de solide blanc.
Point de fusion : 88°C
RMN ¹H (CDCl₃) : 7,4 (m, 1H) ; 7,2 à 7,0 (ms, 4H) ; 6,8 (m, 2H) ; 4,75 (s, 4H) ; 4,25 (m, 2H) ; 3,25 (m, 4H) ; 3,0 (m, 2H) ; 2,8 à 2,5 (ms, 6H) ; 2,5 à 2,3 (ms, 4H) ; 1,8 (m, 2H) ; 1,7 à 1,3 (ms, 12H)

### Exemple 24 : préparation de la 1-[5-(3-pipéridinopropoxy)isoindolin-2-yl]-6-[4-(3-trifluorométhylphényl)pipérazin-1-yl]hexan-1-one

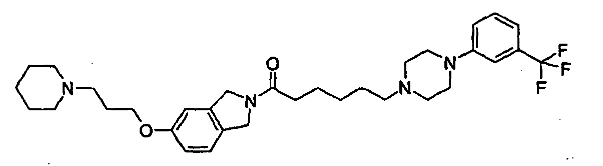

En opérant comme à l'exemple 23, mais à partir de 0,10 g de 1-(5-hydroxyisoindolin-2-yl)-6-[4-(3-trifluorométhylphényl)pipérazin-1-yl]hexan-1-one (exemple 8, étape a) et de 44 mg de chlorhydrate de 1-(3-chloropropylpipéridine, on obtient 75 mg de 1-[5-(3-pipéridinopropoxy)isoindolin-2-yl]-6-[4-(3-trifluorométhylphényl)pipérazin-1-yl]hexan-1-one sous forme de solide beige.
Point de fusion : 92°C
RMN ¹H (CDCl₃) : 7,35 (m, 1H) ; 7,25 à 7,0 (ms, 4H) ; 6,9 à 6,75 (ms, 2H) ; 4,75 (s, 2H) ; 4,7 (s, 2H) ; 4,0 (t, 2H, J = 7Hz) ; 3,25 (m, 4H) ; 2,75 à 2,45 (ms, 8H) ; 2,45 à 2,3 (ms, 6H) ; 2,1 (m, 2H) ; 1,9 à 1,35 (ms, 12H)

### Exemple 25 : préparation de la 1-[5-(3-pyrrolidinopropoxy)isoindolin-2-yl]-6-[4-(3-trifluorométhylphényl)pipérazin-1-yl]hexan-1-one

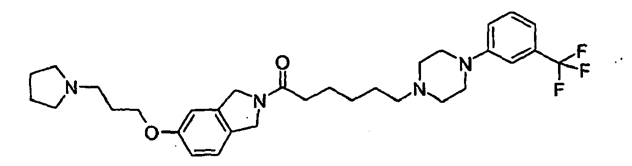

En opérant comme à l'exemple 23, mais à partir de 0,10 g de 1-(5-hydroxyisoindolin-2-yl)-6-[4-(3-trifluorométhylphényl)pipérazin-1-yl]hexan-1-one (exemple , étape a) et de 40 mg de chlorhydrate de 1-chloropropylpyrrolidine, on obtient 30 mg de 1-[5-(3-pyrrolidinopropoxy)isoindolin-2-yl]-6-[4-(3-trifluorométhylphényl)pipérazin-1-yl]hexan-1-one sous forme de solide beige.
Point de fusion : 92°C
RMN ¹H (CDCl₃) : 7,35 (m, 1 H) ; 7,25 à 7,0 (ms, 4H) ; 6,9 à 6,75 (ms, 2H) ; 4,75 (s, 4H) ; 4,05 (t, 2H, J = 7Hz) ; 3,25 (m, 4H) ; 2,95 à 2,65 (ms, 6H) ; 2,6 (m, 4H) ; 2,5 à 2,3 (ms, 4H) ; 2,15 (m, 2H) ; 1,9 (m, 2H) ; 1,8 (m, 2H) ; 1,6 (m, 2H) ; 1,45 (m, 2H)

### Exemple 26 : préparation de la 1-(5-acétylisoindolin-2-yl)-6-[4-(3-trifluorométhylphényl)pipérazin-1-yl]hexan-1-one

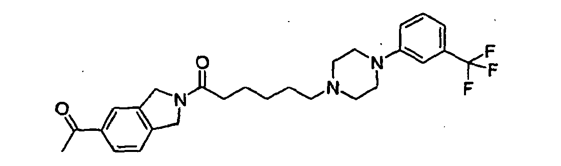

### Etape a : préparation de la 5-acétylisoindoline

Une solution de 0,3 g (1,7 mmol) de 5-méthoxycarbonylisoindoline dans 4 mL de tétrahydrofurane est chauffée à 50°C. Une solution de 3,3 mL (8,5 mmol) de chlorure de méthylmagnésium dans le tétrahydrofurane est alors ajoutée goutte à goutte.
Le milieu réactionnel est porté au reflux pendant trois heures puis est refroidi à 0°C. Une addition de 40 mL d'eau est effectuée. La suspension est filtrée sur célite. Le filtrat est extrait deux fois avec du dichlorométhane. Les phases d'extraction sont réunies, séchées sur sulfate de magnésium, filtrées et concentrées. On obtient ainsi 0,24 g de 5-acétylisoindoline.

### Etape b : préparation de la 1-(5-acétylisoindolin-2-yl)-6-[4-(3-trifluorométhylphényl)pipérazin-1-yl]hexan-1-one

En opérant comme à l'exemple 1, étape c, mais à partir de 0,23 g de 5-acétylisoindoline et de 0,50 g d'acide 6-[4-(3-trifluorométhoxyphényl)pipérazin-1-yl]hexanoïque, on obtient 45 mg de 1-(5-acétylisoindolin-2-yl)-6-[4-(3-trifluorométhylphényl)pipérazin-1-ylJhexan-1-one sous forme de solide brun.
RMN ¹H (CDCl₃) : 8,0 à 7,9 (ms, 2H) ; 7,45 à 7,25 (ms, 2H) ; 7,15 à 7,0 (ms, 3H) ; 4,85 (s, 4H) ; 3,3 (m, 4H) ; 2,7 (m, 4H) ; 2,65 (s, 3H) ; 2,55 à 2,35 (ms, 4H) ; 1,8 (m, 2H) ; 1,65 (m, 2H) ; 1,45 (m, 2H)

### Exemple 27 : préparation de la 1-[5-(1-hydroxy-1-méthyléthyl)isoindolin-2-yl]-6-[4-(3-trifluorométhylphényl)pipérazin-1-yl]hexan-1-one

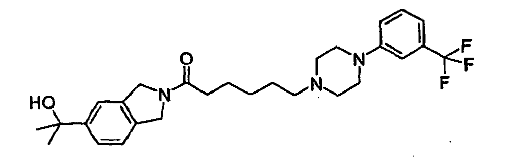

A une solution de 0,25 g (0,5 mmol) de 1-(5-méthoxycarbonylisoindolin-2-yl)-6-[4-(3-trifluorométhylphényl)pipérazin-1-yl]hexan-1-one dans 2 mL de tétrahydrofurane refroidie à -2°C est ajouté 0,67 mL (1,75 mmol) d'une solution de chlorure de méthylmagnésium dans le tétrahydrofurane. Le mélange est agité pendant deux heures à température ambiante puis est refroidi à -2°C. Un ajout de 0,38 mL (1 mmol) d'une solution de chlorure de méthylmagnésium dans le tétrahydrofurane est effectué. Le mélange est agité pendant une heure à température ambiante puis est refroidi à 0°C.
De l'eau est ajoutée puis le milieu est dilué avec de l'acétate d'éthyle. La suspension est filtrée sur célite. La phase organique est décantée, séchée sur sulfate de magnésium, filtrée et concentrée. Le produit est purifié par chromatographie sur gel de silice (éluant dichlorométhane/méthanol 95/5). On obtient ainsi 38 mg (15%) de 1-[5-(1-hydroxy-1-méthyléthyl)isoindolin-2-yl]-6-[4-(3-trifluorométhylphényl)pipérazin-1-yl]hexan-1-one sous forme d'huile.
RMN ¹H (CDCl₃) : 7,5 à 7,2 (ms, 4H) ; 7,2 à 7,0 (ms, 3H) ; 4,8 (s, 4H) ; 3,2 (m, 4H) ; 2,65 (m, 4H) ; 2,55 à 2,3 (ms, 4H) ; 1,8 (m, 2H) ; 1,75 à 1,55 (ms, 9H) ; 1,45 (m, 2H)

### Exemple 28 : préparation du chlorhydrate de la 1-[5-(1-hydroxyéthyl)isoindolin-2-yl]-6-[4-(3-trifluorométhylphényl)pipérazin-1-yl]hexan-1-one

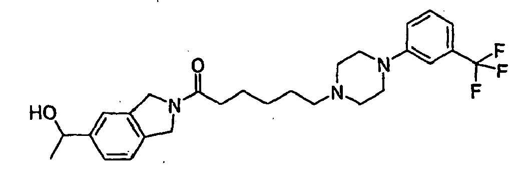

A une solution de 99 mg (0,2 mmol) de 1-(5-acétylisoindolin-2-yl)-6-[4-(3-trifluorométhylphényl)pipérazin-1-yl]hexan-1-one dans 4 mL de méthanol, sont ajoutés, à 5°C, 8 mg (0,2 mmol) de borohydrure de sodium. La solution est agitée pendant deux heures à température ambiante.

Après concentration du méthanol, le résidu est extrait avec de l'acétate d'éthyle et lavé avec de l'eau. La phase organique est séchée sur sulfate de magnésium, filtrée et concentrée.
Le résidu huileux est dissout dans de l'oxyde de diéthyle et acidifié avec une solution saturée de chlorure d'hydrogène dans l'oxyde de diéthyle. Le sel est filtré, lavé avec de l'oxyde de diéthyle et séché sous vide. On obtient ainsi 33 mg de chlorhydrate de la 1-[5-(l-hydroxyéthyl)isoindolin-2-yl]-6-[4-(3-trifluorométhylphényl)pipérazin-1-yl]hexan-1-one.
RMN ¹H (DMSO D₆) : 10,2 (s large, 1H) ; 7,45 (m, 1H) 7,35 à 7,15 (ms, 5H) ; 7,1. (m, 1H) ; 4,8 (s, 2H) ; 4,7 (m, 1H) ; 4,6 (s, 2H) ; 4,0 (m, 2H) ; 3,6 à 3,3 (ms, 4H); 3,2 à 3,0 (ms, 4H) ; 2,3 (m, 2H) ; 1,75 (m, 2H) ; 1,6 (m, 2H); 1,4 (m, 2H) ; 1,3 (d, 3H, J = 7Hz)

### Exemple 29 : préparation de la 1-(5-aminocarbonylisoindolin-2-yt)-6-[4-(3-trifluorométhylphényl)pipérazin-1-yl]hexan-1-one

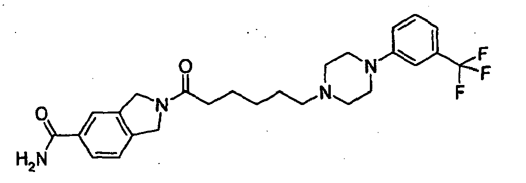

Un mélange de 0,14 g (0,28 mmol) de 1-(5-carboxyisoindolin-2-yl)-6-[4-(3-trifluorométhylphényl)pipérazin-1-yl]hexan-1-one (exemple 13), de 38 mg (0,28 mmol) d'hydroxybenzotriazole, de 54 mg (0,28 mmol) de chlorhydrate de 1-(3-diméthylaminopropyl)-3-éthylcarbodiimide, de 30 mg (0,56 mmol) de chlorure d'ammonium et de 5 mL de tétrahydrofurane est refroidi à 0°C. Une solution de 85 mg (0,84 mmol) de triéthylamine dans 0,5 mL de dichlorométhane est ajoutée. Le mélange est agité à température ambiante pendant une nuit. Le solvant est concentré sous vide et le résidu est dilué avec de l'eau. Le solide est filtré et séché sous vide. On obtient ainsi 78 mg (57%) de 1-(5-aminocarbonylisoindolin-2-yl)-6-[4-(3-trifluorométhyfphényl)pipérazin-1-yl]hexan-1-one sous forme de solide beige.
RMN ¹H (CDCl₃) : 7,85 à 7,65 (ms, 2H) ; 7,4 (m, 2H) ; 7,2 à 7,0 (ms, 3H) ; 6,1 (s large, 1H) ; 5,7 (s large, 1H) ; 4,85 (s, 4H) ; 3,35 (m, 4H) ; 2,75 (m, 4H) ; 2,55 (m, 2H) ; 2,4 (t, 2H, J = 7Hz) ; 1,9 à 1,6 (ms, 4H) ; 1,5 (m, 2H)

### Exemple 30 : préparation du chlorhydrate de la 1-isoindolin-2-yl-6-[4-(3-trifluorométhylphényl)pipérazin-1-yl]hexan-1-one

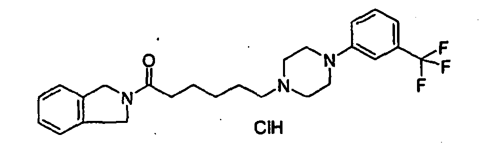

### Etape a : préparation de la 2-(6-bromohexanoyl)isoindoline.

Une solution contenant 1,2 g (10 mmol) d'isoindoline, 20 mL de dichlorométhane et 1,5 mL (11 mmol) de triéthylamine est refroidie à 5°C. Une addition de 1,53 mL (10 mmol) de chlorure de 6-bromohexanoyle est alors effectuée à 5°C. Le bain de refroidissement est enlevé et l'agitation est poursuivie pendant 6 heures à température ambiante.
Le milieu réactionnel est dilué avec 30 mL de dichlorométhane et est lavé avec de l'eau (2 fois 50 mL). La phase organique est séchée sur sulfate de magnésium, filtrée et concentrée sous vide. Le résidu cristallisé obtenu est agité avec 10 mL d'oxyde de diisopropyle. Après filtration et séchage sous vide, on obtient 2,65 g de 2-(6-bromohexanoyl)isoindoline sous forme de cristaux blancs.
Point de fusion : 88°C.
RMN ¹H (CDCl₃) : 7,45 à 7,2 (ms, 4H) ; 4,8 (s, 4 H) ; 3,45 (t, 2H, J = 7,5 Hz) ; 2,4 (t, 2H, J = 7,5 Hz) ; 2,05 à 1,85 (ms, 2H) ; 1,85 à 1,65 (ms, 2H) ; 1,65 à 1,45 (ms, 2H)

### Etape b : préparation de la 1-isoindolin-2-yl-6-[4-(3-trifluorométhyl-phényl)-pipérazin-1-yl]hexan-1-one

Un mélange de 1,2 g (4,05 mmol) de 2-(6-bromohexanoyl)isoindoline, de 0,93 g (4,05 mmol) de 1-(3-trifluorométhylphényl)pipérazine, de 15 mL d'acétonitrile et de 0,56 g (4,05 mmol) de carbonate de potassium est porté au reflux pendant 18 heures.
Après concentration de l'acétonitrile, le résidu est repris avec 20 mL de dichlorométhane et lavé deux fois avec 15 mL d'eau. La phase organique est séchée sur sulfate de magnésium, filtrée et concentrée. Le résidu huileux obtenu est agité avec 15 mL d'oxyde de diisopropyle pendant une heure. Le précipité est filtré et séché sous vide. On obtient ainsi 1,5 g de 1-isoindolin-2-yl-6-[4-(3-trifluorométhylphényl)pipérazin-1-yl]hexan-1-one.
Point de fusion : 100-105°C
RMN ¹H (CDCl₃) : 7,4 à 7,2 (ms, 5H) ; 7,15 à 7,0 (ms, 3H) ; 4,8 (s, 4H) ; 3,3 à 3,15 (ms, 4H) ; 2,7 à 2,55 (ms, 4H) ; 2,5 à 2,3 (ms, 4H) ; 1,9 à 1,7 (ms, 2H) ; 1,7 à 1,5 (ms, 2H) ; 1,5 à 1,35 (ms, 2H)

### Etape c : préparation du chlorhydrate de la 1-isoindolin-2-yl-6-[4-(3-trifluorométhylphényl)pipérazin-1-yl]hexan-1-one

Une addition de 3 mL d'une solution saturée de chlorure d'hydrogène dans de l'oxyde de diéthyle est effectuée sur une solution de 0,4 g (0,9 mmol) de la 1-isoindolin-2-yl-6-[4-(3-trifluorométhylphényl)pipérazin-1-yl]hexan-1-one dans 10 mL d'acétone. Après agitation pendant 15 minutes, le précipité est filtré puis séché sous vide sur pentaoxyde de phosphore. On obtient ainsi 0,37 g de chlorhydrate de 1-isoindoliri-2-yl-6-[4-(3-trifluorométhylphényl)pipérazin-1-yl]- hexan-1-one.
Point de fusion : 165°C
RMN ¹H (DMSO d₆) : 11,1 (s large, 1H) ; 7,3 (t, 1H, J= 5 Hz) ; 7,4 à 7,2 (ms, 6H) ; 7,1 (d, 1H, J = 5Hz) ; 4,8 (s, 2H) ; 4,6 (s, 2H) ; 3,9 (d, 2H, J = 8,5 Hz) ; 3,5 (d, 2H, J = 8,5 Hz) ; 3,25 (t, 2H, J = 7,5 Hz) ; 3,15 à 2,9 (ms, 4H) ; 2,35 (t, 2H, J = 7,5 Hz) ; 1,9 à 1,7 (ms, 2H) ; 1,7 à 1,5 (ms, 2H) ; 1,45 à 1,25 (ms, 2H)

### Exemple 31 : préparation de la 1-isoindolin-2-yl-6-[4-(2-méthoxyphényl)pipérazin-1-yl]hexan-1-one

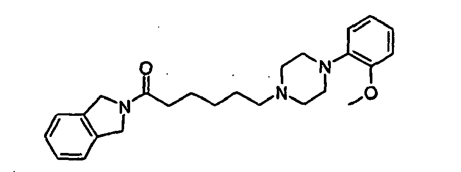

En opérant comme à l'exemple 30, étape b, mais à partir de 0,305 g de 2-(6-bromohexanoyl)isoindoline et 0,198 g de 1-(2-méthoxyphényl)pipérazine, on obtient 0,205 g de 1-isoindolin-2-yl-6-[4-(2-méthoxyphényl)pipérazin-1-yl]hexan-1-one sous forme de solide beige.
Point de fusion 100,5°C
RMN ¹H (CDCl₃) : 7,4 à 7,2 (ms, 4H) ; 7,1 à 6,8 (ms, 4H) ; 4,8 (s, 4H) ; 3,8 (s, 3H) ; 3,25 à 3,0 (ms, 4H) ; 2,8 à 2,55 (ms, 4H) ; 2,55 à 2,3 (ms, 4H) ; 1,9 à 1,5 (ms, 4H) ; 1,5 à 1,3 (ms, 2H)

### Exemple 32: préparation de la 1-isoindolin-2-yl-6-[4-(6,7,8,9-tétrahydro-5H-benzocyloheptèn-1-yl)pipérazin-1-yl]hexan-1-one

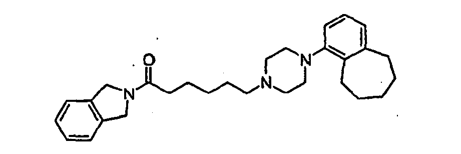

### Etape a : préparation de la 6,7,8,9-tétrahydro-5H-benzocycloheptèn-1-ylamine

En opérant comme à l'exemple 5, étape a, mais à partir de 0,5 g de 1-nitro-6,7,8,9-tétrahydro-5*H*-benzocycloheptène, on obtient 0,43 g de 6,7,8,9-tétrahydro-5H-benzocycloheptén-1-ylamine utilisée telle quelle dans les synthèses ultérieures.

Le 1-nitro-6,7,8,9-tétrahydro-5*H*-benzocyloheptène peut être obtenu selon le procédé décrit dans J. Am. Chem. Soc. (1969), 91, 3558-3566.

### Etape b: préparation de la 1-(6,7,8,9-tétrahydro-5H-benzocycloheptén-2-yl)pipérazine.

En opérant comme à l'exemple 5, étape b, mais à partir de 0,43 g de 1-(6,7,8,9-tétrahydro-5H-benzocycloheptén-2-yl)amine et 0,48 g de bis(2-chloroéthyl)amine, on obtient 0,54 g de 1-(6,7,8,9-tétrahydro-5*H*-benzocycloheptén-2-yl)pipérazine.
Rf : 0,43 dichlorométhane/méthanollarnmoniaque 90/10/0,1
7,05 (d, 1H, J = 7,5 Hz) ; 6,95 (s, 1H) ; 6,9 (t, 1H, J = 7,5 Hz) ; 3,15 à 2,9 (ms, 8H) ; 2,9 à 2,7 (ms, 4H) ; 1,95 à 1,75 (ms, 2H) ; 1,75 à 1,5 (ms, 4H)

### Etape c : préparation de la 1-isoindolin-2-yl-6-[4-(6,7,8,9-tétrahydro-5H-benzocyloheptén-1-yl)pipérazin-1-yl]hexan-1-one

En opérant comme à l'exemple 30, étape b, mais à partir de 191,5 mg de 1-(6,7,8,9-tétrahydro=5H-benzocycloheptén-2-yl)pipérazine et de 246,7 mg de 2-(6-bromohexanoyl)isoindoline, on obtient 106 mg de 1-isoindolin-2-yl-6-[4-(6,7,8,9-tétrahydro-5*H*-benzocyloheptén-1-yl)pipérazin-1-yl]hexan-1- one sous forme de solide crème.
Point de fusion : 131°C
RMN ¹H (CDCl₃) : 7,35 à 7,2 (ms, 4H) ; 7,15 à 6,8 (ms, 3H) ; 4,8 (s, 4H) ; 3,0 à 2,85 (ms, 4H) ; 3,85 à 2,5 (ms, 6H) ; 2,5 à 2,2 (ms, 6H) ; 1,95 à 1,7 (ms, 4H) ; 1,7 à 1,5 (ms, 6H) ; 1,5 à 1,3 (ms, 2H)

### Exemple 33: préparation de la 1-isoindolin-2-yl-6-[4-(2,3-diméthylphényl)-pipérazin-1-yl]hexan-1-one

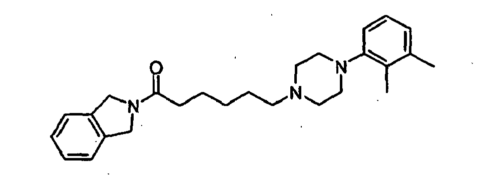

En opérant comme à l'exemple 30, étape b, mais à partir de 0,20 g de 1-(2,3-diméthylphényl)pipérazine et de 0,30 g de 2-(6-bromohexanoyl)isoindoline, on obtient 0,28 g de 1-isoindolin-2-yl-6-[4-(2,3-diméthylphényl)pipérazin-1-yl]hexan-1-one sous forme de solide beige.
Point de fusion : 114°C
RMN ¹H (CDCl₃) : 7,4 à 7,2 (ms, 4H) ; 7,2 à 7,0 (ms, 1H) ; 7,0 à 6,8 (ms, 2H) ; 4,8 (s, 4H) ; 3,05 à 2,8 (ms, 4H) : 2,8 à 2,5 (ms, 4H) ; 2,5 à 2,35 (ms, 4H) ; 2,3 (s, 3H) ; 2,1 (s, 3H) ; 1,9 à 1,7 (ms, 2H) ; 1,7 à 1,55 (ms, 2H) ; 1,55 à 1,3 (ms, 2H)

### Exemple 34 : préparation de la 1-isoindolin-2-yl-6-[4-benzofuran-7-ylpipérazin-1-yl]hexan-1-one

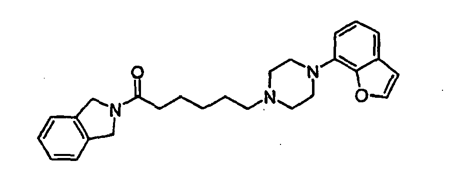

En opérant comme à l'exemple 30, étape b, mais à partir de 0,102 g de 1-benzofuran-7-ylpipérazine et de 0,150 g de 2-(6-bromohexanoyl)isoindoline, on obtient 41 mg de 1-isoindolin-2-yl-6-[4-benzofuran-7-ylpipérazin-1-yl]hexan-1-one sous forme de solide blanchâtre.
Point de fusion : 90°C
RMN ¹H (CDCl₃) : 7,6 (d, 1H. J = 2,5 Hz) ; 7,4 à 7,1 (ms, 6H) ; 6,8 à 6,7 (ms, 2H) ; 4,8 (s, 4H) ; 3,5 à 3,25 (ms, 4H) ; 2,85 à 2,6 (ms, 4H) ; 2,6 à 2,35 (ms, 4H) ; 1,9 à 1,55 (ms, 4H) ; 1,55 à 1,35 (ms, 2H)

La 1-benzofuran-7-ylpipérazine peut être préparée selon le procédé décrit dans J. Med. Chem. (1988), 31,1934-1940.

### Exemple 35 : préparation de la 1-isoindolin-2-yl-6-[4-(2-cyano-3-trifluorométhylphényl)pipérazin-1-yl]hexan-1-one

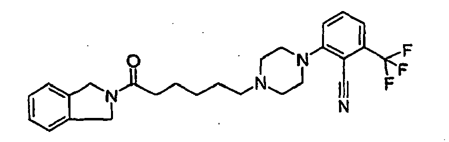

### Etape a : préparation de la 1-(2-cyano-3-trifluorométhylphényl)pipérazine.

Un mélange de 3 g (15,8 mmol) de 2-fluoro-6-trifluorométhylbenzonitrile, de 7,5 g (87 mmol) de pipérazine et de 24 mL de dioxane est chauffé au reflux pendant 5 heures.
Le milieu réactionnel est concentré sous vide et le résidu est repris avec de l'acétate d'éthyle. Après lavage avec de l'eau, la phase organique est séchée sur sulfate de magnésium, filtrée et concentrée. Le produit cristallise à température ambiante. Après séchage sous vide, on obtient 3,6 g de 1-(2-cyano-3-trifluorométhylphényl)pipérazine utilisé telle quelle dans les synthèses ultérieures. Rf : 0,65 dichlorométhane/méthanol/ammoniaque 90/10/1

### Etape b : préparation de la 1-isoindolin-2-yl-6-[4-(2-cyano-3-trifluorométhylphényl)pipérazin-1-yl]hexan-1-one

En opérant comme à l'exemple 30, étape b, mais à partir de 0,14 g de 1-(2-cyano-3-trifluorométhylphényl)pipérazine et de 0,15 g de 2-(6-bromobutanoyl)-isoindoline, on obtient 0,12 g de 1-isoindolin-2-yl-6-[4-(2-cyano-3-trifluorométhylphényl)pipérazin-1-yl]hexan-1-one sous forme de solide.
Point de fusion : 160°C
RMN ¹H (CDCl₃) : 7,5 (t, 1H, J = 8,5 Hz) ; 7,4 à 7,15 (ms, 6H) ; 4,8 (s, 4H) ; 3,4 à 3,2 (ms, 4H) ; 2,8 à 2,6 (ms, 4H) ; 2,55 à 2,3 (ms 4H) ; 1,9 à 1,7 (ms, 2H) ; 1,7 à 1,5 (ms, 2H) ; 1,5 à 1,35 (ms, 2H)

### Exemple 36 : préparation de la 1-isoindolin-2-yl-6-(4-indan-5ylpipérazin-1-yl)hexan-1-one

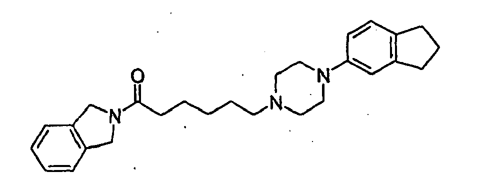

En opérant comme à l'exemple 30, étape b, mais à partir de 0,20 g de 1-(indan-5-yl)pipérazine et de 0,30 g de 2-(6-bromobutanoyl)isoindoline, on obtient 0,13 g de 1-isoindolin-2-yl-6-(4-indan-5ylpipérazin-1-yl)hexan-1-one sous forme de solide blanc.
Point de fusion : 131 °C
RMN ¹H (CDCl₃) : 7,4 à 7,2 (ms, 4H) ; 7,1 (m, 1H) ; 6,85 (s, 1H) ; 6,75 (m, 1H) ; 4,8 (s, 4H) ; 3,2 (m, 4H) ; 2,9 (m, 4H) ; 2,6 (m, 4H) ; 2,5 à 2,35 (ms, 4H) ; 2,1 (m, 2H) ; 1,8 (m, 2H) ; 1,6 (m, 2H) ; 1,5 (m, 2H)

### Exemple 37 : préparation de la 1-isoindolin-2-yl-6-(4-indan-4ylpipérazin-1-yl)hexah-1-one

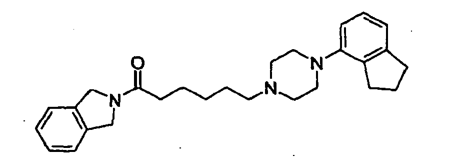

En opérant comme à l'exemple 30, étape b, mais à partir de 0,21 g de 1-(indan-4-yl)pipérazine et de 0,30 g de 2-(6-bromobutanoyl)isoindoline, on obtient 0,27 g de 1-isoindolin-2-yl-6-(4-indan-4ylpipérazin-1-yl)hexan-1-one sous forme de solide beige.
Point de fusion : 112°C
RMN ¹H (CDCl₃) : 7,4 à 7,2 (ms, 4H) ; 7,1 (m, 1 H) ; 6,9 (m, 1 H) ; 6,7 (m, 1H) ; 4,8 (s, 4H) ; 3,0 (m, 4H) ; 2,85 (m, 4H) ; 2,6 (m, 4H) ; 2,5 à 2,3 (ms, 4H) ; 2,1 (m, 2H) ; 1,8 (m, 2H) ; 1,7 à 1,55 (m, 2H) ; 1,45 (m, 2H)

### Exemple 38 : préparation de la 1-isoindolin-2-yl-6-[4-(5,6,7,8-tétrahydronaphtalén-1-yl)piperazin-1-yl]hexan-1-one

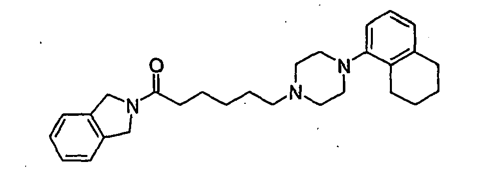

En opérant comme à l'exemple 30, étape c, mais à partir de 0,16 g de 1-(5,6,7,8-tétrahydronaphtalén-1-yl)pipérazine et de 0,22 g de 2-(6-bromobutanoyl)-isoindoline, on obtient 0,17 g de 1-isoindolin-2-yl-6-[4-(5,6,7,8-tétrahydronaphtalén-1-yl)piperazin-1-yl]hexan-1-one sous forme de solide beige.
Point de fusion : 115°C
RMN ¹H (CDCl₃) : 7,4 à 7,2 (ms, 4H) ; 7,1 (m, 1H) ; 6,9 (s, 1H) ; 6,85 (m, 1H) ; 4,8 (s, 4H) ; 2,95 (m, 4H) ; 2,85 (m, 2H) ; 2,75 (m, 2H) ; 2,6 (m, 4H) ; 2,5 à 2,3 (ms, 4H) ; 1,9 à 1,55 (ms, 8H) ; 1,45 (m, 2H)

### Exemple 39 : préparation de la 1-isoindolin-2-yl-6-[4-(3-méthanesulfonyloxyphényl)pipérazin-1-yl]hexan-1-one

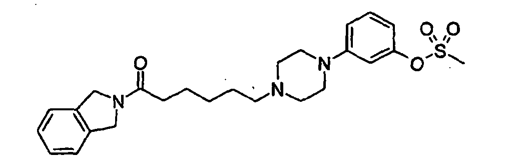

### Etape a : préparation de la 1-isoindolin-2-yl-6-[4-(3-hydroxyphényl)pipérazin-1-yl]hexan-1-one

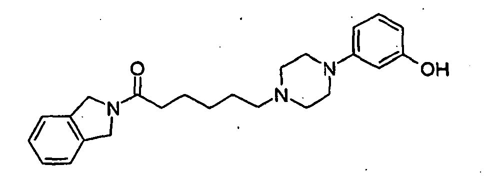

En opérant comme à l'exemple 30, étape b, mais à partir de 0,36 g de 1-(3-hydroxyphényl)pipérazine et de 0,60 g de 2-(6-bromobutanoyl)isoindoline, on obtient 0,21 g de 1-isoindolin-2-yl-6-[4-(3-hydroxyphényl)pipérazin-1-yl]hexan-1-one sous forme de solide.
RMN ¹H (DMSO D₆) : 9,0 (s, 1H); 7,4 à 7,2 (ms, 4H) ; 6,95 (m, 1H) ; 6,3 (m, 1H); 6,2 (s, 1H) ; 6,15 (m, 1H) ; 4,8 (s, 2H), 4,6 (s, 2H) ; 3,0 (m, 4H) ; 2,45 (m, 4H) ; 2,4 à 2,2 (ms, 4H) ; 1,6 (m, 2H) ; 1,45(m, 2H) ; 1,3 (m, 2H)

### Etape b : préparation de la 1-isoindolin-2-yl-6-[4-(3-méthanesulfonyloxyphényl)pipérazin-1-yl]hexan-1-one

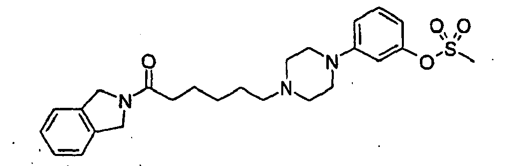

Une solution de 0,2 g (0,51 mmol) de 1-isoindolin-2-yl-6-[4-(3-hydroxyphényl)pipérazin-1-yl]hexan-1-one et de 56 mg (0,56 mmol) de triéthylamine dans 5 mL de dichlorométhane est refroidie à 0°C. Une addition de 64 mg (0,56 mmol) de chlorure de méthane sulfonyle est alors effectuée.
Le mélange est agité pendant une nuit à température ambiante, dilué avec du dichlorométhane et lavé trois fois avec de l'eau. La phase organique est séchée sur sulfate de magnésium, filtrée et concentrée.
Le résidu huileux est chromatographié sur gel de silice (gradiant dichlorométhane/méthanol 98/2 à 95/5). On obtient ainsi 75 mg (31%) de 1-isoindolin-2-yl-6-[4-(3-méthanesulfonyloxyphényl)pipérazin-1-yl]hexan-1-one sous forme de solide beige.
Point de fusion: 141°C
RMN ¹H (CDCl₃) : 7,4 à 7,2 (ms, 5H) ; 6,85 (m, 1H) ; 6,8 (s, 1H) ; 6,7 (m, 1H) ; 4,8 (s, 4H) ; 3,25 (m, 4H) ; 3,15 (s, 3H) ; 2,6 (m, 4H) ; 2,5 à 2,3 (ms, 4H) ; 1,8 (m, 2H) ; 1,6 (m, 2H) ; 1,45 (m, 2H)

### Exemple 40 : préparation de la 1-isoindolin-2-yl-6-[4-(3,4-dihydro-2H-benzo[b][1,4]dioxépin-6-yl)pipérazin-1-yl]hexan-1-one

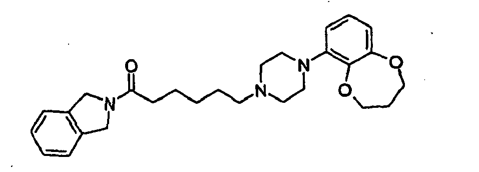

En opérant comme à l'exemple 30, étape b, mais à partir de 0,22 g de 1-(3,4-dihydro-2*H*-benzo[*b*][1,4]dioxépin-6-yl)pipérazine et de 0,17 g de 2-(6-bromobutanoyl)isoindoline, on obtient 60 mg de 1-isoindolin-2-yl-6-[4-(3,4-dihydro-2*H-*benzo[b][1,4]dioxépin-6-yl)pipérazin-1-yl]hexan-1-one sous forme de solide beige.
Point de fusion : 131 °C
RMN ¹H (CDCl₃) : 7,4 à 7,2 (ms, 4H) ; 6,85 (m, 1H) ; 6,7 à 6,55 (ms, 2H) ; 4,8 (s, 4H) ; 4,25 (m, 4H) ; 3,1 (m, 4H) ; 2,6 (m, 4H) ; 2,5 à 2,35 (ms, 4H) ; 2,2 (m, 2H) ; 1,8 (m, 2H) ; 1,6 (m, 2H) ; 1,45 (m, 2H)

### Exemple 41 : préparation de la 1-isoindolin-2-yl-6-[4-(3-trifluorométhyl-5-cyanophényl)pipérazin-1-yl]hexan-1-one

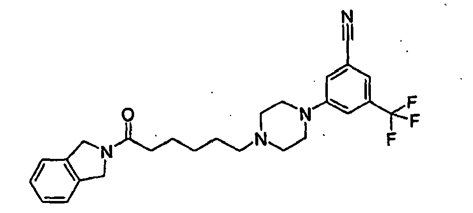

En opérant comme à l'exemple 30, étape b, mais à partir de 0,25 g de 1-(3-trifluorométhyl-5-cyanophényl)pipérazine et de 0,30 g de 2-(6-bromo-butanoyl)-isoindoline, on obtient 0,22 g de 1-isoindolin-2-yl-6-[4-(3-trifluorométhyl-5-cyanophényl)pipérazin-1-yl]hexan-1-one sous forme de solide.
Point de fusion : 128°C
RMN ¹H (CDCl₃) : 7,35 à 7,15 (ms, 7H) ; 4,8 (s, 4H) ; 3,3 (m, 4H) ; 2,6 (m, 4H) ; 2,5 à 2,3 (ms, 4H) ; 1,8 (m, 2H) ; 1,6 (m, 2H) ; 1,45 (m, 2H)

### Exemple 42 : préparation de la 1-isoindolin-2-yl-6-[4-(5,6,7,8-tétrahydronaphtalèn-2-yl)-piperazin-1-yl]hexan-1-one

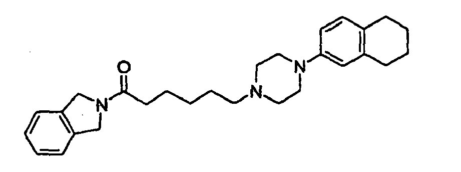

En opérant comme à l'exemple 30, étape b, mais à partir de 0,21 g de 1-(5,6,7,8-tétrahydronaphtalèn-2-yl)pipérazine et de 0,29 g de 2-(6-bromobutanoyl)-isoindoline, on obtient 0,19 g de 1-isoindolin-2-yl-6-[4-(5,6,7,8-tétrahydronaphtalèn-2-yl)-piperazin-1-yl]hexan-1-one sous forme de solide beige.
Point de fusion : 123°C
RMN ¹H (CDCl₃) : 7,35 à 7,2 (ms, 3H) ; 7,0 (m, 1 H) ; 6,75 (m, 2H) ; 6,65 (s, 1H) ; 4,8 (s, 4H) ; 3,15 (m, 4H) ; 2,75 (m, 4H) ; 2,6 (m, 4H) ; 2,5 à 2,35 (ms, 4H) ; 1,85 à 1,7 (ms, 6H) ; 1,6 (m, 2H) ; 1,45 (m, 2H)

### Exemple 43 : préparation de la 1-isoindolin-2-yl-6-[4-(3-isopropoxyphényl)-pipérazin-1-yl]hexan-1-one

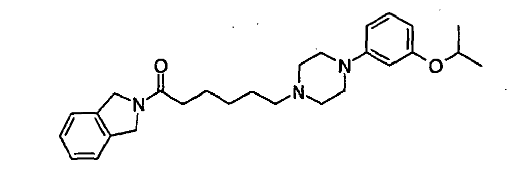

En opérant comme à l'exemple 30, étape b, mais à partir de 0,20 g. de1-(3-isopropoxyphényl)pipérazine et de 0,30 g de 2-(6-bromobutanoyl)isoindoline, on obtient 0,29 g de 1-isoindolin-2-yl-6-[4-(3-isopropoxyphényl)pipérazin-1-yl]hexan-1-one sous forme de solide beige.
RMN ¹H (CDCl₃) : 7,4 à 7,2 (ms, 4H) ; 7,15 (m, 1H) ; 6,6 à 6,45 (ms, 3H) ; 4,8 (s, 4H) ; 4,55 (m, 1H) ; 3,2 (m, 4H) ; 2,6 (m, 4H) ; 2,5 à 2,3 (ms, 4H) ; 1,8 (m, 2H) ; 1,6 (m, 2H) ; 1,45 (m, 2H) ; 1,3 (d, 6H, J = 7Hz)

### Exemple 44 : préparation de la 1-isoindolin-2-yl-6-[4-(2-cyano-3-méthylphényl)-pipérazin-1-yl]hexan-1-one

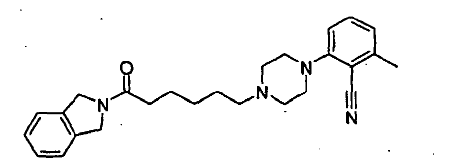

En opérant comme à l'exemple 30, étape b, mais à partir de 0,20 g de 1-(2-cyano-3-méthylphényl)pipérazine et de 0,30 g de 2-(6-bromobutanoyl)isoindoline, on obtient 0,21 g de 1-isoindolin-2-yl-6-[4-(2-cyano-3-méthylphényl)pipérazin-1-yl]hexan-1-one sous forme de solide beige.
Point de fusion: 126°C
RMN ¹H (CDCl₃) : 7,4 à 7,2 (m, 5H) ; 6,95 à 6,75 (ms, 2H) ; 4,8 (s, 4H) ; 3,2 (m, 4H) ; 2,7 (m, 4H) ; 2,5 (s, 3H) ; 2,45 à 2,3 (ms, 4H) ; 1,8 (m, 2H) ; 1,6 (m, 2H); 1,45 (m, 2H)

### Exemple 45 : préparation de la 1-isoindolin-2-yl-6-[4-(2-cyano-3-isopropoxy)-pipérazin-1-yl]hexan-1-one

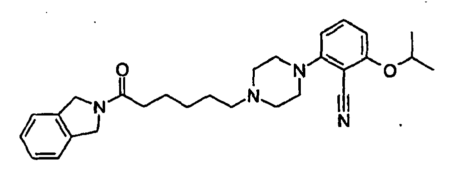

En opérant comme à l'exemple 30, étape b, mais à partir de 90 mg de 1-(2-cyano-3-isopropoxyphényl)pipérazine et de 0,15 g de 2-(6-bromobutanoyl)-isoindoline, on obtient 66 mg de 1-isoindolin-2-yl-6-[4-(2-cyano-3-isopropoxy)-pipérazin-1-yl]hexan-1-one sous forme de solide beige.
Point de fusion : 112°C
RMN ¹H (CDCl₃) : 7,4 à 7,2 (ms, 5H) ; 6,5 (m, 2H) ; 4,8 (s, 4H) ; 4,6 (m, 1H), 3,2 (m, 4H) ; 2,65 (m, 4H) ; 2,55 à 2,2,3 (ms, 4H) ; 1,8 (m, 2H) ; 1,6 (m, 2H) ; 1,45 (m, 2H) ; 1,4 (d, 6H, J = 7Hz)

### Exemples 46 à 48 : préparation parallèle des 1-(5,6-diméthoxyisoindolin-2-yl)-6-[4-(3-trifluorométhylphényl)pipérazin-1-yl]hexan-1-one, 1-isoindolin-2-yl-6-[4-(2-cyanophényl)pipérazin-1-yl]hexan-1-one et 1-isoindolin-2-yl-6-[4-(2-méthylphényl)pipérazin-1-yl]hexan-1-one

### Etape a : préparation du 6-[4-(2-cyanophényl)pipérazin-1-yl]hexanoate d'éthyle

En opérant comme à l'exemple 1, étape a, mais à partir de 5 g 1-(2-cyanophényl)pipérazine et de 5,95 g 6-bromohexanoate d'éthyle, on obtient 8,25 g 6-[4-(2-cyanophényl)pipérazin-1-yl]hexanoate d'éthyle.

### Etape b : préparation de l'acide 6-[4-(2-cyanophényl)pipérazin-1-yl]hexanoïque

En opérant comme à l'exemple 1, étape b, mais à partir de 8,25 g 6-[4-(2-cyanophényl)pipérazin-1-yl]hexanoate d'éthyle, on obtient 6,83 g d'acide 6-[4-(2-cyanophényl)pipérazin-1-yl]hexanoïque sous forme de solide blanc.
Point de fusion: 120°C.

### Etape c : préparation du 6-[4-(2-méthylphényl)pipérazin-1-yl]hexanoate d'éthyle

En opérant comme à l'exemple 1, étape a, mais à partir de 4,98 g de dichlorhydrate de 1-(2-méthylphényl)pipérazine, de 8,28 g de carbonate de potassium et de 4,46 g 6-bromohexanoate d'éthyle, on obtient 6,3 g de 6-[4-(2-méthylphényl)pipérazin-1-yl]hexanoate d'éthyle.

### Etape d : préparation de l'acide 6-[4-(2-méthylphényl)pipérazin-1-yl]hexanoïque

En opérant comme à l'exemple 1, étape b, mais à partir de 6,3 g de 6-[4-(2-méthylphényl)pipérazin-1-yl]hexanoate d'éthyle, on obtient, en deux jets, 3,35 g d'acide 6-[4-(2-méthylphényl)pipérazin-1-yl]hexanoïque sous forme de solide blanc.
Point de fusion : 125°C.

### Etape e : préparation parallèle des 1-(5,6-diméthoxyisoindolin-2-yl)-6-[4-(3-trifluorométhyl-phényl)pipérazin-1-yl]hexan-1-one, 1-isoindolin-2-yl-6-[4-(2-cyanophényl)pipérazin-1-yl]hexan-1-one et 1-isoindolin-2-yl-6-[4-(2-méthyl-phényl)-pipérazin-1-yl]hexan-1-one

A une solution d'acide (0,3 mmol) dans 2 mL de N,N-diméthylformamide anhydre, on ajoute un mélange de 0,3 mmol de dicyclohexylcarbodiimide et de 0,3 mmol de 1-hydroxybenzotriazole. Le mélange est laissé sous agitation pendant quatre heures à 70°C. Il est ensuite refroidi à température ambiante, filtré pour éliminer la dicyclohexylurée. L'amine (0,2 mmol) est ensuite ajoutée, puis on laisse vingt heures à température ambiante.
Le *N*,*N*-diméthylformamide est évaporé sous vide et le brut est repris par 2 mL de dichlorométhane. On lave ensuite par deux fois 1 mL d'une solution aqueuse normale de soude, puis par 1 mL d'eau. La phase organique est séchée puis concentrée sous vide.
A partir d'acide 6-[4-(3-trifluorométhyl-phényl)pipérazin-1-yl]hexanoïque et de 5,6-diméthoxyisoindotine, on obtient ainsi la 1-(5,6-diméthoxyisoindolin-2-yl)-6-[4-(3-trifluorométhyl-phényl)pipérazin-1-yl]hexan-1-one.
Rf : 0,76 dichlorométhane/méthanol 85/15
A partir d'acide 6-[4-(2-cyanophényl)pipérazin-1-yl]hexanoïque et d'isoindoline, on obtient ainsi la 1-isoindolin-2-yl-6-[4-(2-cyanophényl)pipérazin-1-yl]hexan-1-one.
Rf : 0,58 dichlorométhane/méthanol 90/10
A partir d'acide 6-[4-(2-méthyl-phényl)pipérazin-1-yl]hexanoïque et d'isoindoline, on obtient ainsi la 1-isoindolin-2-yl-6-[4-(2-méthyl-phényl)pipérazin-1-yl]hexan-1-one.
Rf : 0,58 dichlorométhane/méthanol 90/10

### Exemple 49: préparation de la 1-isoindolin-2-yl-6-(4-phényl-3,6-dihydro-2H-pyridin-1-yl)hexan-1-one

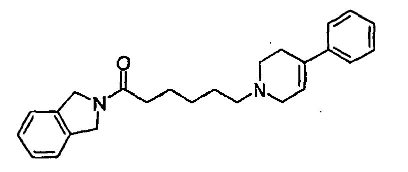

En opérant comme à l'exemple 30, étape b, mais à partir de 0,21 g de 4-phényl-1,2,3,6-tétrahydropyridine et de 0,30 g de 2-(6-bromobutanoyl)isoindoline, on obtient 0,17g de 1-isoindolin-2-yl-6-[4-(phényl-3,6-dihydro-2H-pyridin-1-yl]hexan-1-one sous forme de solide beige.
Point de fusion : 129°C
RMN ¹H (CDCl₃) : 7,45 à 7,15 (ms, 9H) ; 6,05 (m, 1 H) ; 4,85 (s, 2H) ; 4,5 (s, 2H) ; 3,15 (m, 2H) ; 2,7 (m, 2H) ; 2,6 (m, 2H) ; 2,55 à 2,3 (4H) ; 1,8 (m, 2H) ; 1,6 (m, 2H) ; 1,45 (m, 2H)

### Exemple 50: préparation de la 1-isoindolin-2-yl-6-[4-(3-trifluorométhylphényl)-3,6-dihydro-2H-pyridin-1-yl]hexan-1-one

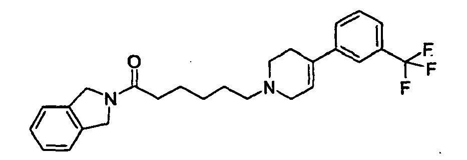

En opérant comme à l'exemple 30, étape b, mais à partir de 0,13 g de 4-(3-trifluorométhylphényl)-1,2,3,6-tétrahydropyridine et de 0,15 g de 2-(6-bromobutanoyl)isoindoline, on obtient 0,10 g de 1-isoindolin-2-yl-6-[4-(3-trifluorométhylphényl-3,6-dihydro-2*H*-pyridin-1-yl]hexan-1-one sous forme de solide beige.
Point de fusion : 86°C
RMN ¹H (CDCl₃) : 7,65 (s, 1H) ; 7,6 à 7,35 (ms, 3H) ; 7,35 à 7,2 (ms, 4H) ; 6,15 (m, 1 H) ; 4,8 (s, 4H) ; 3,2 (m, 2H) ; 2,7 (t, 2H, J = 7Hz) ; 2,6 (m, 2H) ; 2,55 à 2,35 (ms, 4H) ; 1,8 (m, 2H) ; 1,6 (m, 2H) ; 1,45 (m, 2H)

### Exemple 51 : préparation de la 1-isoindolin-2-yl-6-[4-(3-trifluorométhyl-phényl)-pipéridin-1-yl]hexan-1-one

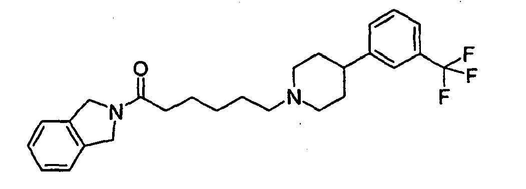

En opérant comme à l'exemple 30, étape b, mais à partir de 0,21 g de 4-(3-trifluorométhylphényl)pipéridine et de 0,25 g de 2-(6-bromobutanoyl)isoindoline, on obtient 0,23 g de 1-isoindolin-2-yi-6-[4-(3-trifluorométhylphényl)pipéridin-1-yl]hexan-1-one sous forme de solide gris clair.
Point de fusion: 71 °C
RMN ¹H (CDCl₃) : 7,55 à 7,35 (ms, 4H) ; 7,35 à 7,2 (ms, 4H) ; 4,8 (s, 4H) ; 3,1 (m, 2H) ; 2,6 (m, 1H) ; 2,4 (t, 2H; J = 7Hz) ; 2,1 (m, 2H) ; 1,95 à 1,7 (ms, 6H) ;
1,7 à 1,55 (ms, 4H) ; 1,45 (m, 2H)

### Exemple 52: préparation de la 1-isoindolin-2-yl-6-[4-(2-cyano-3-méthylphényl)-3,6-dihydro-2H-pyridin-1-yl]hexan-1-one

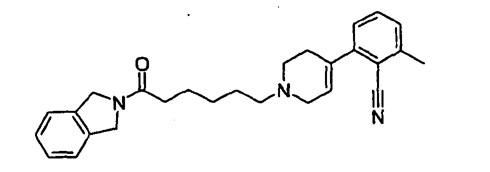

### Etape a : préparation du 4-(2-cyano-3-méthylphényl)-3,6-dihydro-2H-pyridin-1-carboxylate de tert-butyle

Une solution de 1,5 g (7,6 mmol) de 2-bromo-6-méthylbenzonitrile dans 10 mL de tétrahydrofurane est ajoutée à -65°C sur une solution de 4 mL de butyllithium 2,5M dans l'hexane. Après trente minutes d'agitation à -78°C, une solution de 16 mL (8 mmol) de chlorure de zinc 0,5 M dans le tétrahydrofurane est ajoutée. L'agitation est poursuivie pendant 90 minutes en laissant la température remonter. Le milieu réactionnel est refroidi à -10°C. Une addition de 0,24 g (0,31 mmol) de palladium tétrakistriphénylphosphine est effectuée suivie d'une addition de 1,7 g (5 mmol) de 4-trifluorométhanesulfonyloxy-3,6-dihydro-2*H*-pyridin-1-carboxylate de *tert*-buyle (préparation décrite dans J. Med. Chem., (2000), 43, 2703-2718) dans 4 mL de tétrahydrofurane. Le milieu est chauffé à 40°C pendant une heure.
Après refroidissement à température ambiante, le milieu est versé sur 150 mL d'une solution aqueuse hydrogénocarbonate de sodium. Le produit est extrait deux fois avec de l'acétate d'éthyle. Les phases d'extractions sont réunies, séchées sur sulfate de magnésium, filtrées et concentrées. Le produit est purifié par chromatographie sur gel de silice (éluant heptane/acétate d'éthyle 4/1). On obtient ainsi 1,4 g (94%) de 4-(2-cyano-3-méthylphényl)-3,6-dihydro-2*H*-pyridin-1-carboxylate de *tert*-buyle sous forme d'huile qui cristallise à température ambiante.

### Etape b : préparation du chlorhydrate de la 4-(2-cyano-3-méthylphényl)-3,6-dihydro-2H-pyridine

A une solution de 1,4 g (4,7 mmol) de 4-(2-cyano-3-méthylphényl)-3,6-dihydro-2*H*-pyridin-1-carboxylate de *tert*-butyle dans 15 mL d'acétate d'éthyle refroidie à 0°C sont ajoutés 15 mL d'une solution saturée de chlorure d'hydrogène dans l'oxyde de diéthyle. L'agitation est maintenue pendant une nuit à température ambiante.
Le précipité est filtré, lavé avec de l'oxyde de diéthyle et séché sous vide. On obtient ainsi 0,88 g (81%) de chlorhydrate de la 4-(2-cyano-3-méthylphényl)-3,6-dihydro-2*H*-pyridine.

### Etape c : préparation de la 1-isoindolin-2-yl-6-[4-(2-cyano-3-méthylphényl-3,6-dihydro-2H-pyridin-1-yl]hexan-1-one

En opérant comme à l'exemple 30, étape b, mais à partir de 0,40 g de chlorhydrate de la 4-(2-cyano-3-méthylphényl)-3,6-dihydro-2*H*-pyridine et de 0,50 g de 2-(6-bromobutanoyl)isoindoline, on obtient 0,57 g de 1-isoindolin-2-yl-6-[4-(2-cyano-3-méthylphényl)-3,6-dihydro-2H-pyridin-1-yl]hexan-1-one sous forme de solide beige
Point de fusion : 71 °C
RMN ¹H (CDCl₃) : 7,4 (m, 1H); 7,35 à 7,2 (ms, 4H), 7,15 (m, 2H) ; 6,0 (m, 1H); 4,8 (s, 4H) ; 3,2 (m, 2H) ; 2,75 (t, 2H, J = 5Hz) ; 2,65 à 2,5 (ms, 7H) ; 2,4 (t, 2H, J = 7Hz) ; 1,8 (m, 2H) ; 1,6 (m, 2H) ; 1,45 (m, 2H)

### Exemple 53 : préparation de la 1-isoindolin-2-yl-6-[4-(2-cyano-3-méthylphényl)-pipéridin-1-yl]hexan-1-one

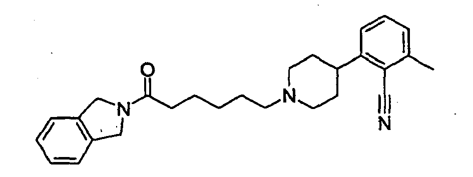

Un mélange de 0,27 g (0,65 mmol) de 1-isoindolin-2-yl-6-[4-(2-cyano-3-méthylphényl)-3,6-dihydro-2H-pyridin-1-yl]hexan-1-one, de 10 mL de méthanol, de 25 µL d'acide acétique et de 0,14 g de palladium sur charbon à 10% est hydrogéné à pression atmosphérique et à température ambiante pendant une nuit.
Après filtration sur célite et rinçage avec du dichlorométhane, le filtrat est concentré puis trituré dans de l'éther de pétrole. Le solide est filtré et dissout dans de l'eau. La solution est basifiée avec une solution aqueuse de soude normale. Le produit est filtré, lavé avec de l'eau et séché sous vide. On obtient ainsi 0,18 g de 1-isoindolin-2-yl-6-[4-(2-cyano-3-méthylphényl)pipéridin-1-yl]hexan-1-one
Point de fusion : 132°C
RMN ¹H (CDCl₃) : 7,4 (m, 1H) ; 7,35 à 7,1 (ms, 6H) ; 4,8 (s, 4H) ; 3,1 (m, 2H) ; 3,0 (m, 1H) ; 2,55 (s, 3H) ; 2,4 (m, 4H) ; 2,15 (m, 2H) ; 2,0 à 1,7 (ms, 6H) ; 1,6 (m, 2H) ; 1,45 (m, 2H)

### Résultats Biologiques : Détermination de l'affinité des composés pour le récepteur humain D3 à la dopamine

### Liaison de [³H] spiperone :

Des cellules CHO ont été transfectées par le gène codant pour le récepteur humain D3 à la dopamine (hD3). La liaison de [³H]spiperone (0,5 à 2 nM) s'effectue en présence de 5 à 10 µg de protéines membranaires dans un milieu contenant 120 mM NaCl, 5 mM KCl, et 50 mM Tris HCL pH 7,4 ; une incubation de 60 minutes à température ambiante est nécessaire. La liaison non spécifique est estimée en présence de 5 µM d'halopéridol. Les cellules non transfectées sont dépourvues de toute activité de liaison.

**Tableau 1: Effets antagonistes (Ki, nM) sur les récepteurs hD3 mesurés par liaison de [³H] spiperone**

| N° d'exemple | hD3 Ki (nM) |
|---|---|
| 1 | 0,9 |
| 3 | 2,6 |
| 7 | 1,05 |
| 10 | 1,17 |
| 12 | 0,85 |
| 24 | 1,21 |
| 38 | 0,84 |
| 50 | 0,93 |
| 51 | 19,65 |

### Exemple comparatif

WO 01/49769 décrit des composés présentant un groupe phényle sur la position 4 de la pipérazine, ledit groupe phényle étant substitué par un atome d'halogène. Le résultat suivant démontre que un tel composé présente une faible affinité pour le récepteur D3 de la dopamine :

| Structure | Ki (nM) |
|---|---|
| 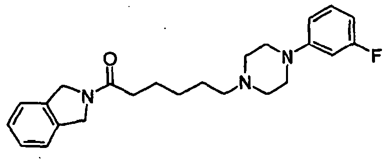 | 321 nM |

## Revendications

1. Composés de formule (I) : 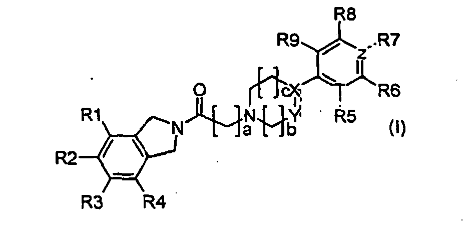 dans laquelle :
■ a = 5 ou 6 ;
■ b et c identiques ou différents représentent 1 ou 2 ;
■ X ---- Y représente >N-CH₂-, >C=CH-, >CH-CH₂- ;
■ Z et T identiques ou différents représentent indépendamment un atome d'azote ou de carbone ;
■ ----- est absent ou représente une simple liaison, étant entendu que lorsque Z et/ou T représente un atome d'azote, - est absent ;
■ R1, R2, R3 et R4 représentent chacun indépendamment un atome d'hydrogène ou d'halogène ou un groupement hydroxy, alkyle, monofluoroalkyle, polyfluoroalkyle, alkoxy, polyfluoroalkoxy, alkylsulfanyle, polyfluoroalkylsulfanyle, cyano, -OS(O)ₘ-Alkyle, -NRR', -COOR, -COR, -CONRR', -C(OH)RR', -alkylC(OH)RR', -alkylCOOR, -alkylCOR, -alkylCONRR', -alkylNRR', -OalkylNRR', -Oalkyl(NRR')COOR ou hétérocycle saturé ou insaturé ou bien deux des R1, R2, R3 et R4 adjacents sont reliés entre eux pour former un cycle hydrocarboné ou un hétérocycle saturé ou insaturé, fusionné au noyau phényle auquel ils sont rattachés ;
■ R5, R6, R7, R8 et R9 représentent chacun indépendamment un atome d'hydrogène ou un groupement hydroxy, alkyle, monofluoroalkyle, polyfluoroalkyle, alkoxy, polyfluoroalkoxy, alkylsulfanyle, polyfluoroalkylsulfanyle, cyano, -NRR', -COOR, -COR, -CONRR' ou bien deux des R5, R6, R7, R8 et R9 adjacents sont reliés entre eux pour former un cycle hydrocarboné ou un hétérocycle saturé ou insaturé, fusionné au noyau phényle auquel ils sont rattachés ;
où R, R' identiques ou différents représentent indépendamment un atome d'hydrogène, un groupe alkyle éventuellement substitué par un ou plusieurs groupes choisis parmi OH, NRR', OR, COOR ou R et R' sont reliés entre eux pour former un polyméthylène ou un oxapolyméthylène ;
m= 0, 1 ou 2 ;
étant entendu que lorsque T=Z=N, R5 et R7 sont absents, R6 et R8 sont choisis indépendamment parmi un atome d'hydrogène ou un groupement alkyle, monofluoroalkyle, polyfluoroalkyle, alkoxy, polyfluoroalkoxy, alkylsulfanyle, polyfluoroalkylsulfanyle, cyano, -COOR, -COR, -CONRR' et R9 est choisi parmi un atome d'hydrogène ou un groupement hydroxy, alkyle, monofluoroalkyle, polyfluoroalkyle, alkoxy, polyfluoroalkoxy, alkylsulfanyle, polyfluoroalkylsulfanyle, -NRR', -COOR, -COR, -CONRR'
ou bien R8 et R9 sont reliés entre eux pour former un cycle hydrocarboné ou un hétérocycle saturé ou insaturé, fusionné au noyau phényle auquel ils sont rattachés ;
ainsi que leurs stéréoisomères ou leur mélanges, leurs formes tautomères, leurs hydrates, solvates, leurs sels et esters pharmaceutiquement acceptables,

2. Composés selon la revendication 1 selon laquelle a = 5.

3. Composés selon la revendication 1 ou 2 selon laquelle b et c représentent 1.

4. Composés selon l'une quelconque des revendications précédentes tels que ---- Y représente >N-CH₂-.

5. Composés selon l'une quelconque des revendications précédentes tels que Z=T=C ou Z=T=N.

6. Composés selon l'une quelconque des revendications précédentes tels que R1, R2, R3, R4 représentent indépendamment un atome d'hydrogène ou d'halogène ou un groupe alkoxy, -OS(O)*ₘ*-Alkyle, cyano, -COOR, -COR, -CONRR', -C(OH)RR', -OalkylNRR'

7. Composés selon l'une quelconque des revendications précédentes tels que lorsque T=Z=C, R5, R6, R7, R8, R9 représentent indépendamment un atome d'hydrogène ou un groupement alkoxy, alkyle, cyano, polyfluoroalkoxy, tel qu'un groupe perfluoroalkoxy, tel que trifluorométhoxy, ou un groupement polyfluoroalkyle, tel qu'un groupe perfluoroalkyle, tel que trifluorométhyle, ou bien deux des R5, R6, R7, R8 et R9 adjacents sont reliés entre eux pour former un cycle hydrocarboné ou un hétérocycle saturé ou insaturé, fusionné au noyau phényle auquel ils sont rattachés, tel que un cycle benzocycloheptène ou benzofurane.

8. Composés selon l'une quelconque des revendications 1 à 6 tels que lorsque T=Z=N, R5 et R7 sont absents, R6 et R8 sont choisis indépendamment parmi un atome d'hydrogène ou un groupement alkyle, monofluoroalkyle, polyfluoroalkyle, alkoxy, polyfluoroalkoxy, alkylsulfanyle, polyfluoroalkylsulfanyle, cyano, -COOR, -COR, -CONRR' et R9 est choisi parmi un atome d'hydrogène ou un groupement hydroxy, alkyle, monofluoroalkyle, polyfluoroalkyle, alkoxy, polyfluoroalkoxy, alkylsulfanyle, polyfluoroalkylsulfanyle, -NRR', -COOR, -COR, -CONRR' ou bien R8 et R9 sont reliés entre eux pour former un cycle hydrocarboné ou un hétérocycle saturé ou insaturé, fusionné au noyau phényle auquel ils sont rattachés.

9. Composés selon l'une quelconque des revendications 1 à 7 tels qu'ils sont choisis parmi les composés de formule (l') : 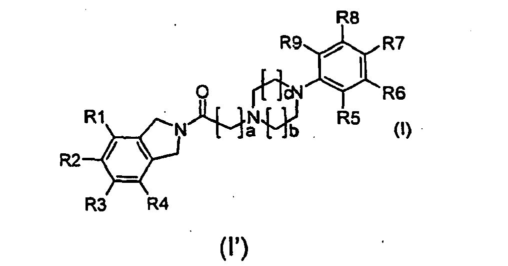 dans laquelle a, b et c, R1, R2, R3 et R4, R5, R6, R7, R8 et R9 sont tels que définis en formule (I),
ainsi que leurs stéréoisomères ou leur mélanges, leurs formes tautomères, leurs hydrates, solvates, leurs sels et esters pharmaceutiquement acceptables.

10. Composés selon l'une quelconque des revendications précédentes choisis parmi :
- 1-(5-méthoxyisoindolin-2-yl)-6-[4-(3-trifluorométhylphényl)pipérazin-1-yl]hexan-1-one
- 1-(5-méthanesulfonyloxyisoindolin-2-yl)-6-[4-(3-trifluorométhylphényl)-pipérazin-1-yl]hexan-1-one
- chlorhydrate de 1-(5-cyanoisoindolin-2-yl)-6-[4-(3-trifluorométhylphényl)-pipérazin-1-yl]-hexan-1-one
- 1-isoindolin-2-yl-6-[4-(3-trifluorométhoxyphényl)pipérazin-1-yl]hexan-1-one
- 1-isoindolin-2-yl-6-[4-(6,7,8,9-tétrahydro-5*H*-benzocyloheptén-2-yl)pipérazin-1-yl]hexan-1-one
- 1-(4-fluoroisoindolin-2-yl)-6-[4-(3-trifluorométhylphényl)pipérazin-1-yl]hexan-1-one
- 1-(5-fluoroisoindolin-2-yl)-6-[4-(3-trifluorométhylphényl)pipérazin-1-yl]hexan-1-one
- 1-(5-isopropoxyisoindolin-2-yl)-6-[4-(3-trifluorométhylphényl)pipérazin-1-yl]hexan-1-one
- 1-(4-chloroisoindolin-2-yl)-6-[4-(3-trifluorométhylphényl)pipérazin-1-yl]hexan-1-one
- 1-(6,7-dihydro-5*H*-[1,3]dioxolo[4,5-*f*]isoindol-6-yl)-6-[4-(3-trifluorométhylphényl)pipérazin-1-yl]hexan-1-one
- 1-(5-hydroxyméthylisoindolin-2-yl)-6-[4-(3-trifluorométhylphényl)-pipérazin-1-yl]hexan-1-one
- chlorhydrate de la 1-(5-méthoxycarbonylisoindolin-2-yl)-6-[4-(3-trifluorométhylphényl)pipérazin-1-yl]hexan-1-one
- 1-(5-carboxyisoindolin-2-yl)-6-[4-(3-trifluorométhylphényl)pipérazin-1-yl]hexan-1-one
- 1-[5-(2-hydroxyéthylaminocarbonyl)isoindolin-2-yl]-6-[4-(3-trifluorométhylphényl)pipérazin-1-yl]hexan-1-one
- 1-(5-morpholinocarbonylisoindolin-2-yl)-6-[4-(3-trifluorométhylphényl)-pipérazin-1-yl]hexan-1-one
- 1-(5-(pyrrolidin-1-ylcarbonylisoindolin-2-yl)-6-[4-(3-trifluorométhylphényl)-pipérazin-1-yl]hexan-1-one
- 1-(5-pipéridin-1-ylcarbonylisoindolin-2-yl)-6-[4-(3-trifluorométhylphényl)-pipérazin-1-yl]hexan-1-one
- 1-[5-(2-diméthylamino-éthylaminocarbonyl)isoindolin-2-yl]-6-[4-(3-trifluorométhylphényl)pipérazin-1-yl]hexan-1-one
- 1-[5-(4,5-dihydrooxazol-2yl)isoindolin-2-yl]-6-[4-(3-trifluorométhylphényl)-pipérazin-1-yl]hexan-1-one
- chlorhydrate de la 1-(5-morpholinoylméthylisoindolin-2-yl)-6-[4-(3-trifluorométhylphényl)pipérazin-1-yl]hexan-1-one
- 1-[5-(pyrrolidin-1-ylméthyl)isoindolin-2-yl]-6-[4-(3-trifluorométhylphényl)-pipérazin-1-yl]hexan-1-one
- 1-[5-(diméthylaminométhyl)isoindolin-2-yl]-6-[4-(3-trifluorométhylphényl)-pipérazin-1-yl]hexan-1-one
- 1-[5-(2-pipéridinoéthoxy)isoindolin-2-yl]-6-[4-(3-trifluorométhylphényl)-pipérazin-1-yl]hexan-1-one
- 1-[5-(3-pipéridinopropoxy)isoindolin-2-yl]-6-[4-(3-tritluorométhylphényl)-pipérazin-1-yl]hexan-1-one
- 1-[5-(3-pyrrolidinopropoxy)isoindolin-2-yl]-6-[4-(3-trifluorométhylphényl)-pipérazin-1-yl]hexan-1-one
- 1-(5-acétylisoindolin-2-yl)-6-[4-(3-trifluorométhylphényl)pipérazin-1-yl]hexan-1-one
- 1-[5-(1-hydroxy-1-méthyléthyl)isoindolin-2-yl]-6-[4-(3-trifluorométhylphényl)pipérazin-1-yl]hexan-1-one
- chlorhydrate de la 1-[5-(1-hydroxyéthyl)isoindolin-2-yl]-6-[4-(3-trifluorométhylphényl)pipérazin-1-yl]hexan-1-one
- 1-(5-aminocarbonylisoindolin-2-yl)-6-[4-(3-trifluorométhylphényl)-pipérazin-1-yl]hexan-1-one
- chlorhydrate de la 1-isoindolin-2-yl-6-[4-(3-trifluorométhylphényl)-pipérazin-1-yl]hexan-1-one
- 1-isoindolin-2-yl-6-[4-(2-méthoxyphényl)pipérazin-1-yl]hexan-1-one
- 1-isoindolin-2-yl-6-[4-(6,7,8,9-tétrahydro-5*H*-benzocyloheptén-1-yl)pipérazin-1-yl]hexan-1-one
- 1-isoindolin-2-yl-6-[4-(2,3-diméthylphényl)pipérazin-1-yl]hexan-1-one
- 1-isoindolin-2-yl-6-[4-benzofuran-7-ylpipérazin-1-yl]hexan-1-one
- 1-isoindolin-2-yl-6-[4-(2-cyano-3-trifluorométhylphényl)pipérazin-1-yl]hexan-1-one
- 1-isoindolin-2-yl-6-(4-indan-5ylpipérazin-1-yl)hexan-1-one
- 1-isoindolin-2-yl-6-(4-indan-4ylpipérazin-1-yl)hexan-1-one
- 1-isoindolin-2-yl-6-[4-(5,6,7,8-tétrahydronaphtalén-1-yl)piperazin-1-yl]hexan-1-one
- 1-isoindolin-2-yl-6-[4-(3-méthanesulfonyloxyphényl)pipérazin-1-yl]hexan-1-one
- 1-isoindolin-2-yl-6-[4-(3,4-dihydro-2*H*-benzo[*b*][1,4]dioxépin-6-yl)pipérazin-1-yl]hexan-1-one
- 1-isoindolin-2-yl-6-[4-(3-trifluorométhyl-5-cyanophényl)pipérazin-1-yl]hexan-1-one
- 1-isoindolin-2-yl-6-[4-(5,6,7,8-tétrahydronaphtalén-2-yl)-piperazin-1-yl]hexan-1-one
- 1-isoindolin-2-yl-6-[4-(3-isopropoxyphényl)pipérazin-1-yl]hexan-1-one
- 1-isoindolin-2-yl-6-[4-(2-cyano-3-méthylphényl)pipérazin-1-yl]hexan-1-one
- 1-isoindolin-2-yl-6-[4-(2-cyano-3-isopropoxy)pipérazin-1-yl]hexan-1-one
- 1-(5,6-diméthoxyisoindolin-2-yl)-6-[4-(3-trifluorométhylphényl)pipérazin-1-yl]hexan-1-one
- 1-isoindolin-2-yl-6-[4-(2-cyanophényl)pipérazin-1-yl]hexan-1-one
- 1-isoindolin-2-yl-6-[4-(2-méthyl-phényl)pipérazin-1-yl]hexan-1-one
- 1-isoindolin-2-yl-6-(4-phényl-3,6-dihydro-*2H*-pyridin-1-yl)hexan-1-one
- 1-isoindolin-2-yl-6-[4-(3-trifluorométhylphényl)-3,6-dihydro-*2H*-pyridin-1-yl]hexan-1-one
- 1-isoindolin-2-yl-6-[4-(3-trifluorométhylphényl)pipéridin-1-yl]hexan-1-one
- 1-isoindolin-2-yl-6-[4-(2-cyano-3-méthylphényl)-3,6-dihydro-*2H*-pyridin-1-yl]hexan-1-one
- 1-isoindolin-2-yl-6-[4-(2-cyano-3-méthylphényl)pipéridin-1-yl]hexan-1-one,
ainsi que leurs stéréoisomères ou leurs mélanges, leurs formes tautomères, leurs hydrates, solvates, leurs sels, formes libres et esters pharmaceutiquement acceptables.

11. Composés selon l'une quelconque des revendications précédentes choisis parmi :
- 1-(5-méthoxyisoindolin-2-yl)-6-[4-(3-trifluorométhylphényl)pipérazin-1-yl]hexan-1-one
- chlorhydrate de 1-(5-cyanoisoindolin-2-yl)-6-[4-(3-trifluorométhylphényl)-pipérazin-1-yl]-hexan-1- one
- 1-(5-fluoroisoindolin-2-yl)-6-[4-(3-trifluorométhyl-phényl)pipérazin-1-yl]hexan-1-one
- 1-(6,7-dihydro-5*H-*[1,3]dioxo(o[4,5-f]isoindol-6-yl)-6-[4-(3-trifluorométhylphényl)-pipérazin-1-yl]hexan-1-one
- chlorhydrate de la 1-(5-méthoxycarbonylisoindolin-2-yl)-6-[4-(3-trifluorométhyl-phényl)pipérazin-1-yl]hexan-1-one
- 1-[5-(3-pipéridinopropoxy)isoindolin-2-yl]-6-[4-(3-trifluorométhylphényl)pipérazin-1-yl]hexan-1-one
- 1-isoindolin-2-yl-6-[4-(5,6,7,8-tétrahydro-naphtalén-1-yl)piperazin-1-yl]hexan-1-one
- 1-isoindolin-2-yl-6-[4-(3-trifluorométhylphényl)-3,6-dihydro-2*H*-pyridin-1-yl]hexan-1-one
- 1-isoindolin-2-yl-6-[4-(3-trifluorométhyl-phényl)pipéridin-1-yl]hexan-1-one
ainsi que leurs stéréoisomères ou leurs mélanges, leurs formes tautomères , leurs hydrates, solvates, leurs sels, formes libres et esters pharmaceutiquement acceptables.

12. Procédé de préparation d'un composé de formule (I) selon l'une quelconque des revendications précédentes tel que ledit procédé comprend l'étape de couplage des composés de formule (II) et (III) ; 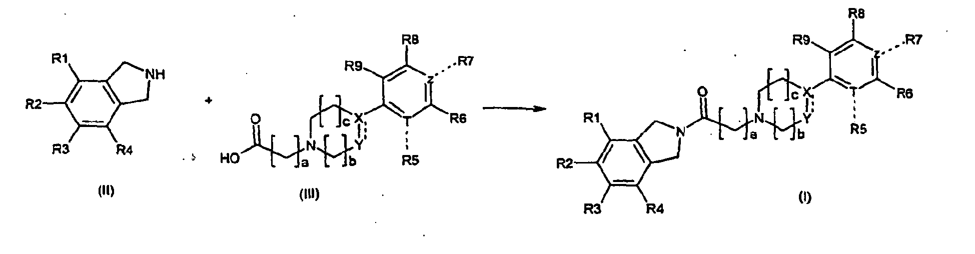 où, dans la formule (II) et (III). R1, R2, R3, R4, R5, R6, R7, R8, R9, X, Y, Z, T, ..., ..., a, b et c ont la même signification que dans la formule (I).

13. Procédé de préparation d'un composé de formule (I) selon l'une quelconque des revendications 1 à 11 tel que ledit procédé comprend l'étape de couplage de composés de formule (VI) dans laquelle R5, R6, R7, R8, R9, X, Y, Z, T, ..., ..., b et c ont la même signification que dans la formule (I) et de dérivés halogénés de formule (X) dans laquelle R1, R2, R3, R4 et a ont la même signification que dans la formule (I) et Hal représente un halogène:

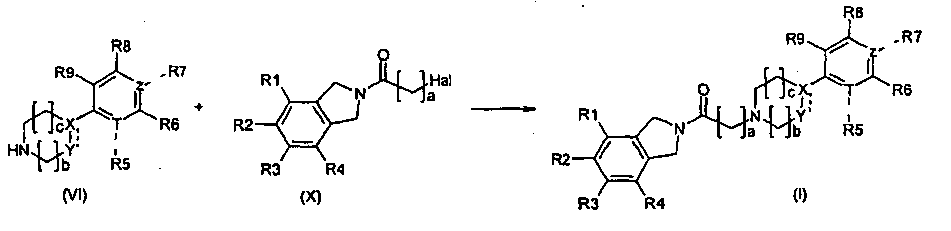

14. Procédé selon la revendication 12 ou 13 tel que ledit procédé comprend en outre l'étape ultérieure d'isolation des produits de formule (I) obtenus.

15. Composition pharmaceutique, **caractérisée en ce qu'**elle comprend une quantité thérapeutiquement efficace d'au moins un composé selon l'une quelconque des revendications 1 à 11 avec un véhicule ou excipient pharmaceutiquement acceptable.

16. Utilisation d'un composé de formule générale (I) selon l'une quelconque des revendications 1 à 11 pour la préparation de compositions pharmaceutiques destinées à agir comme ligand du récepteur D3 de la dopamine.

17. Utilisation d'un composé de formule générale (I) selon l'une quelconque des revendications 1 à 11 pour la préparation de compositions pharmaceutiques destinées à prévenir et/ou traiter une affection neuropsychiatrique.

18. Utilisation d'un composé de formule générale (I) selon l'une quelconque des revendications 1 à 11 pour la préparation de compositions pharmaceutiques destinées à prévenir et/ou traiter les affections faisant intervenir le récepteur D3 de la dopamine.

19. Utilisation selon la revendication 18 telle que ladite affection est choisie parmi la pharmacodépendance, les troubles de la sphère sexuelle, les troubles moteurs, la maladie de Parkinson, la psychose ou état psychotique, la dépression ou pharmacodépendance.

20. Utilisation selon la revendication 19, telle que ladite pharmacodépendance comprend tout état lié à la désaccoutumance, l'abstinence et/ou à la désintoxication d'un sujet dépendant de tout agent, notamment les agents actifs thérapeutiques, tels que les morphiniques, et/ou drogues telles que la cocaïne, l'héroïne, ou encore l'alcool et/ou la nicotine.

21. Utilisation selon la revendication 19 telle que lesdits troubles de la sphère sexuelle comprennent l'impuissance, notamment l'impuissance masculine.

22. Utilisation selon la revendication 19 telle que ladite prévention et/ou traitement de la maladie de Parkinson est un traitement complémentaire de la maladie de Parkinson.

23. Utilisation selon la revendication 19, telle que lesdits troubles moteurs comprennent les dyskinésies essentielles ou iatrogènes, et/ou les tremblements essentiels ou iatrogènes.

## Claims

1. Compounds of the formula (I) : 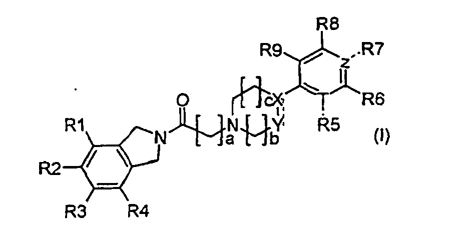 in which:
■ a = 5 or 6;
■ b and c, identical or different, represent 1 or 2;
■ X---Y represents >N-CH₂-, >C=CH-, >CH-CH₂-;
■ Z and T, identical or different, independently represent a nitrogen or carbon atom;
■ --- is absent or represents a single bond, it being understood that, when Z and/or T represents a nitrogen atom, --- is absent;
■ R1, R2, R3 and R4 each independently represent a hydrogen or halogen atom or a hydroxy, alkyl, monofluoroalkyl, polyfluoroalkyl, alkoxy, polyfluoroalkoxy, alkylsulfanyl, polyfluoroalkylsulfanyl, cyano, -OS(O)ₘ-alkyl, -NRR', -COOR, -COR, -CONRR', -C(OH)RR', -alkylC(OH)RR', -alkylCOOR, -alkylCOR, -alkylCONRR', -alkylNRR', -OalkylNRR', -Oalkyl(NRR')COOR group or a saturated or unsaturated heterocycle or alternatively two adjacent ones of R1, R2, R3 and R4 are joined to one another to form a hydrocarbon ring or a saturated or unsaturated heterocycle fused to the phenyl nucleus to which they are attached;
■ R5, R6, R7, R8 and R9 each independently represent a hydrogen atom or a hydroxy, alkyl, monofluoroalkyl, polyfluoroalkyl, alkoxy, polyfluoroalkoxy, alkylsulfanyl, polyfluoroalkylsulfanyl, cyano, -NRR', -COOR, -COR, -CONRR' group or alternatively two adjacent ones of R5, R6, R7, R8 and R9 are joined to one another to form a hydrocarbon ring or a saturated or unsaturated heterocycle fused to the phenyl nucleus to which they are attached;
where R, R,' identical or different, independently represent a hydrogen atom, an alkyl group optionally substituted by one or more groups selected from among OH, NRR', OR, COOR or R and R' are joined to one another to form a polymethylene or an oxapolymethylene;
m = 0, 1 or 2;
it being understood that when T=Z=N, R5 and R7 are absent, R6 and R8 are independently selected from among a hydrogen atom or an alkyl, monofluoroalkyl, polyfluoroalkyl, alkoxy, polyfluoroalkoxy, alkylsulfanyl, polyfluoroalkylsulfanyl, cyano, -COOR, -COR, -CONRR' group and R9 is selected from among a hydrogen atom or a hydroxy, alkyl, monofluoroalkyl, polyfluoroalkyl, alkoxy, polyfluoroalkoxy, alkylsulfanyl, polyfluoroalkylsulfanyl, -NRR', -COOR, -COR, -CONRR' group
or alternatively R8 and R9 are joined to one another to form a hydrocarbon ring or a saturated or unsaturated heterocycle fused to the phenyl nucleus to which they are attached;
together with the stereoisomers thereof or the mixtures thereof, the tautomeric forms thereof, the hydrates, solvates thereof, the pharmaceutically acceptable salts and esters thereof.

2. Compounds according to claim 1 according in which a = 5.

3. Compounds according to claim 1 or claim 2 in which b and c represent 1.

4. Compounds according to any one of the preceding claims such that ---Y represents >N-CH₂-.

5. Compounds according to any one of the preceding claims such that Z=T=C or Z=T=N.

6. Compounds according to any one of the preceding claims such that R1, R2, R3, R4 independently represent a hydrogen or halogen atom or an alkoxy group, -OS(O)ₘ-alkyl, cyano, -COOR, -COR, -CONRR', -C(OH)RR', -OalkylNRR'.

7. Compounds according to any one of the preceding claims such that, when T=Z=C, R5, R6, R7, R8, R9 independently represent a hydrogen atom or an alkoxy, alkyl, cyano, polyfluoroalkoxy group, such as a perfluoroalkoxy group, such as trifluoromethoxy, or a polyfluoroalkyl group, such as a perfluoroalkyl group, such as trifluoromethyl, or alternatively two adjacent ones of R5, R6, R7, R8 and R9 are joined to one another to form a hydrocarbon ring or a saturated or unsaturated heterocycle fused to the phenyl nucleus to which they are attached, such as a benzocycloheptene or benzofuran ring.

8. Compounds according to any one of claims 1 to 6 such that, when T=Z=N, R5 and R7 are absent, R6 and R8 are independently selected from among a hydrogen atom or an alkyl, monofluoroalkyl, polyfluoroalkyl, alkoxy, polyfluoroalkoxy, alkylsulfanyl, polyfluoroalkylsulfanyl, cyano, -COOR, -COR, -CONRR' group and R9 is selected from among a hydrogen atom or a hydroxy, alkyl, monofluoroalkyl, polyfluoroalkyl, alkoxy, polyfluoroalkoxy, alkylsulfanyl, polyfluoroalkylsulfanyl, -NRR', -COOR, -COR, -CONRR' group or alternatively R8 and R9 are joined to one another to form a hydrocarbon ring or a saturated or unsaturated heterocycle fused to the phenyl nucleus to which they are attached.

9. Compounds according to any one of claims 1 to 7 such that they are selected from among compounds of the formula (I'): 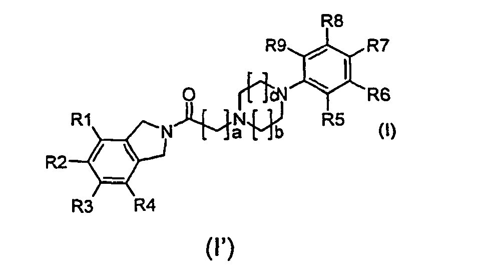 in which a, b and c, R1, R2, R3 and R4, R5, R6, R7, R8 and R9 are as defined in formula (I),
together with the stereoisomers thereof or the mixtures thereof, the tautomeric forms thereof, the hydrates, solvates thereof, the pharmaceutically acceptable salts and esters thereof.

10. Compounds according to any one of the preceding claims selected from among:
- 1-(5-methoxyisoindolin-2-yl)-6-[4-(3-trifluoromethylphenyl)piperazin-1-yl]hexan-1-one
- 1-(5-methanesulfonyloxyisoindolin-2-yl)-6-[4-(3-trifluoromethylphenyl)piperazin-1-yl]hexan-1-one
- 1-(5-cyanoisoindolin-2-yl)-6-[4-(3-trifluoromethylphenyl)piperazin-l-yl]-hexan-1-one hydrochloride
- 1-isoindolin-2-yl-6-[4-(3-trifluoromethoxyphenyl)piperazin-1-yl]hexan-1-one
- 1-isoindolin-2-yl-6-[4-(6,7,8,9-tetrahydro-5H-benzocylohepten-2-yl)piperazin-1-yl]hexan-1-one
- 1-(4-fluoroisoindolin-2-yl)-6-[4-(3-trifluoromethylphenyl)piperazin-1-yl]hexan-1-one
- 1-(5-fluoroisoindolin-2-yl)-6-[4-(3-trifluoromethylphenyl)piperazin-1-yl]hexan-1-one
- 1-(5-isopropoxyisoindolin-2-yl)-6-[4-(3-trifluoromethylphenyl)piperazin-1-yl]hexan-1-one
- 1-(4-chloroisoindolin-2-yl)-6-[4-(3-trifluoromethylphenyl)piperazin-1-yl]hexan-1-one
- 1-(6,7-dihydro-5H-[1,3]dioxolo[4,5-f]isoindol-6-yl)-6-[4-(3-trifluoromethylphenyl)piperazin-1-yl]hexan-1-one
- 1-(5-hydroxymethylisoindolin-2-yl)-6-[4-(3-trifluoromethylphenyl)piperazin-1-yl]hexan-1-one
- 1-(5-methoxycarbonylisoindolin-2-yl)-6-[4-(3-trifluoromethylphenyl)piperazin-1-yl]hexan-1-one hydrochloride
- 1-(5-carboxyisoindolin-2-yl)-6-[4-(3-trifluoromethylphenyl)piperazin-1-yl]hexan-1-one
- 1-[5-(2-hydroxyethylaminocarbonyl)isoindolin-2-yl]-6-[4-(3-trifluoromethylphenyl)piperazin-1-yl]hexan-1-one
- 1-(5-morpholinocarbonylisoindolin-2-yl)-6-[4-(3-trifluoromethylphenyl)piperazin-1-yl]hexan-1-one
- 1-(5-(pyrrolidin-1-ylcarbonylisoindolin-2-yl)-6-[4-(3-trifluoromethylphenyl)piperazin-1-yl]hexan-1-one
- 1-(5-piperidin-1-ylcarbonylisoindolin-2-yl)-6-[4-(3-trifluoromethylphenyl)piperazin-1-yl]hexan-1-one
- 1-[5-(2-dimethylaminoethylaminocarbonyl)-isoindolin-2-yl]-6-[4-(3-trifluoromethylphenyl)-piperazin-1-yl]hexan-1-one
- 1-[5-(4,5-dihydrooxazol-2-yl)isoindolin-2-yl]-6-[4-(3-trifluoromethylphenyl)piperazin-1-yl]hexan-1-one
- 1-(5-morpholinoylmethylisoindolin-2-yl)-6-[4-(3-trifluoromethylphenyl)piperazin-1-yl]hexan-1-one hydrochloride
- 1-[5-(pyrrolidin-1-ylmethyl)isoindolin-2-yl]-6-[4-(3-trifluoromethylphenyl)piperazin-1-yl]hexan-1-one
- 1-[5-(dimethylaminomethyl)isoindolin-2-yl]-6-[4-(3-trifluoromethylphenyl)piperazin-1-yl]hexan-1-one
- 1-[5-(2-piperidinoethoxy)isoindolin-2-yl]-6-[4-(3-trifluoromethylphenyl)piperazin-1-yl]hexan-1-one
- 1-[5-(3-piperidinopropoxy)isoindolin-2-yl]-6-[4-(3-trifluoromethylphenyl)piperazin-1-yl]hexan-1-one
- 1-[5-(3-pyrrolidinopropoxy)isoindolin-2-yl]-6-[4-(3-trifluoromethylphenyl)piperazin-1-yl]hexan-1-one
- 1-(5-acetylisoindolin-2-yl)-6-[4-(3-trifluoromethylphenyl)piperazin-1-yl]hexan-1-one
- 1-[5-(1-hydroxy-1-methylethyl)isoindolin-2-yl]-6-[4-(3-trifluoromethylphenyl)piperazin-1-yl]hexan-1-one
- 1-[5-(1-hydroxyethyl)isoindolin-2-yl]-6-[4-(3-trifluoromethylphenyl)piperazin-1-yl]hexan-1-one hydrochloride
- 1-(5-aminocarbonylisoindolin-2-yl)-6-[4-(3-trifluoromethylphenyl)piperazin-1-yl]hexan-1-one
- 1-isoindolin-2-yl-6-[4-(3-trifluoromethylphenyl)-piperazin-1-yl]hexan-1-one hydrochloride
- 1-isoindolin-2-yl-6-[4-(2-methoxyphenyl)-piperazin-1-yl]hexan-1-one
- 1-isoindolin-2-yl-6-[4-(6,7,8,9-tetrahydro-5H-benzocylohepten-1-yl)piperazin-1-yl]hexan-1-one
- 1-isoindolin-2-yl-6-[4-(2,3-dimethylphenyl)-piperazin-1-yl]hexan-1-one
- 1-isoindolin-2-yl-6-[4-benzofuran-7-ylpiperazin-1-yl]hexan-1-one
- 1-isoindolin-2-yl-6-[4-(2-cyano-3-trifluoromethylphenyl)piperazin-1-yl]hexan-1-one
- 1-isoindolin-2-yl-6-(4-indan-5-ylpiperazin-1-yl)-hexan-1-one
- 1-isoindolin-2-yl-6-(4-indan-4-ylpiperazin-1-yl)-hexan-1-one
- 1-isoindolin-2-yl-6-[4-(5,6,7,8-tetrahydronaphthalen-1-yl)piperazin-1-yl]hexan-1-one
- 1-isoindolin-2-yl-6-[4-(3-methanesulfonyloxyphenyl)piperazin-1-yl]hexan-1-one
- 1-isoindolin-2-yl-6-[4-(3,4-dihydro-2*H-*benzo[b][1,4]dioxepin-6-yl)piperazin-1-yl]hexan-1-one
- 1-isoindolin-2-yl-6-[4-(3-trifluoromethyl-5-cyanophenyl)piperazin-1-yl]hexan-1-one
- 1-isoindolin-2-yl-6-[4-(5,6,7,8-tetrahydronaphthalen-2-yl)piperazin-1-yl]hexan-1-one
- 1-isoindolin-2-yl-6-[4-(3-isopropoxyphenyl)-piperazin-1-yl]hexan-1-one
- 1-isoindolin-2-yl-6-[4-(2-cyano-3-methylphenyl)-piperazin-1-yllhexan-1-one
- 1-isoindolin-2-yl-6-[4-(2-cyano-3-isopropoxy)piperazin-1-yl]hexan-1-one
- 1-(5,6-dimethoxyisoindolin-2-yl)-6-[4-(3-trifluoromethylphenyl)piperazin-1-yl]hexan-1-one
- 1-isoindolin-2-yl-6-[4-(2-cyanophenyl)piperazin-1-yllhexan-1-one
- 1-isoindolin-2-yl-6-[4-(2-methylphenyl)piperazin-1-yl]hexan-1-one
- 1-isoindolin-2-yl-6-(4-phenyl-3,6-dihydro-2*H-*pyridin-1-yl)hexan-1-one
- 1-isoindolin-2-yl-6-[4-(3-trifluoromethylphenyl)-3,6-dihydro-2*H*-pyridin-1-yl]hexan-1-one
- 1-isoindolin-2-yl-6-[4-(3-trifluoromethylphenyl)-piperidin-1-yl]hexan-1-one
- 1-isoindolin-2-yl-6-[4-(2-cyano-3-methylphenyl)-3,6-dihydro-2*H*-pyridin-1-yl]hexan-1-one
- 1-isoindolin-2-yl-6-[4-(2-cyano-3-methylphenyl)-piperidin-1-yl]hexan-1-one,
together with the stereoisomers thereof or the mixtures thereof, the tautomeric forms thereof, the hydrates, solvates thereof, the pharmaceutically acceptable salts, free forms and esters thereof.

11. Compounds according to any one of the preceding claims selected from among:
- 1-(5-methoxyisoindolin-2-yl)-6-[4-(3-trifluoromethylphenyl)piperazin-1-yl]hexan-1-one
- 1-(5-cyanoisoindolin-2-yl)-6-[4-(3-trifluoromethylphenyl)piperazin-1-yl]-hexan-1-one hydrochloride
- 1-(5-fluoroisoindolin-2-yl)-6-[4-(3-trifluoromethylphenyl)piperazin-1-yl]hexan-1-one
- 1-(6,7-dihydro-5*H*-[1,3]dioxolo[4,5-*f*]isoindol-6-yl)-6-[4-(3-trifluoromethylphenyl)piperazin-1-yl]hexan-1-one
- 1-(5-methoxycarbonylisoindolin-2-yl)-6-[4-(3-trifluoromethylphenyl)piperazin-1-yl]hexan-1-one hydrochloride
- 1-[5-(3-piperidinopropoxy)isoindolin-2-yl]-6-[4-(3-trifluoromethylphenyl)piperazin-1-yl]hexan-1-one
- 1-isoindolin-2-yl-6-[4-(5,6,7,8-tetrahydronaphthalen-1-yl)piperazin-1-yl]hexan-1-one
- 1-isoindolin-2-yl-6-[4-(3-trifluoromethylphenyl)-3,6-dihydro-2*H*-pyridin-1-yl]hexan-1-one
- 1-isoindolin-2-yl-6-[4-(3-trifluoromethylphenyl)-piperidin-1-yl]hexan-1-one
together with the stereoisomers thereof or the mixtures thereof, the tautomeric forms thereof, the hydrates, solvates thereof, the pharmaceutically acceptable salts, free forms and esters thereof.

12. A method of preparing a compound of the formula (I) according to any one of the preceding claims such that said method comprises the step of coupling the compounds of the formulae (II) and (III); 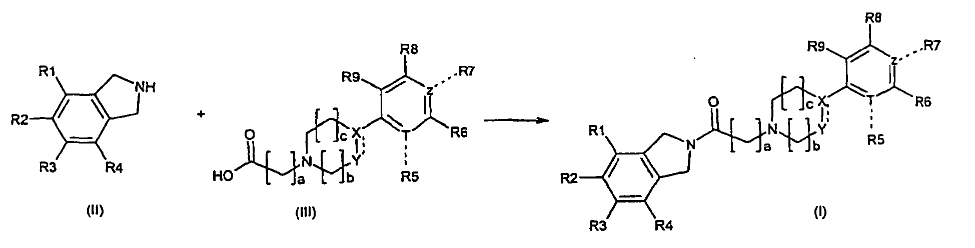 where, in the formulae (II) and (III), R1, R2, R3, R4, R5, R6, R7, R8, R9, X, Y, Z, T, ---, ---, a, b and c have the same meaning as in the formula (I).

13. A method of preparing a compound of the formula (I) according to any one of claims 1 to 11 such that said method comprises the step of coupling compounds of the formula (VI) in which R5, R6, R7, R8, R9, X, Y, Z, T, ---, ---, b and c have the same meaning as in the formula (I) and halogenated derivatives of the formula (X) in which R1, R2, R3, R4 and a have the same meaning as in the formula (I) and Hal represents a halogen:

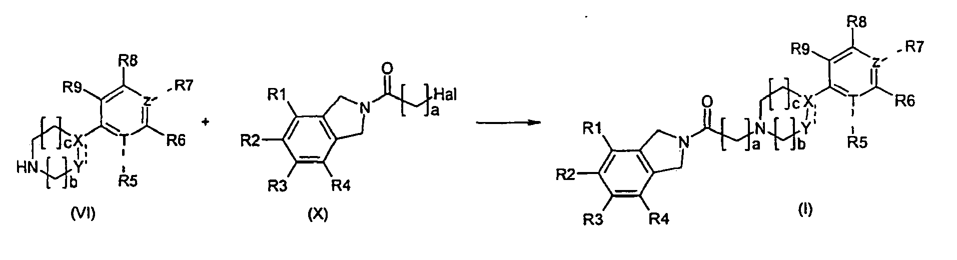

14. A method according to claim 12 or claim 13 such that said method furthermore comprises the subsequent step of isolating the resultant products of the formula (I) .

15. A pharmaceutical composition, **characterised in that** it comprises a therapeutically effective quantity of at least one compound according to any one of claims 1 to 11 with a pharmaceutically acceptable vehicle or excipient.

16. Use of a compound of the general formula (I) according to any one of claims 1 to 11 for preparing pharmaceutical compositions intended to act as a dopamine D3 receptor ligand.

17. Use of a compound of the general formula (I) according to any one of claims 1 to 11 for preparing pharmaceutical compositions intended to prevent and/or treat a neuropsychiatric illness.

18. Use of a compound of the general formula (I) according to any one of claims 1 to 11 for preparing pharmaceutical compositions intended to prevent and/or treat disorders involving the dopamine D3 receptor.

19. Use according to claim 18 such that said illness is selected from among drug dependency, disorders of a sexual nature, motor disorders, Parkinson's disease, psychosis or psychotic state, depression or drug dependency.

20. Use according to claim 19, such that said drug dependency comprises any state associated with the cessation, abstinence and/or detoxification of an individual dependent on any agent, in particular therapeutic active agents, such as opioids, and/or drugs such as the cocaine, heroin, or alternatively alcohol and/or nicotine.

21. Use according to claim 19 such that said disorders of a sexual nature comprise impotence, in particular male impotence.

22. Use according to claim 19 such that said prevention and/or treatment of Parkinson's disease is a complementary treatment for Parkinson's disease.

23. Use according to claim 19, such that said motor disorders comprise essential or iatrogenic dyskinesia and/or essential or iatrogenic tremor.

## Patentansprüche

1. Verbindungen der Formel (I) 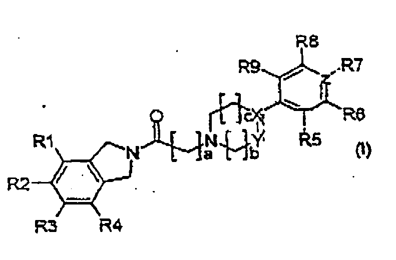 wobei:
a = 5 oder 6;
b und c, gleich oder verschieden, 1 oder 2 bedeuten,
X---Y > N-CH₂-, >C=CH-, >CH-CH₂- darstellt;
Z und T, gleich oder verschieden, unabhängig voneinander ein Stickstoff- oder Kohlenstoffatom darstellen;
----- abwesend ist oder eine Einfachbindung darstellt, wobei, wenn Z und/oder T ein Stickstoffatom darstellt, ----- abwesend Ist;
R1, R2, R3 und R4 jeweils unabhängig voneinander ein Wasserstoff- oder Halogenatom darstellen oder eine Gruppe Hydroxy, Alkyl, Monofluorolkyl, Polyfluoralkyl, Alkoxy, Polyfluoralkoxy, Alkylsulfanyl, Polyfluoroalkylsulfanyl, Cyano, -OS(O)ₘ-Alkyl, -NRR', -COOR, -COR, -CONRR', -C(OH)RR', -AlkylC(OH)RR', -AlkylCOOR, -AlkylCOR, -AlkylCONRR', -AlkylNRR', -OAlkylNRR', -OAlkyl(NRR')COOR oder einen gesättigten oder ungesättigten Heterozyklus darstellt, oder zwei der benachbarten Reste R1, R2, R3 und R4 aneinander gebunden sind, um einen gesättigten oder ungesättigten Kohlenwasserstoff-Ring oder Heterozyklus zu bilden, der mit dem Phenylkern kondensiert Ist, an den sie gebunden sind;
R5, R6, R7, R8 und R9 jeweils unabhängig voneinander ein Wasserstoffatom oder eine Gruppe Hydroxy, Alkyl, Monofluoralkyl, Polyfluoralkyl, Alkoxy, Polyfluoralkoxy, Alkylsulfanyl, Polyfluoralkylsulfanyl, Cyano, -NRR', -COOR, -COR, -CONRR' darstellen, oder zwei benachbarte R5, R6. R7, R8 und R9 aneinander gebunden sind unter Bildung eines gesättigten oder ungesättigten Kohlenwasserstoffrings oder Heterozyklus, der mit dem Phenylkern kondensiert ist, an den sie gebunden sind;
wobei R, R', gleich oder verschieden, unabhängig voneinander ein Wasserstoffatom, eine gegebenenfalls durch einen oder mehrere aus OH, NRR', OR, COOR ausgewählte Gruppen substituierte Alkylgruppe darstellen, oder R und R' aneinander unter Bildung eines Polymethylen oder eines Oxapolymethylen gebunden sind;
m 0, 1 oder 2 ist;
wobei, wenn T = Z = N, R5 und R7 abwesend sind, R6 und R8 unabhängig voneinander ausgewählt werden aus einem Wasserstoffatom oder einer Gruppe Alkyl, Monofluoralkyl, Polyfluoralkyl, Alkoxy, Polyfluoralkoxy, Alkylsulfanyl, Polyfluoralkylsulfanyl, Cyano, -COOR, -COR, -CONRR', und R9 ausgewählt wird aus einem Wasserstoffatom oder einer Gruppe Hydroxy, Alkyl, Monofluoralkyl, Polyfluoralkyl, Alkoxy, Polyfluoralkoxy, Alkylsulfanyl, Polyfluoralkylsulfanyl, -NRR', -COOR, -COR, -CONRR',
oder R8 und R9 aneinander gebunden sind, um einen gesättigten oder ungesättigten Kohlenwasserstoffring oder Heterozyklus zu bilden, der mit dem Phenylkern kondensiert ist, an den sie gebunden sind;
sowie ihre pharmazeutisch verträglichen Stereoisomere oder Gemische, tautomeren Formen, Hydrate, Solvate, Salze und Ester.

2. Verbindungen noch Anspruch 1 , wobei a = 5,

3. Verbindungen nach Anspruch 1 oder 2, wobei b und c 1 darstellen.

4. Verbindungen nach einem der vorstehenden Ansprüche, wobei ---Y > N-CH₂- darstellt.

5. Verbindungen nach einem der vorstehenden Ansprüche, wobei Z=T=C oder Z=T=N.

6. Verbindungen nach einem der vorstehenden Ansprüche, wobei R1, R2, R3, R4 unabhängig voneinander ein Wasserstoff- oder Halogenatom darstellen oder eine Gruppe Alkoxy, -OS(O)ₘ-Alkyl, Cyano, -COOR, -COR, -CONRR', -C(OH)RR', -OAlkylNRR' darstellen,

7. Verbindungen nach einem der vorstehenden Ansprüche, wobei, wenn T=Z=C, R5, R6, R7, R8, R9 unabhängig voneinander ein Wasserstoffatom oder eine Gruppe Alkoxy, Alkyl, Cyano, Polyfluoralkoxy darstellen, wie eine Perfluoralkoxygruppe, wie eine Trifluormethoxygruppe, oder eine Polyfluoralkylgruppe wie eine Perfluoralkylgruppe wie Trifluormethyl, oder zwei benachbarte R5, R6, R7, R8 und R9 aneinander gebunden sind, um einen gesättigten oder ungesättigten Kohlenwasserstoffring oder Heterozyklus zu bilden, der mit dem Phenylkern kondensiert ist, an den sie gebunden sind, wie einen Benzocyclohepten- oder Benzofuranring.

8. Verbindungen nach einem der Ansprüche 1 bis 6, wobei, wenn T=Z=N, R5 und R7 abwesend sind, R6 und R8 unabhängig voneinander ausgewählt werden aus einem Wasserstoffatom oder einer Gruppe Alkyl, Monofluoralkyl, Polyfluoralkyl, Alkoxy, Polyfluoralkoxy, Alkylsulfanyl, Polyfluorolkylsulfanyl, Cyano, -COOR, -COR, -CONRR', und R9 ausgewählt wird aus einem Wasserstoffatom oder einer Gruppe Hydroxy, Alkyl, Monofluoralkyl, Polyfluoralkyl, Alkoxy, Polyfluoralkoxy, Alkylsulfanyl, Polyfluoralkylsulfanyl, -NRR', COOR, -COR, -CONRR', oder R8 und R9 aneinander gebunden sind unter Bildung eines gesättigten oder ungesättigten Kohlenwasserstoffrings oder Heterozyklus, der mit dem Phenylkern kondensiert ist, an den sie gebunden sind.

9. Verbindungen nach einem der Ansprüche 1 bis 7, ausgewählt aus den Verbindungen der Formel (1'): 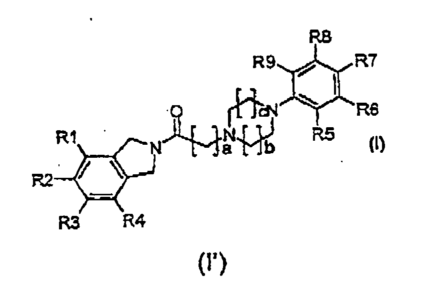 wobei a, b und c, R1, R2, R3 und R4, R5, R6, R7, R8 und R9 so wie in Formel (I) definiert sind,
genauso wie ihre pharmazeutisch verträglichen Stereoisomere oder Gemische, tautomeren Formen, Hydrate, Solvote, Salze und Ester.

10. Verbindungen nach einem der vorstehenden Ansprüche, ausgewählt aus;
- 1-(5-Methoxylsolndolin-2-yl)-6-[4-(3-trifluormethylphenyl)piperazln-1-yl]hexanl-1-on,
- 1-(5-Methansulfonyloxylsoindolin-2-yl)-6-[4-(3-trifluormethylphenyl)-plperazin-1-yl]hexan-1-on,
- Hydrochlorid von 1-(5-Cyanolsoindolin-2-yl)-6-[4-(3-trlfluormethylphenyl)-plperozin-1-yl]-hexan-1-on,
- 1-Isoindolin-2-yl-6-[4-(3-trifluormethoxyphenyl)piperazin-1-yl]-hexan-1-on,
- 1-Isoindolin-2-yl-6-[4-(6,7,8,9-tetrohydro-5H-benzocyclohepten-2-yl)piperazin-1-yl]hexan-1-on,
- 1-(4-Fluorisoindolin-2-yl)-b-[4-(3-trifluormethylphenyl)plperazin-1-yl]hexan-1-on,
- 1-(5-Fluorisoindolin-2-yl)-6-[4-(3-trifluormethylphenyl)piperazin-1-yl]hexan-1-on,
- 1-(5-Isopropoxyisoindolin-2-yl)-6-[4-(3-trifluormethylphenyl)-piperazin-1-yl]hexan-1-on,
- 1-(4-Chlorisoindolin-2-yl)-6-[4-(3-trifluormethlyphenyl)piperazin-1-yl]hexan-1-on,
- 1-(6,7-Dihydro-5H-[1,3]dioxolo[4,5-f]isoindol-6-yl)-6-[4-(3-trifluormethylphenyl)piperazin-1-yl]hexan-1-on,
- 1-(5-Hydroxymethylisoindolin-2-yl)-6-(4-(3-trifluormethylphenyl)-piperazin-1-yl]hexan-1-on,
- Hydrochlorid von 1-(5-Methoxycarbonylisoindolin-2-yl)-6-[4-(3-trifluormethylphenyl)piperazin-1-yl]hexan-1-on,
- 1-(5-Carboxylsoindolin-2-yl)-6-[4-(3-trifluormethylphenyl)piperazin-1 yl]hexan-1-on,
- 1-[5-(2-Hydroxyethylaminocarbonyl)isoindolin-2-yl]-6-[4-(3-trifluormethylphenyl)piperazin-1-yl]hexan-1-on,
- 1 -(5-Morpholinocarbonylisoindolin-2-yl)-6-[4-(3-trifluormethylphenyl)piperazin-1-yl]hexan-1-on,
- 1-(5-Pyrrolidin-1-ylcarbonylisoindolin-2-yl)-6-[4-(3-trifluormethylphenyl)-piperazin-1-yl]hexan-1-on,
- 1-(5-Piperidin-1-ylcarbonylisoindolin-2-yl)-6-[4-(3-trifluormethylphenyl)piperazin-1-yl]hexan-1-on,
- 1-[5-(2-Dimethylamino-ethylaminocarbonyl)isoindolin-2-yl]-6-[4-(3-trifluormethylphenyl)piperazin-1-yl]hexan-1-on,
- 1-[5-(4,5-dihydrooxazol-2-yl)isoindolin-2-yl]-6-[4-(3-trifluormethylphenyl)piperazin-1-yl]hexan-1-on,
- Hydrochlorid von 1-(5-Morpholinoylmethylisoindolin-2-yl)-6-[4-(3-trlfluormethylphenyl)piperazin-1-yl]hexan-1-on,
- 1-[5-(Pyrrolidin-1-ylmethyl)isoindolin-2-yl]-6-[4-(3-trifluormethylphenyl)piperazin-1-yl]hexan-1-on,
- 1-[5-(Dimethylaminomethyl)isoindolin-2-yl]-6-(3-trifluormethylphenyl)piperazin-1-yl]hexan-1-on,
- 1-[5-(2-Piperidinethoxy)isoindolin-2-yl]-6-[4-(3-trifluormethylphenyl)-piperazin-1-yl]hexan-1-on,
- 1-[5-(3-Piperidinpropoxy)isoindolin-2-yl]-6-[4-(3-trifluormethylphenyl)piperazin-1-yl]hexan-1-on,
- 1-[5-(3-Pyrrolidinpropoxy)isoindolin-2-yl]6-[4-(3-trifluormethlphenyl)-piperazin-1-yl]hexan-1-on,
- 1-[5-Acetylisoindolin-2-yl)6-[4-(3-trifluormethylphenyl)piperazin-1-yl]hexan-1-on,
- 1-[5-(1-Hydroxy-1-methylethyl)isoindolin-2-yl]-6-[4-(3-trifluormethylphenyl)piperazin-1-yl]hexan-1-on,
- Hydrochlorid von 1-[5-(1-Hydroxyethyl)isoindolin-2-yl]-6-[4-(3-trifluormethylphenyl)piperazin-1-yl]hexan-1-on,
- 1-(5-Aminocarbonylisoindolin-2-yl)-6-[4-(3-trifluormethylphenyl)-piperazin-1-yl]hexan-1-on,
- Hydrochlorid von 1-Isoindolin-2-yl-6-[4-(3-trifluormethylphenyl)-piperain-1-yl]hexan-1-on,
- 1-Isoindolin-2-yl-6-[4-(2-methoxyphenyl)piperazin-1-yl]hexan-1-on,
- 1-Isoindolin-2-yl-6-[4-(6,7,8,9-tetrahydro-5H-benzocyclohepten-1-yl)piperazin-1-yl]hexan-1-on,
- 1-Isoindolin-2-yl-6-[4-(2,3-dimethylphenyl)piperazin-1-yl]hexan-1-on,
- 1-Isoindolin-2-yl-6-[4-benzofuran-7-yl-piperazin-1-yl]hexan-1-on,
- 1-Isoindolin-2-yl-6-[4-(2-cyano-3-trifluormethylphenyl)piperazin-1-yl]hexan-1-on,
- 1-Isoindolin-2-yl-6-(4-indan-5-yl-piperazin-1-yl)hexan-1-on,
- 1-Isoindolin-2-yl-6-(4-indan-5-yl-piperazin-1-yl)hexan-1-on,
- 1-Isoindolin-2-yl-6-[4-(5,6,7,8-tetrahydronaphthalin-1-yl)piperazin-1-yl]hexan-1-on,
- 1-Isoindolin-2-yl-6-[4-(3-methansulfonyloxyphenyl)piperazin-1-yl]hexan-1-on,
- 1-Isoindolin-2-yl-6-[4-(3,4-dihydro-2H-benzo[b][1,4]dioxepin-6-yl)piperazin-1-yl]hexan-1-on,
- 1-Isoindolin-2-yl-6-[4-(3-trifluormethyl-5-cyanophenyl)piperazin-1-yl]hexan-1-on,
- 1-Isoindolin-2-yl-6-[4-(5,6,7,8-tetrahydronaphthalin-2-yl)-piperazin-1-yl]hexan-1-on,
- 1-Isoindolin-2-yl-6-[4-(3-isopropoxyphenyl)piperazin-1-yl]hexan-1-on,
- 1-Isoindolin-2-yl-6-[4-(2-cyano-3-methylphenyl)piperazin-1-yl]hexan-1-on,
- 1-Isoindolin-2-yl-6-[4-(2-cyano-3-Isopropoxy)piperazin-1-yl]hexan-1-on,
- 1-(5,6-Dimethoxyisoindolin-2-yl)-6-[4-(3-trifluormethylphenyl)-piperazin-1-yl]hexan-1-on,
- 1-Isoindolin-2-yl-6-[4-(2-cyanophenyl)piperazin-1-yl]hexan-1-on,
- 1-Isoindolin-2-yl-6-[4-(2-methylphenyl)piperazin-1-yl]hexan-1-on,
- 1-Isoindolin-2-yl-6-(4-phenyl-3,6-dihydro-2H-pyridin-1-yl)hexan-1-on,
- 1-Isoindolin-2-yl-6-[4-(3-trifluormethylphenyl)-3,6-dihydro-2H-pyridin-1-yl]hexan-1-on,
- 1-Isoindolin-2-yl-6-[4-(3-trifluormethylphenyl)piperidin-1-yl]hexan-1-on,
- 1-Isoindolin-2-yl-6-[4-(2-cyano-3-methylphenyl)-3,6-dihydro-2H-pyridin-1-yl]hexan-on,
- 1-Isoindolin-2-yl-6-[4-(2-cyano-3-methylphenyl)piperidin-1-yl]hexan-1-on,
sowie Ihre , pharmazeutisch verträglichen Stereoisomere oder Gemische, tautomeren Formen, Hydrate, Solvate, Salze, freien Formen und Ester.

11. Verbindungen nach einem der vorstehenden Ansprüche, ausgewählt aus:
- 1-(5-Methoxylsolndolin-2-yl)-6-[4-(3-trifluormethylphenyl)piperazin-1-yl]hexan-1-on,
- Hydrochlorid von 1-(5-Cyanoisoindolin-2-yl)-6-[4-(3-trifluormethylphenyl)-piperazin-1-yl]hexan-1-on,
- 1-(5-Fluorisoindolin-2-yl)-6-[4-(3-trifluormethylphenyl)piperazin-1-yl]hexan-1-on,
- 1-(6,7-Dihydro-5H-[1,3]dioxolo[4,5-f]isoindol-6-yl)-6-[4-(3-trifluormethylphenyl)-piperazin-1-yl]hexan-1-on,
- Hydrochlorid von 1-(5-Methoxycarbonylisoindolin-2-yl)-6-[4-(3-trifluormethylphenyl)piperazin-1-yl]hexan-1-on,
- 1-[5-(3-Piperidinpropoxy)isoindolin-2-yl]-6-[4-(3-trifluormethylphenyl)piperazin-1-yl]hexan-1-on,
- 1-Isoindolin-2-yl-6-[4-(5,6,7,8-tetrahydro-naphthalin-1-yl)piperazin-1-yl]hexan-1-on,
- 1-Isoindolin-2-yl-6-[4-(3-trifluormethylphenyl)-3,6-dihydro-2H-pyridin-1-yl]hexan-1-on,
- 1-Isoindolin-2-yl-6-[4-(3-trifluormethylphenyl)piperidin-1-yl]hexan-1-on,
genauso wie ihre pharmazeutisch verträglichen Stereoisomere oder Gemische, tautomeren Formen, Hydrate, Solvate, Salze, freien Formen und Ester.

12. Verfahren zur Herstellung einer Verbindung der Formel (I) nach einem der vorstehenden Ansprüche, wobei das Verfahren den Schritt der Kopplung der Verbindungen der Formel (II) und (III) umfasst: 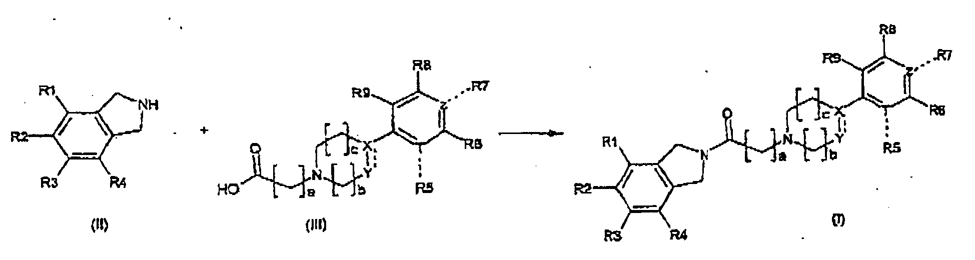 wobei in Formel (II) und (III) R1, R2, R3, R4, R5, R6, R7, R8, R9, X, Y, Z, T, ..., ---, a, b und c dieselbe Bedeutung haben wir In Formel (I).

13. Verfahren zur Herstellung einer Verbindung der Formel (I) nach einem der Ansprüche 1 bis 11, wobei das Verfahren den Schritt der Kopplung der Verbindungen der Formel (VI, wobei R5, R6, R7, R8, R9, X, Y, Z, T, ..., ---, a, b und c dieselbe Bedeutung haben wir In Formel (I) und den halogenierten Derivaten der Formel (X)) umfasst, in denen R1, R2, R3, R4 und a dieselbe Bedeutung haben wie In Formel (I) und Hal ein Halogen darstellt:

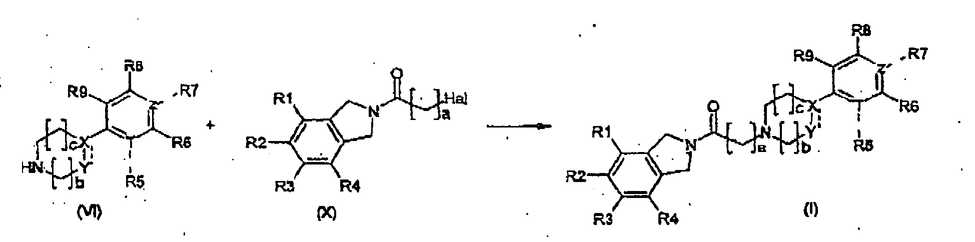

14. Verfahren nach Anspruch 12 oder 13, wobei das Verfahren einen letzten Schritt der Isolierung der erhaltenen Produkte der Formel (I) umfasst.

15. Pharmazeutische Zusammensetzung, **dadurch charakterisiert, dass** sie eine therapeutisch wirksame Menge mindestens einer Verbindung nach einem der Ansprüche 1 bis 1 1 mit einem pharmazeutisch verträglichen Vehikel oder Exzipienten umfasst.

16. Verwendung einer Verbindung der allgemeinen Formel (I) nach einem der Ansprüche 1 bis 1 1 zur Herstellung von pharmazeutischen Zusammensetzungen, die dazu bestimmt sind, als Ligand des Dopamin-Rezeptors D3 zu wirken,

17. Verwendung einer Verbindung der allgemeinen Formel (I) nach einem der Ansprüche 1 bis 11 zur Herstellung von pharmazeutischen Zusammensetzungen, die zur Vorbeugung und/oder Behandlung einer neuropsychiatrischen Erkrankung bestimmt sind.

18. Verwendung einer Verbindung der allgemeinen Formel (I) nach einem der Ansprüche 1 bis 1 1 zur Herstellung von pharmazeutischen Zusammensetzungen, die zur Vorbeugung und/oder Behandlung von Erkrankungen bestimmt sind, die eine Beteiligung des Dopamin-D3-Rezeptors beinhalten.

19. Verwendung nach Anspruch 18, wobei die Erkrankung ausgewählt wird aus Arznelmlttelabhängigkeit, sexuellen Störungen, motorischen Störungen, der Parklnson-Krankheit, Psychosen oder psychotischen Zuständen, Depression oder Arznelmlttelabhöngigkeit.

20. Verwendung noch Anspruch 19, wobei die Arzneimitteiabhängigkelt Jeden Zustand umfasst, der mit der Entwöhnung, Abstinenz und/oder Entgiftung eines Patienten zusammenhängt, der von einem beliebigen Mittel abhängig ist, insbesondere therapeutisch wirksamen Mitteln wie Morphinen, und/oder Drogen wie Kokain, Heroin oder auch Alkohol und/oder Nikotin.

21. Verwendung nach Anspruch 19, wobei die sexuellen Störungen Impotenz umfassen, Insbesondere männliche Impotenz.

22. Verwendung nach Anspruch 19, wobei die Vorbeugung und/oder Behandlung der Parkinson-Krankhelt eine Komplementärbehandlung der Parkinson-Krankhelt Ist.

23. Verwendung nach Anspruch 19, wobei die motorischen Störungen essentielle oder iatrogene Dyskineslen umfassen, und/oder essentielles oder iatrogenes Zittern.
